(19) Europäisches Patentamt
European Patent Office
Office européen des brevets

(11) EP 4 263 548 B1

(12) # EUROPEAN PATENT SPECIFICATION

(45) Date of publication and mention of the grant of the patent:
**02.07.2025 Bulletin 2025/27**

(21) Application number: **21824584.3**

(22) Date of filing: **13.12.2021**

(51) International Patent Classification (IPC):
***C07D 491/048*** *(2006.01)* ***A61K 31/506*** *(2006.01)*
***A61P 11/00*** *(2006.01)* ***A61P 11/06*** *(2006.01)*

(52) Cooperative Patent Classification (CPC):
**C07D 491/048; A61P 11/00; A61P 11/06**

(86) International application number:
**PCT/EP2021/085367**

(87) International publication number:
**WO 2022/128843 (23.06.2022 Gazette 2022/25)**

(54) **DIHYDROFUROPYRIDINE DERIVATIVES AS RHO- KINASE INHIBITORS**
DIHYDROFUROPYRIDINDERIVATE ALS RHO-KINASE-INHIBITOREN
DÉRIVÉS DE DIHYDROFUROPYRIDINE EN TANT QU'INHIBITEURS DE RHO-KINASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **15.12.2020 EP 20214144**

(43) Date of publication of application:
**25.10.2023 Bulletin 2023/43**

(73) Proprietor: **Chiesi Farmaceutici S.p.A.**
**43122 Parma (IT)**

(72) Inventors:
- **RANCATI, Fabio**
  **43122 Parma (IT)**
- **ACCETTA, Alessandro**
  **43122 Parma (IT)**
- **CAPELLI, Anna Maria**
  **43122 Parma (IT)**
- **PALA, Daniele**
  **43122 Parma (IT)**
- **EDWARDS, Christine**
  **43122 Parma (IT)**
- **PASQUA, Adele Elisa**
  **43122 Parma (IT)**
- **KAPADNIS, Prashant Bhimrao**
  **43122 Parma (IT)**
- **CHEGUILLAUME, Arnaud Jean François Auguste**
  **43122 Parma (IT)**
- **CLARK, David Edward**
  **43122 Parma (IT)**

(74) Representative: **Chiesi Farmaceutici S.p.A.**
**Via Palermo, 26/A**
**43122 Parma (IT)**

(56) References cited:
**WO-A1-2004/039796 WO-A1-2019/238628**

- **OLIVIER DEFERT ET AL: "Rho kinase inhibitors: a patent review (2014 - 2016)", EXPERT OPINION ON THERAPEUTIC PATENTS, vol. 27, no. 4, 16 January 2017 (2017-01-16), GB, pages 507 - 515, XP055400492, ISSN: 1354-3776, DOI: 10.1080/ 13543776.2017.1272579**
- **YANGBO FENG ET AL: "Rho Kinase (ROCK) Inhibitors and Their Therapeutic Potential", JOURNAL OF MEDICINAL CHEMISTRY, vol. 59, no. 6, 30 October 2015 (2015-10-30), US, pages 2269 - 2300, XP055535566, ISSN: 0022-2623, DOI: 10.1021/acs.jmedchem.5b00683**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 263 548 B1

## Description

### FIELD OF THE INVENTION

The present invention relates to novel compounds inhibiting Rho Kinase (hereinafter ROCK Inhibitors); methods of preparing such compounds, pharmaceutical compositions containing them and therapeutic use thereof.

### BACKGROUND OF THE INVENTION

The compounds of the invention are inhibitors of the activity or function of the ROCK-I and/or ROCK-II isoforms of the Rho-associated coiled-coil forming protein kinase (ROCK).

Rho-associated coiled-coil forming protein kinase (ROCK) belongs to the AGC (PKA/PKG/PKC) family of serine-threonine kinases. Two human isoforms of ROCK have been described, ROCK-I (also referred to as p160 ROCK or ROKβ or ROCK1) and ROCK-II (ROKα or ROCK2) are approximately 160 kDa proteins containing an N-terminal Ser/Thr kinase domain, followed by a coiled-coil structure, a pleckstrin homology domain, and a cysteine-rich region at the C-terminus (Riento, K.; Ridley, A. J. Rocks: multifunctional kinases in cell behaviour. Nat. Rev. Mol. Cell Biol. 2003, 4, 446-456).

Both ROCK-II and ROCK-I are expressed in many human and rodent tissues including the heart, pancreas, lung, liver, skeletal muscle, kidney and brain (above Riento and Ridley, 2003). In patients with pulmonary hypertension, ROCK activity is significantly higher in both lung tissues and circulating neutrophils as compared with controls (Duong-Quy S, Bei Y, Liu Z, Dinh-Xuan AT. Role of Rho-kinase and its inhibitors in pulmonary hypertension. Pharmacol Ther. 2013;137(3):352-64). A significant correlation was established between neutrophil ROCK activity and the severity and duration of pulmonary hypertension (Duong-Quy et al., 2013).

There is now substantial evidence that ROCK is involved in many of the pathways that contribute to the pathologies associated with several acute and chronic pulmonary diseases, including asthma, COPD, bronchiectasis and ARDS/ALI. Given the biological effect of ROCK, selective inhibitors have the potential to treat a number of pathological mechanisms in respiratory diseases, such as smooth muscle hyper-reactivity, bronchoconstriction, airway inflammation and airway remodeling, neuromodulation and exacerbations due to respiratory tract viral infection (Fernandes LB, Henry PJ, Goldie RG. Rho kinase as a therapeutic target in the treatment of asthma and chronic obstructive pulmonary disease. Ther Adv Respir Dis. 2007 Oct;1(1):25-33). Indeed the Rho kinase inhibitor Y-27632 causes bronchodilatation and reduces pulmonary eosinophilia trafficking and airways hyperresponsiveness (Gosens, R.; Schaafsma, D.; Nelemans, S. A.; Halayko, A. J. Rhokinase as a drug target for the treatment of airway hyperresponsiveness in asthma. Mini-Rev. Med. Chem. 2006, 6, 339-348). Pulmonary ROCK activation has been demonstrated in humans with idiopathic pulmonary fibrosis (IPF) and in animal models of this disease. ROCK inhibitors can prevent fibrosis in these models, and more importantly, induce the regression of already established fibrosis, thus indicating ROCK inhibitors as potential powerful pharmacological agents to halt progression of pulmonary fibrosis (Jiang, C.; Huang, H.; Liu, J.; Wang, Y.; Lu, Z.; Xu, Z. Fasudil, a rho-kinase inhibitor, attenuates bleomycin-induced pulmonary fibrosis in mice. Int. J. Mol. Sci. 2012, 13, 8293-8307).

Various compounds have been described in the literature as Rho Kinase Inhibitors. See e.g. WO2004/039796 disclosing phenylaminopyrimidine compounds derivatives; WO2006/009889 disclosing indazole compound derivatives; WO2010/032875 disclosing nicotinamide compounds derivatives; WO2009/079008 disclosing pyrazole derivatives; WO2014/118133 disclosing pyrimidine derivatives and, of the same Applicant of the present invention, WO2018/115383 disclosing bicyclic dihydropyrimidine and WO 2018/138293, WO 2019/048479, WO 2019/121223, WO 2019/121233, WO 2019/121406, WO 2019/238628, WO 2020/016129 disclosing tyrosine-amide compounds derivatives and analogues.

The compounds disclosed exhibit substantial structural differences from the compounds of the present invention.

There remains a potential for developing novel and pharmacologically improved ROCK inhibitors in many therapeutic areas.

In view of the number of pathological responses which are mediated by ROCK enzymes, there is a continuing need for inhibitors of such enzymes which can be useful in the treatment of many disorders. The present invention relates to novel compounds differing from the structures disclosed in the art at least for a common new core scaffold. In fact the invention relates to compounds that are characterized by the 2,3-dihydrofuro[3,2-c]pyridine moiety, particularly 2,3-dihydrofuro[3,2-c]pyridin-4-amine, particularly preferably N-(3-(((2,3-dihydrofuro[3,2-c]pyridin-4-yl)amino)methyl)phenyl)formamide and 3-(((2,3-dihydrofuro[3,2-c]pyridin-4-yl)amino)methyl)benzamide derivatives, which are inhibitors of ROCK-I and ROCK-II isoforms of the Rho-associated coiled-coil forming protein kinase (ROCK) that have therapeutically desirable characteristics, are particularly promising in the field of respiratory diseases but not excluding other fields such as that of immune system disorders including Graft-versus-host disease (GVHD), and for some pulmonary diseases including asthma, chronic obstructive pulmonary disease (COPD), idiopathic pulmonary fibrosis (IPF) and pulmonary hypertension (PH) and specifically pulmonary arterial hypertension (PAH). The compounds of the invention may be

prepared for administration by any route consistent with their pharmacokinetic properties. The compound of the invention are active as inhibitors of ROCK-I and ROCK-II isoforms, they are potent and have advantageously other improved properties such as selectivity and other in vitro properties indicative for a preferred route of administration.

## SUMMARY OF THE INVENTION

The present invention is directed to a class of compounds, acting as inhibitors of the Rho Kinase (ROCK), of formula (I)

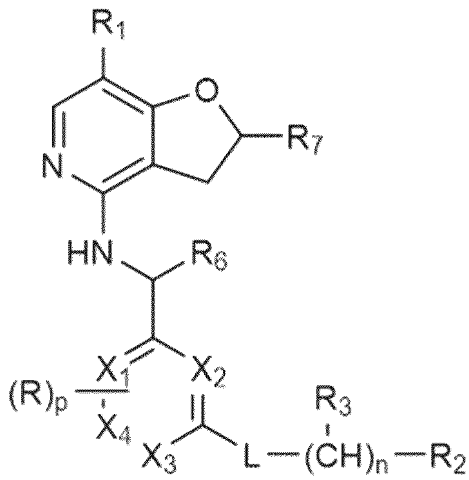

(I)

Wherein the variables $X_1$, $X_2$, $X_3$ and $X_4$, p, R, $R_1$, L, n, $R_2$ and $R_3$, $R_6$ and $R_7$ are as defined in the detailed description of the invention; or pharmaceutically acceptable salts and solvates thereof.

In one aspect, the present invention refers to a compound of formula (I) for use as a medicament. In one aspect the present invention provides the use of a compound of the invention for the manufacture of a medicament.

In a further aspect, the present invention provides the use of a compound of the invention for the preparation of a medicament for the treatment of any disease associated with ROCK enzyme mechanisms, that is to say characterized by ROCK enzyme aberrant activity and/or wherein an inhibition of activity is desirable and in particular through the selective inhibition of the ROCK enzyme isoforms over other Kinases.

In another aspect, the present invention provides a compound of formula (I) for use in a method for prevention and/or treatment of any disease associated with ROCK enzyme mechanisms as above defined, said method comprises administering to a patient in need of such treatment a therapeutically effective amount of a compound of the invention.

In a Particular aspect the compounds of the invention are used alone or combined with other active ingredients and may be administered for the prevention and/or treatment of immune system disorders including Graft-versus-host disease (GVHD), and for pulmonary diseases including asthma, chronic obstructive pulmonary disease (COPD), idiopathic pulmonary fibrosis (IPF) and pulmonary hypertension (PH) and specifically pulmonary arterial hypertension (PAH).

## DETAILED DESCRIPTION OF THE INVENTION

### Definitions

The term "Pharmaceutically acceptable salts" refers to derivatives of compounds of formula (I) wherein the parent compound is suitably modified by converting any of the free acid or basic group, if present, into the corresponding addition salt with any base or acid conventionally intended as being pharmaceutically acceptable.

Suitable examples of said salts may thus include mineral or organic acid addition salts of basic residues such as amino groups, as well as mineral or organic basic addition salts of acid residues such as carboxylic groups.

Cations of inorganic bases which can be suitably used to prepare salts of the invention comprise ions of alkali or alkaline earth metals such as potassium, sodium, calcium or magnesium. Those obtained by reacting the main compound, functioning as a base, with an inorganic or organic acid to form a salt comprise, for example, salts of hydrochloric, hydrobromic, sulfuric, phosphoric, methane sulfonic, camphor sulfonic, acetic, oxalic, maleic, fumaric, succinic and citric acids.

Many organic compounds can form complexes with solvents in which they are reacted or from which they are precipitated or crystallized. These complexes are known as "solvates" which are a further object of the invention. Polymorphs and crystalline forms of compounds of formula (I), or of pharmaceutically acceptable salts, or solvates thereof are a further object of the invention.

The term "Halogen" or "halogen atoms" includes fluorine, chlorine, bromine, and iodine atom ; meaning Fluoro, Chloro, Bromo, Iodo as substituent.

The term "($C_1$-$C_6$)Alkyl" refers to straight-chained or branched alkyl groups wherein the number of carbon atoms is in the range 1 to 6. Particular alkyl groups are for example methyl, ethyl, n-propyl, isopropyl, t-butyl, 3-methylbutyl and the like.

The expressions "($C_1$-$C_6$)Haloalkyl" refer to the above defined "($C_1$-$C_6$)alkyl" groups wherein one or more hydrogen atoms are replaced by one or more halogen atoms, which can be the same or different from each other. Examples include halogenated, poly-halogenated and fully halogenated alkyl groups wherein all of the hydrogen atoms are replaced by halogen atoms, e.g. trifluoromethyl or difluoro methyl groups.

By way of analogy, the terms "($C_1$-$C_6$)Hydroxyalkyl" and "($C_1$-$C_6$)aminoalkyl" refer to the above defined "($C_1$-$C_6$) alkyl" groups wherein one or more hydrogen atoms are replaced by one or more hydroxy (OH) or amino group respectively, examples being hydroxymethyl and aminomethyl and the like.

The definition of aminoalkyl encompasses alkyl groups (i.e. "($C_1$-$C_6$)alkyl" groups) substituted by one or more amino groups ($-NR_8R_9$). An example of aminoalkyl is a mono-aminoalkyl group such as $R_8R_9N$-($C_1$-$C_6$)alkyl. The substituents $R_8$ and $R_9$ are defined as $R_4$ and $R_5$ in the above detailed description of the invention.

Derived expression such as aminoalkoxyl thus refer to the above define aminoalkyl linked to the rest of the molecule from the alkil side via an ether bridge, e.g. with linear representation $-O-(CH_2)_mNR_4R_5$.

The term "($C_3$-$C_{10}$)cycloalkyl" likewise "($C_3$-$C_8$)cycloalkyl" or "($C_3$-$C_6$)cycloalkyl" refers to saturated cyclic hydrocarbon groups containing the indicated number of ring carbon atoms. Examples include cyclopropyl, cyclobutyl, cyclopentyl, cyclohexyl and cycloheptyl, and polycyclic ring systems such as adamantan-yl.

The expression "Aryl" refers to mono, bi- or tri-cyclic carbon ring systems which have 6 to 20, preferably from 6 to 15 ring atoms, wherein at least one ring is aromatic.

The expression "heteroaryl" refers to mono-, bi- or tri-cyclic ring systems with 5 to 20, preferably from 5 to 15 ring atoms, in which at least one ring is aromatic and in which at least one ring atom is a heteroatom (e.g. N, S or O).

Examples of aryl or heteroaryl monocyclic ring systems include, for instance, phenyl, thienyl, pyrrolyl, pyrazolyl, imidazolyl, isoxazolyl, oxazolyl, isothiazolyl, thiazolyl, pyridinyl, pyrimidinyl, pyrazinyl, pyridazinyl, triazinyl, furanyl radicals and the like.

Examples of aryl or heteroaryl bicyclic ring systems include naphthalenyl, biphenylenyl, purinyl, pteridinyl, pyrazolopyrimidinyl, benzotriazolyl, benzoimidazoleyl, quinolinyl, isoquinolinyl, indolyl, isoindolyl, indazolyl, benzothiopheneyl, benzodioxinyl, dihydrobenzodioxinyl, indenyl, dihydro-indenyl, dihydrobenzo[1,4]dioxinyl, benzothiazole-2-yl, dihydrobenzodioxepinyl, benzooxazinyl, 1,2,3,4-tetrahydroisoquinoline-6-yl, 4,5,6,7-tetrahydrothiazolo[4,5-c]pyridine, 4,5,6,7-tetrahydrobenzo[d]thiazol-2-yl, 5,6,7,8-tetrahydro-1,7-naphthyridine, 4,5,6,7-tetrahydrothiazolo[5,4-c]pyridin-2-yl radicals and the like.

Examples of aryl or heteroaryl tricyclic ring systems include fluorenyl radicals as well as benzocondensed derivatives of the aforementioned heteroaryl bicyclic ring systems.

The derived expression "($C_3$-$C_{10}$)heterocycloalkyl" likewise "($C_3$-$C_8$)heterocycloalkyl" or "($C_3$-$C_6$)heterocycloalkyl" refers to saturated or partially unsaturated mono, bi- or tri- cycloalkyl groups of the indicated number of carbons, in which at least one ring carbon atom is replaced by at least one heteroatom (e.g. N, NH, S or O) and/or may bear an -oxo (=O) substituent group. Said heterocycloalkyl (i.e. heterocyclic radical or group) is further optionally substituted on the available points in the ring, namely on a carbon atom, or on an heteroatom available for substitution. Examples of heterocycloalkyl are represented by: oxetanyl, tetrahydro-furanyl, pyrrolidinyl, imidazolidinyl, thiazolidinyl, piperazinyl, piperidinyl, morpholinyl, thiomorpholinyl, dihydro- or tetrahydro-pyridinyl, tetrahydropyranyl, pyranyl, 2H- or 4H-pyranyl, dihydro- or tetrahydrofuranyl, dihydroisoxazolyl, pyrrolidin-2-one-yl, dihydropyrrolyl, 5-oxopyrrolidin-3-yl, (1R,5S,6r)-3-oxabicyclo[3.1.0]hexan-6-yl, octahydrocyclopenta[c]pyrrol-5-yl, 4,5,6,7-tetrahydropyrazolo[1,5-a]pyrazin-2-yl; 4,5,6,7-tetrahydrothiazolo[5,4-c]pyridin-2-yl radicals and the like.

The term "Aryl($C_1$-$C_6$)alkyl" refers to an aryl ring linked to a straight-chained or branched alkyl group wherein the number of constituent carbon atoms is in the range from 1 to 6, e.g. phenylmethyl (i.e. benzyl), phenylethyl or phenylpropyl.

Likewise the term "Heteroaryl($C_1$-$C_6$)alkyl" refers to an heteroaryl ring linked to a straight-chained or branched alkyl group wherein the number of constituent carbon atoms is in the range from 1 to 6, e.g. furanylmethyl.

The term "alkanoyl", refers to HC(O)- or to alkylcarbonyl groups (e.g. ($C_1$-$C_6$)alkylC(O)-) wherein the group "alkyl" has the meaning above defined. Examples include formyl, acetyl, propanoyl, butanoyl.

The term "($C_1$-$C_{10}$) alkoxy" or "($C_1$-$C_{10}$) alkoxyl", likewise "($C_1$-$C_6$) alkoxy" or "($C_1$-$C_6$) alkoxyl" etc., refers to a straight or branched hydrocarbon of the indicated number of carbons, linked to the rest of the molecule through an oxygen bridge. "($C_1$-$C_6$)Alkylthio" refers to the above hydrocarbon linked through a sulfur bridge.

The derived expression "($C_1$-$C_6$)haloalkoxy" or "($C_1$-$C_6$)haloalkoxyl" refers to the above defined haloalkyl, linked through an oxygen bridge. An example of ($C_1$-$C_6$)haloalkoxy is trifluoromethoxy.

Likewise derived expression "($C_3$-$C_6$)heterocycloalkyl-($C_1$-$C_6$)alkyl" and "($C_3$-$C_6$)cycloalkyl-($C_1$-$C_6$)alkyl" refer to the above defined heterocycloalkyl and cycloalkyl groups linked to the rest of the molecule via an alkyl group of the indicated number of carbons, corresponding e.g. to linear formula ($C_3$-$C_6$)heterocycloalkyl-$(CH_2)_m$- or ($C_3$-$C_6$)cycloalkyl-$(CH_2)_m$- for example piperidin-4-yl-methyl, cyclohexylethyl.

The derived expression "($C_1$-$C_6$)alkoxy-($C_1$-$C_6$)alkyl" refers to the above defined alkoxy group linked to the rest of the molecule via an alkyl group of the indicated number of carbons, for example methoxymethyl.

Likewise "($C_1$-$C_6$)haloalkoxy ($C_1$-$C_6$)alkyl" refers to the above defined ($C_1$-$C_6$)haloalkoxy" group linked to the rest of the molecule via an alkyl group of the indicated number of carbons, for example difluoromethoxypropyl.

Derived expression "($C_3$-$C_8$)heterocycloalkyl-($C_1$-$C_6$)alkoxyl" or "($C_3$-$C_6$)heterocycloalkyl-($C_1$-$C_6$)alkoxyl" and "($C_3$-$C_8$)cycloalkyl-($C_1$-$C_6$)alkoxyl" or "($C_3$-$C_6$)cycloalkyl-($C_1$-$C_6$)alkoxyl" refer to the above defined heterocycloalkyl and cycloalkyl groups linked to the rest of the molecule via an alkoxyl group as above defined of the indicated number of carbons, corresponding e.g. to linear formula ($C_3$-$C_8$)cycloalkyl - $(CH_2)_m$O- ($C_3$-$C_8$)heterocycloalkyl -$(CH_2)_m$O- for example piperazin-1-yl-ethoxyl.

An oxo moiety is represented by (O) as an alternative to the other common representation, e.g. (=O). Thus, in terms of general formula, the carbonyl group is herein preferably represented as -C(O)- as an alternative to the other common representations such as -CO-, -(CO)- or -C(=O)-. In general the bracketed group is a lateral group, not included into the chain, and brackets are used, when deemed useful, to help disambiguating linear chemical formulas; e.g. the sulfonyl group $-SO_2-$ might be also represented as-$S(O)_2$- to disambiguate e.g. with respect to the sulfinic group -S(O)O-.

Likewise the group $-(CHR_3)_n-R_2$ herein is a linear representation of the terminal part of the charachterizing group

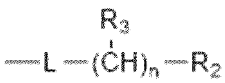

found in formula (I) and (Ia).

When a numerical index the statement (value) "p is zero" or "p is 0" means that the substituent or group bearing the index p (e.g. (R)p) is absent, that is to say no substituent, other than H when needed, is present. Likewise when the index is attached to a bridging divalent group (e.g. $(CH_2)$n) the statement "n in each occurrence is zero..." or "n is 0" means that the bridging group is absent, that is to say it is a bond.

Whenever basic amino or quaternary ammonium groups are present in the compounds of formula (I), physiological acceptable anions, selected among chloride, bromide, iodide, trifluoroacetate, formate, sulfate, phosphate, methanesulfonate, nitrate, maleate, acetate, citrate, fumarate, tartrate, oxalate, succinate, benzoate, p-toluenesulfonate, pamoate and naphthalene disulfonate may be present. Likewise, in the presence of acidic groups such as COOH groups, corresponding physiological cation salts may be present as well, for instance including alkaline or alkaline earth metal ions.

Compounds of formula (I) when they contain one or more stereogenic center, may exist as optical stereoisomers.

Where the compounds of the invention have at least one stereogenic center, they may accordingly exist as enantiomers. Where the compounds of the invention possess two or more stereogenic centers, they may additionally exist as diastereoisomers. It is to be understood that all such single enantiomers, diastereoisomers and mixtures thereof in any proportion are encompassed within the scope of the present invention. The absolute configuration (R) or (S) for carbon bearing a stereogenic center is assigned on the basis of Cahn-Ingold-Prelog nomenclature rules based on groups' priorities.

"Single stereoisomer", "single diastereoisomer" or "single enantiomer", when reported near the chemical name of a compound indicate that the isomer was isolated as single diastereoisomer or enantiomer (e.g via chiral chromatography) but the absolute configuration at the relevant stereogenic center was not determined/assigned.

Atropisomers result from hindered rotation about single bonds where the steric strain barrier to rotation is high enough to allow for the isolation of the conformers (Bringmann G et al, Angew. Chemie Int. Ed. 44 (34), 5384-5427, 2005. doi:10.1002/anie.200462661).

Oki defined atropisomers as conformers that interconvert with a half-life of more than 1000 seconds at a given temperature (Oki M, Topics in Stereochemistry 14, 1-82, 1983).

Atropisomers differ from other chiral compounds in that in many cases they can be equilibrated thermally whereas in the other forms of chirality isomerization is usually only possible chemically.

Separation of atropisomers is possible by chiral resolution methods such as selective crystallization. In an atropo-enantioselective or atroposelective synthesis one atropisomer is formed at the expense of the other. Atroposelective synthesis may be carried out by use of chiral auxiliaries like a Corey Bakshi Shibata (CBS) catalyst, an asymmetric catalyst derived from proline, or by approaches based on thermodynamic equilibration when an isomerization reaction favors one atropisomer over the other.

Racemic forms of compounds of formula (I) as well as the individual atropisomers (substantially free of its corresponding enantiomer) and stereoisomer-enriched atropisomer mixtures are included in the scope of the present invention.

The invention further concerns the corresponding deuterated derivatives of compounds of formula (I). In the context of the present invention, deuterated derivative means that at least one position occupied by a hydrogen atom is occupied by deuterium in an amount above its natural abundance. Preferably, the percent of deuterium at that position is at least 90%, more preferably at least 95%, even more preferably 99%.

All preferred groups or embodiments described above and herebelow for compounds of formula (I) may be combined among each other and apply as well *mutatis mutandis.*

As above mentioned, the present invention refers to compounds of general formula (I), acting as ROCK inhibitors, to processes for the preparation thereof, pharmaceutical compositions comprising them either alone or in combination with one or more active ingredient, in admixture with one or more pharmaceutically acceptable carriers.

In a first aspect the present invention is directed to a class of compounds of formula (I)

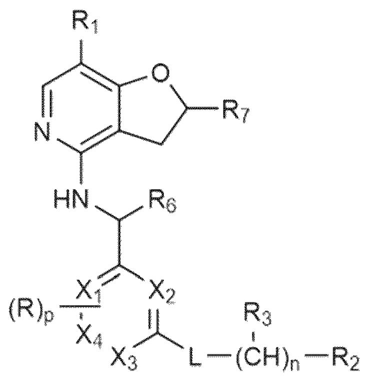

(I)

wherein

**$X_1$, $X_2$, $X_3$** and **$X_4$** are all CH or one of $X_1$, $X_2$, $X_3$ and $X_4$ is N and the others are CH;
p is zero or an integer from 1 to 4;
each R, when present, is in each occurrence independently selected from $(C_1-C_6)$alkyl and halogen selected from F, Cl, Br and I; wherein preferably R is F, Cl or methyl;
$R_1$ is pyrimidinyl, preferably pyrimidin-4yl, substituted by one or more group selected from $-(CH_2)_mNH_2$; particularly preferably $R_1$ is 2-aminopyrimidin-4-yl;
L is -C(O)NH- or -NHC(O)- ;
n is in each occurrence independently 0 or an integer selected from 1, 2 or 3;
$R_2$ and $R_3$ are in each occurrence independently selected from the group consisting of
-H,
halogen,
-OH,
$-(CH_2)_mNR_4R_5$,
$(C_1-C_6)$alkyl,
$(C_1-C_6)$hydroxyalkyl,
$(C_1-C_6)$ alkoxy,
$(C_1-C_6)$ alkoxy $(C_1-C_6)$alkyl,
$(C_1-C_6)$haloalkyl,
$(C_1-C_6)$haloalkoxy,
$(C_1-C_6)$haloalkoxy $(C_1-C_6)$alkyl,
$(C_3-C_{10})$cycloalkyl,
aryl, heteroaryl and $(C_3-C_6)$heterocycloalkyl,
each of which cycloalkyl, aryl, heteroaryl and heterocycloalkyl
is in its turn optionally and independently substituted with one or more groups selected from
halogen,
-OH,
$(C_1-C_6)$alkyl,

$(C_1-C_6)$hydroxyalkyl,
$(C_1-C_6)$ alkoxy,
$(C_1-C_6)$ alkoxy $(C_1-C_6)$alkyl,
$(C_1-C_6)$haloalkyl,
$(C_1-C_6)$haloalkoxy,
$-(CH_2)_mNR_4R_5$,
$-O-(CH_2)_mNR_4R_5$,
alkanoyl,
aryl, heteroaryl, cycloalkyl,
aryl-$(C_1-C_6)$alkyl,
heteroaryl-$(C_1-C_6)$alkyl,
$(C_3-C_8)$heterocycloalkyl, preferably $(C_3-C_6)$heterocycloalkyl,
$(C_3-C_8)$heterocycloalkyl-$(C_1-C_6)$alkyl, preferably $(C_3-C_6)$heterocycloalkyl-$(CH_2)_{m-}$,
$(C_3-C_8)$cycloalkyl-$(C_1-C_6)$alkyl,
each of said aryl, heteroaryl, cycloalkyl, heterocycloalkyl is still further optionally substituted by one or more group selected independently from halogen selected from F, Cl, Br and I, preferably F, -OH, $(C_1-C_8)$alkyl, $(C_1-C_6)$haloalkyl, $(C_1-C_6)$hydroxyalkyl;
m is in each occurrence independently 0 or an integer selected from 1, 2 or 3;
$R_4$ and $R_5$, the same or different, are selected from the group consisting of
-H,
$(C_1-C_6)$alkyl,
$(C_1-C_6)$haloalkyl,
$(C_1-C_6)$hydroxyalkyl,
$(C_3-C_6)$heterocycloalkyl;
$R_6$ and $R_7$ are independently selected from the group consisting of -H, $(C_1-C_6)$alkyl;
single enantiomers, diastereoisomers and mixtures thereof in any proportion
and pharmaceutically acceptable salts and solvates thereof.

In a preferred embodiment the invention is directed to a compound of formula (I) wherein $X_1$, $X_3$ and $X_4$ are all CH groups and $X_2$ is a CH group or a nitrogen atom;

$R_1$ is 2-aminopyrimidin-4-yl;
all the other variables being as defined above.

Said preferred group of compounds is represented by the formula (Ia)

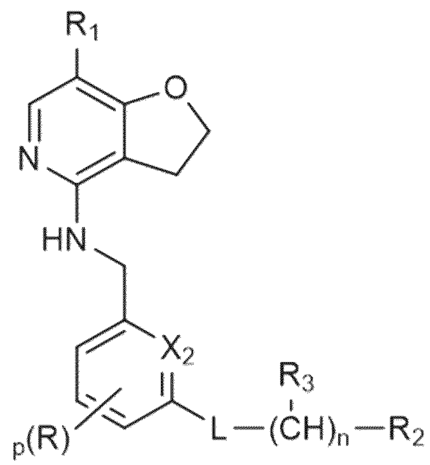

(Ia)

Particularly preferred are compounds of formula (I) as above defined,

wherein $X_1$, $X_2$, $X_3$, $X_4$ are all CH group;
each R, when present, is in each occurrence independently selected from F, Cl, Br and I; wherein preferably R is F;

$R_1$ is pyrimidinyl substituted by $-NH_2$; particularly preferably $R_1$ is 2-aminopyrimidin-4-yl;
L is -C(O)NH-;
n is 0 (i.e. $R_3$ is absent);
$R_2$ is in each occurrence independently selected from the group consisting of $(C_1-C_6)$alkyl,
$(C_1-C_6)$hydroxyalkyl
$(C_1-C_6)$haloalkyl,
$(C_1-C_6)$ alkoxy $(C_1-C_6)$alkyl,
$(C_1-C_6)$haloalkoxy $(C_1-C_6)$alkyl,
all the other variables being as defined above,
single enantiomers, diastereoisomers and mixtures thereof in any proportion
and/or pharmaceutically acceptable salts and solvates thereof.

Said particularly preferred group of compounds is represented by the formula (Ib)

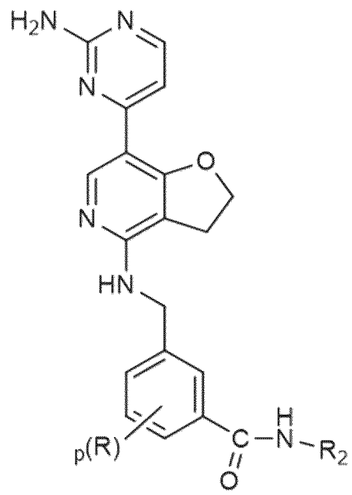

**(Ib)**

Particularly preferred in this last embodiment is a compound of formula (Ib) wherein $R_2$ is selected from methyl, ethyl, propyl, 2-hydroxy-2-methylpropyl, 3-methoxypropyl, 2-methoxyethyl, 2-ethoxyethyl, 3-isopropoxypropyl, 3-methoxy-3-methylbutyl, 2-methoxy-2-methylpropyl, 3-fluoropropyl, 2-fluoro-2-methylpropyl, 2,2-difluoropropyl, 3,3-difluoropropyl;

all the other variables and substitution being as defined above,
single enantiomers, diastereoisomers and mixtures thereof in any proportion
and/or pharmaceutically acceptable salts and solvates thereof.

In another preferred embodiment the invention is directed to a compound of formula (I) wherein $\mathbf{X_1}$, $\mathbf{X_2}$, $\mathbf{X_3}$ and $\mathbf{X_4}$ are all CH;

p is zero or an integer from 1 to 4;
each R, when present, is halogen in each occurrence independently selected from F, Cl, Br and I, wherein preferably R is F;
$R_1$ is pyrimidinyl substituted by $-NH_2$; particularly preferably $R_1$ is 2-aminopyrimidin-4-yl;
L is -C(O)NH-;
n is in each occurrence independently 0 or an integer selected from 1, 2 or 3;
$R_3$, when present, is H,
and
$R_2$ is selected from the group consisting of
heteroaryl and $(C_3-C_6)$heterocycloalkyl, preferably $R_2$ is an heterocycloalkyl,
each of which heteroaryl and heterocycloalkyl
is in its turn optionally substituted with one or more groups selected from $(C_1-C_6)$alkyl,
$(C_1-C_6)$hydroxyalkyl,

$(C_1-C_6)$ alkoxy,
$(C_1-C_6)$ alkoxy $(C_1-C_6)$alkyl,
heteroaryl-$(C_1-C_6)$alkyl,
$(C_3-C_8)$cycloalkyl-$(C_1-C_6)$alkyl,
$-O-(CH_2)_mNR_4R_5$,
$(C_3-C_6)$heterocycloalkyl m is in each occurrence independently 0 or an integer selected from 1, 2 or 3;
$R_4$ and $R_5$, the same or different, are selected from the group consisting of
-H,
$(C_1-C_6)$alkyl,
$(C_1-C_6)$haloalkyl,
$(C_1-C_6)$hydroxyalkyl,
all the other variables being as defined above,
single enantiomers, diastereoisomers and mixtures thereof in any proportion
and/or pharmaceutically acceptable salts and solvates thereof.

Particularly preferred in this last embodiment is a compound wherein $R_2$ is selected from , methoxypyridinyl, ((dimethylamino)ethoxy)pyridin-2-yl, (piperazinyl)pyridinyl, tetrahydrofuranyl, dimethyltetrahydrofuranyl, oxetan-2-yl;

all the other variables and substitution being as defined above,
or pharmaceutically acceptable salts and solvates thereof.

Thus, a group of particularly preferred compounds are

| Example | Chemical Name |
|---|---|
| 3 | 3-(((7-(2-aminopyrimidin-4-yl)-2,3-dihydrofuro[3,2-c]pyridin-4-yl)amino)methyl)-N-(5,5-dimethyltetrahydrofuran-3-yl)benzamide |
| 38 | 3-(((7-(2-aminopyrimidin-4-yl)-2,3-dihydrofuro[3,2-c]pyridin-4-yl)amino)methyl)-N-(oxetan-2-ylmethyl) benzamide |
| 42 | (R)-3-(((7-(2-aminopyrimidin-4-yl)-2,3-dihydrofuro[3,2-c]pyridin-4-yl)amino)methyl)-N-((tetrahydrofuran-2-yl)methyl)benzamide |
| 43 | (S)-3-(((7-(2-aminopyrimidin-4-yl)-2,3-dihydrofuro[3,2-c]pyridin-4-yl)amino)methyl)-N-((tetrahydrofuran-2-yl)methyl)benzamide |
| 53 | 3-(((7-(2-aminopyrimidin-4-yl)-2,3-dihydrofuro[3,2-c]pyridin-4-yl)amino)methyl)-N-(5-(piperazin-1-yl) pyridin-2-yl)benzamide |
| 51 | 3-(((7-(2-aminopyrimidin-4-yl)-2,3-dihydrofuro[3,2-c]pyridin-4-yl)amino)methyl)-N-(5-methoxypyridin-2-yl)benzamide |
| 52 | 3-(((7-(2-aminopyrimidin-4-yl)-2,3-dihydrofuro[3,2-c]pyridin-4-yl)amino)methyl)-N-(5-(2-(dimethylamino)ethoxy)pyridin-2-yl)benzamide |

A further preferred group of compounds according to the invention are those of formula (I) wherein

$\mathbf{X_1}$, $\mathbf{X_2}$, $\mathbf{X_3}$ and $\mathbf{X_4}$ are all CH or $X_2$, is N and the others are CH;
p is zero or 1;
each R, when present, is F;
$R_1$ is 2-aminopyrimidin-4-yl;
L is -C(O)NH- or -NHC(O)- ;
n is in each occurrence independently 0 or an integer selected from 1, 2 or 3;
$R_2$ and $R_3$ are in each occurrence independently selected from the group consisting of
-H,
$(C_1-C_6)$alkyl which is methyl, propyl, isopropyl, tert-butyl,
$(C_1-C_6)$hydroxyalkyl which is 2-hydroxy-2-methylpropyl,
$(C_1-C_6)$ alkoxy $(C_1-C_6)$alkyl which is 3-methoxypropyl, 2-ethoxyethyl, 3-isopropoxypropyl, 3-methoxy-3-methylbutyl, 2-methoxyethyl, 2-methoxy-2-methylpropyl,
$(C_1-C_6)$haloalkyl which is 2,2-difluoroethyl, 2,2,2-trifluoroethyl, 2,2,2-trifluoromethyl, 2-fluoro-2-methylpropyl, 3-fluoropropyl, 2,2-difluoropropyl, 3,3-difluoropropyl,
$(C_1-C_6)$haloalkoxy $(C_1-C_6)$alkyl which is 3-(difluoromethoxy)propyl,
$(C_3-C_{10})$cycloalkyl which is cyclopropyl, cyclobutyl,
Heteroaryl which is pyridinyl, and
$(C_3-C_6)$heterocycloalkyl which is azetidin-3-yl, piperidinyl, morpholinyl, 5-oxopyrrolidin-3-yl, (1R,5S,6r)-3-oxabicyclo [3.1.0]hexan-6-yl, tetrahydrofuranyl, tetrahydro-2H-pyran-4-yl,
each of which cycloalkyl, heteroaryl and heterocycloalkyl
is in its turn optionally and independently substituted with one or more groups selected from
halogen which is selected from F, Cl, Br, I,
$(C_1-C_6)$alkyl which is methyl, tert-butyl,
$(C_1-C_6)$ alkoxy which is methoxy,
$(C_1-C_6)$ alkoxy $(C_1-C_6)$alkyl which is methoxymethyl,
$-O-(CH_2)_mNR_4R_5$, which is 2-(dimethylamino)ethoxy, wherein m is 2 and $R_4$ and $R_5$ are methyl;
$(C_3-C_6)$heterocycloalkyl, which is piperazin-1-yl
$R_6$ and $R_7$ are -H,
single enantiomers, diastereoisomers and mixtures thereof in any proportion and/or pharmaceutically acceptable salts and solvates thereof.

The invention also provides a pharmaceutical composition comprising a compound of formula (I), or a pharmaceutically acceptable salt thereof in admixture with one or more pharmaceutically acceptable carriers or excipients, either alone or in combination with one or more further active ingredient as detailed below.

According to preferred embodiments, the invention provides the compounds listed in the table below and pharmaceutical acceptable salts thereof.

| Example | Chemical Name |
|---|---|
| 1 | 3-(((7-(2-aminopyrimidin-4-yl)-2,3-dihydrofuro[3,2-c]pyridin-4-yl)amino)methyl)-N-cyclobutylbenzamide |
| 2 | 3-(((7-(2-aminopyrimidin-4-yl)-2,3-dihydrofuro[3,2-c]pyridin-4-yl)amino)methyl)-N-((tetrahydro-2H-pyran-4-yl)methyl)benzamide |
| 3 | 3-(((7-(2-aminopyrimidin-4-yl)-2,3-dihydrofuro[3,2-c]pyridin-4-yl)amino)methyl)-N-(5,5-dimethyltetrahydrofuran-3-yl)benzamide |
| 4 | 3-(((7-(2-aminopyrimidin-4-yl)-2,3-dihydrofuro[3,2-c]pyridin-4-yl)amino)methyl)-N-cyclopropylbenzamide |
| 5 | 3-(((7-(2-aminopyrimidin-4-yl)-2,3-dihydrofuro[3,2-c]pyridin-4-yl)amino)methyl)-N-(2-(piperidin-1-yl)ethyl)benzamide |
| 6 | 3-(((7-(2-aminopyrimidin-4-yl)-2,3-dihydrofuro[3,2-c]pyridin-4-yl)amino)methyl)-N-(3-(methoxy-d3)propyl)benzamide |
| 7 | 3-(((7-(2-aminopyrimidin-4-yl)-2,3-dihydrofuro[3,2-c]pyridin-4-yl)amino)methyl)-N-(tetrahydro-2H-pyran-4-yl)benzamide |
| 8 | 3-(((7-(2-aminopyrimidin-4-yl)-2,3-dihydrofuro[3,2-c]pyridin-4-yl)amino)methyl)-N-(3-methoxypropyl)benzamide |
| 9 | 3-(((7-(2-aminopyrimidin-4-yl)-2,3-dihydrofuro[3,2-c]pyridin-4-yl)amino)methyl)-N-(2-methoxyethyl)benzamide |
| 10 | 3-(((7-(2-aminopyrimidin-4-yl)-2,3-dihydrofuro[3,2-c]pyridin-4-yl)amino)methyl)-N-(3,3-difluorocyclobutyl)benzamide |
| 11 | 3-(((7-(2-aminopyrimidin-4-yl)-2,3-dihydrofuro[3,2-c]pyridin-4-yl)amino)methyl)-N-isopropylbenzamide |
| 12 | 3-(((7-(2-aminopyrimidin-4-yl)-2,3-dihydrofuro[3,2-c]pyridin-4-yl)amino)methyl)-N-(2,2,2-trifluoroethyl)benzamide |
| 13 | 3-(((7-(2-aminopyrimidin-4-yl)-2,3-dihydrofuro[3,2-c]pyridin-4-yl)amino)methyl)-N-(1-methylpiperidin-4-yl)benzamide |
| 14 | 3-(((7-(2-aminopyrimidin-4-yl)-2,3-dihydrofuro[3,2-c]pyridin-4-yl)amino)methyl)-N-(3-(difluoromethoxy)propyl)benzamide |

(continued)

| Example | Chemical Name |
|---|---|
| 15 | 3-(((7-(2-aminopyrimidin-4-yl)-2,3-dihydrofuro[3,2-c]pyridin-4-yl)amino)methyl)-N-(3-isopropoxypropyl)benzamide |
| 16 | 3-(((7-(2-aminopyrimidin-4-yl)-2,3-dihydrofuro[3,2-c]pyridin-4-yl)amino)methyl)-N-(3-morpholinopropyl)benzamide |
| 17 | 3-(((7-(2-aminopyrimidin-4-yl)-2,3-dihydrofuro[3,2-c]pyridin-4-yl)amino)methyl)-N-(2-hydroxy-2-methylpropyl)benzamide |
| 18 | 3-(((7-(2-aminopyrimidin-4-yl)-2,3-dihydrofuro[3,2-c]pyridin-4-yl)amino)methyl)-N-((1s,3s)-3-methoxycyclobutyl)benzamide |
| 19 | 3-(((7-(2-aminopyrimidin-4-yl)-2,3-dihydrofuro[3,2-c]pyridin-4-yl)amino)methyl)-N-(2-((2R,6S)-2,6-dimethylpiperidin-1-yl)ethyl)benzamide |
| 20 | 3-(((7-(2-aminopyrimidin-4-yl)-2,3-dihydrofuro[3,2-c]pyridin-4-yl)amino)methyl)-N-(2-ethoxyethyl)benzamide |
| 21 | 3-(((7-(2-aminopyrimidin-4-yl)-2,3-dihydrofuro[3,2-c]pyridin-4-yl)amino)methyl)-N-methylbenzamide |
| 22 | 3-(((7-(2-aminopyrimidin-4-yl)-2,3-dihydrofuro[3,2-c]pyridin-4-yl)amino)methyl)-N-(1-methylcyclopropyl)benzamide |
| 23 | 3-(((7-(2-aminopyrimidin-4-yl)-2,3-dihydrofuro[3,2-c]pyridin-4-yl)amino)methyl)-N-((1R,5S,6r)-3-oxabicyclo[3.1.0]hexan-6-yl)benzamide |
| 24 | 3-(((7-(2-aminopyrimidin-4-yl)-2,3-dihydrofuro[3,2-c]pyridin-4-yl)amino)methyl)-N-(1-(oxetan-3-yl)azetidin-3-yl)benzamide |
| 25 | 3-(((7-(2-aminopyrimidin-4-yl)-2,3-dihydrofuro[3,2-c]pyridin-4-yl)amino)methyl)-N-(cyclopropylmethyl) benzamide |
| 26 | 3-(((7-(2-aminopyrimidin-4-yl)-2,3-dihydrofuro[3,2-c]pyridin-4-yl)amino)methyl)-N-((1s,3s)-3-(difluoromethoxy)cyclobutyl)benzamide |
| 27 | 3-(((7-(2-aminopyrimidin-4-yl)-2,3-dihydrofuro[3,2-c]pyridin-4-yl)amino)methyl)-N-(2-methoxy-2-methylpropyl)benzamide |
| 28 | 3-(((7-(2-aminopyrimidin-4-yl)-2,3-dihydrofuro[3,2-c]pyridin-4-yl)amino)methyl)-N-((1-(methoxymethyl)cyclopropyl)methyl)benzamide |
| 29 | 3-(((7-(2-aminopyrimidin-4-yl)-2,3-dihydrofuro[3,2-c]pyridin-4-yl)amino)methyl)-N-(3-fluoropropyl)benzamide |
| 30 | 3-(((7-(2-aminopyrimidin-4-yl)-2,3-dihydrofuro[3,2-c]pyridin-4-yl)amino)methyl)-N-((1-methylcyclopropyl)methyl)benzamide |
| 31 | 3-(((7-(2-aminopyrimidin-4-yl)-2,3-dihydrofuro[3,2-c]pyridin-4-yl)amino)methyl)-N-(2-fluoro-2-methylpropyl)benzamide |
| 32 | 3-(((7-(2-aminopyrimidin-4-yl)-2,3-dihydrofuro[3,2-c]pyridin-4-yl)amino)methyl)-N-((1-(tert-butyl)-5-oxopyrrolidin-3-yl)methyl)benzamide (Enantiomer 1) |
| 33 | 3-(((7-(2-aminopyrimidin-4-yl)-2,3-dihydrofuro[3,2-c]pyridin-4-yl)amino)methyl)-N-((1-(tert-butyl)-5-oxopyrrolidin-3-yl)methyl)benzamide (Enantiomer 2) |
| 34 | 3-(((7-(2-aminopyrimidin-4-yl)-2,3-dihydrofuro[3,2-c]pyridin-4-yl)amino)methyl)-N-((4-methylmorpholin-2-yl)methyl)benzamide (Enantiomer 1) |
| 35 | 3-(((7-(2-aminopyrimidin-4-yl)-2,3-dihydrofuro[3,2-c]pyridin-4-yl)amino)methyl)-N-((4-methylmorpholin-2-yl)methyl)benzamide (Enantiomer 2) |
| 36 | 3-(((7-(2-aminopyrimidin-4-yl)-2,3-dihydrofuro[3,2-c]pyridin-4-yl)amino)methyl)-N-(3-methoxy-3-methylbutyl)benzamide |
| 37 | 3-(((7-(2-aminopyrimidin-4-yl)-2,3-dihydrofuro[3,2-c]pyridin-4-yl)amino)methyl)-N-((1R,2S)-2-fluorocyclopropyl)benzamide |

(continued)

| Example | Chemical Name |
|---|---|
| 38 | 3-(((7-(2-aminopyrimidin-4-yl)-2,3-dihydrofuro[3,2-c]pyridin-4-yl)amino)methyl)-N-(oxetan-2-ylmethyl) benzamide |
| 39 | 3-(((7-(2-aminopyrimidin-4-yl)-2,3-dihydrofuro[3,2-c]pyridin-4-yl)amino)methyl)-N-(((1r,3r)-3-methoxycyclobutyl)methyl)benzamide |
| 40 | 3-(((7-(2-aminopyrimidin-4-yl)-2,3-dihydrofuro[3,2-c]pyridin-4-yl)amino)methyl)-N-propylbenzamide |
| 41 | 3-(((7-(2-aminopyrimidin-4-yl)-2,3-dihydrofuro[3,2-c]pyridin-4-yl)amino)methyl)-N-((1r,3r)-3-methoxycyclobutyl)benzamide |
| 42 | (R)-3-(((7-(2-aminopyrimidin-4-yl)-2,3-dihydrofuro [3,2-c]pyridin-4-yl)amino)methyl)-N-((tetrahydrofuran-2-yl)methyl)benzamide |
| 43 | (S)-3-(((7-(2-aminopyrimidin-4-yl)-2,3-dihydrofuro[3,2-c]pyridin-4-yl)amino)methyl)-N-((tetrahydrofuran-2-yl)methyl)benzamide |
| 44 | 3-(((7-(2-aminopyrimidin-4-yl)-2,3-dihydrofuro[3,2-c]pyridin-4-yl)amino)methyl)-N-(2,2-difluoropropyl) benzamide |
| 45 | Trans 3-(((7-(2-aminopyrimidin-4-yl)-2,3-dihydrofuro[3,2-c]pyridin-4-yl)amino)methyl)-N-(2-fluorocyclopropyl)benzamide (Enantiomer 1) |
| 46 | Trans 3-(((7-(2-aminopyrimidin-4-yl)-2,3-dihydrofuro[3,2-c]pyridin-4-yl)amino)methyl)-N-(2-fluorocyclopropyl)benzamide (Enantiomer 2) |
| 47 | 3-(((7-(2-aminopyrimidin-4-yl)-2,3-dihydrofuro[3,2-c]pyridin-4-yl)amino)methyl)-N-((1S,2R)-2-fluorocyclopropyl)benzamide |
| 48 | 3-(((7-(2-aminopyrimidin-4-yl)-2,3-dihydrofuro[3,2-c]pyridin-4-yl)amino)methyl)-N-((1s,3s)-3-fluorocyclobutyl)benzamide |
| 49 | 3-(((7-(2-aminopyrimidin-4-yl)-2,3-dihydrofuro[3,2-c]pyridin-4-yl)amino)methyl)-N-((1r,3r)-3-fluorocyclobutyl)benzamide |
| 50 | 3-(((7-(2-aminopyrimidin-4-yl)-2,3-dihydrofuro[3,2-c]pyridin-4-yl)amino)methyl)-N-(3,3-difluoropropyl) benzamide |
| 51 | 3-(((7-(2-aminopyrimidin-4-yl)-2,3-dihydrofuro[3,2-c]pyridin-4-yl)amino)methyl)-N-(5-methoxypyridin-2-yl)benzamide |
| 52 | 3-(((7-(2-aminopyrimidin-4-yl)-2,3-dihydrofuro[3,2-c]pyridin-4-yl)amino)methyl)-N-(5-(2-(dimethylamino)ethoxy)pyridin-2-yl)benzamide |
| 53 | 3-(((7-(2-aminopyrimidin-4-yl)-2,3-dihydrofuro[3,2-c]pyridin-4-yl)amino)methyl)-N-(5-(piperazin-1-yl) pyridin-2-yl)benzamide |
| 54 | 3-(((7-(2-aminopyrimidin-4-yl)-2,3-dihydrofuro[3,2-c]pyridin-4-yl)amino)methyl)-2-fluoro-N-methylbenzamide |
| 55 | 3-(((7-(2-aminopyrimidin-4-yl)-2,3-dihydrofuro[3,2-c]pyridin-4-yl)amino)methyl)-2-fluoro-N-(3-methoxypropyl)benzamide |
| 56 | 3-(((7-(2-aminopyrimidin-4-yl)-2,3-dihydrofuro[3,2-c]pyridin-4-yl)amino)methyl)-2-fluoro-N-(tetrahydro-2H-pyran-4-yl)benzamide |
| 57 | 5-(((7-(2-aminopyrimidin-4-yl)-2,3-dihydrofuro[3,2-c]pyridin-4-yl)amino)methyl)-2-fluoro-N-(3-methoxypropyl)benzamide |
| 58 | 5-(((7-(2-aminopyrimidin-4-yl)-2,3-dihydrofuro[3,2-c]pyridin-4-yl)amino)methyl)-2-fluoro-N-(tetrahydro-2H-pyran-4-yl)benzamide |
| 59 | 3-(((7-(2-aminopyrimidin-4-yl)-2,3-dihydrofuro[3,2-c]pyridin-4-yl)amino)methyl)-5-fluoro-N-methylbenzamide |
| 60 | 3-(((7-(2-aminopyrimidin-4-yl)-2,3-dihydrofuro[3,2-c]pyridin-4-yl)amino)methyl)-5-fluoro-N-(3-methox-ypropyl)benzamide |

(continued)

| Example | Chemical Name |
|---|---|
| 61 | 3-(((7-(2-aminopyrimidin-4-yl)-2,3-dihydrofuro[3,2-c]pyridin-4-yl)amino)methyl)-4-fluoro-N-(tetrahydro-2H-pyran-4-yl)benzamide |
| 62 | 3-(((7-(2-aminopyrimidin-4-yl)-2,3-dihydrofuro[3,2-c]pyridin-4-yl)amino)methyl)-4-fluoro-N-(3-methoxypropyl)benzamide |
| 63 | 6-(((7-(2-aminopyrimidin-4-yl)-2,3-dihydrofuro[3,2-c]pyridin-4-yl)amino)methyl)-N-methylpicolinamide |
| 64 | N-(3-(((7-(2-aminopyrimidin-4-yl)-2,3-dihydrofuro[3,2-c]pyridin-4-yl)amino)methyl)phenyl)-4-methoxybutanamide |

The compounds of the invention, including all the compounds hereabove listed, can be prepared from readily available starting materials using the following general methods and procedures or by using slightly modified processes readily available to those of ordinary skill in the art. Although a particular embodiment of the present invention may be shown or described herein, those skilled in the art will recognize that all embodiments or aspects of the present invention can be prepared using the methods described herein or by using other known methods, reagents and starting materials. When typical or preferred process conditions (i.e. reaction temperatures, times, mole ratios of reactants, solvents, pressures, etc.) are given, other process conditions can also be used unless otherwise stated. While the optimum reaction conditions may vary depending on the particular reactants or solvent used, such conditions can be readily determined by those skilled in the art by routine optimization procedures.

Thus, processes of preparation described below and reported in the following schemes should not be viewed as limiting the scope of the synthetic methods available for the preparation of the compounds of the invention.

In some cases a step is needed in order to mask or protect sensitive or reactive moieties, generally known protective groups (PG) could be employed, in accordance with general principles of chemistry (Protective group in organic syntheses, 3rd ed. T. W. Greene, P. G. M. Wuts). A suitable protective group for intermediates requiring protection of a carboxylic acid (herein reported as $PG_1$) can be $C_1$-$C_4$ esters ($PG_1$: methyl, isopropyl, tert-butyl or ethyl), preferably methyl. A suitable protective group for intermediates requiring the amino group protection (herein reported as $PG_2$) can be carbamates such as tert-butylcarbamate ($PG_2$: tert-butoxycarbonyl or Boc), benzylcarbamate ($PG_2$: Benzyloxycarbonyl or Cbz), ethylcarbamate ($PG_2$: ethoxycarbonyl) or methylcarbamate ($PG_2$: methoxycarbonyl), preferably $PG_2$ is Boc..

The compounds of formula (I), here reported again for clarity, including all the compounds here above listed, can be usually prepared according to the procedures shown in the schemes below. Where a specific detail or step differs from the general schemes it has been detailed in the specific examples, and/or in additional schemes.

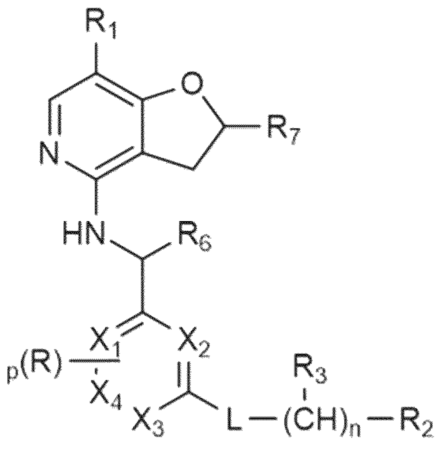

I

Compounds of formula (I) can contain one or more stereogenic centre. Enantiomerically pure compounds can be prepared according to generally known reactions, e.g. according to the reactions described below, by means of enantiomerically pure starting materials and intermediates. These intermediates may be commercially available or readily produced from commercial sources by those of ordinary skill in the art.

In another approach, enantiomerically pure compounds can be prepared from the corresponding racemates by means of chiral chromatography purification. Stereochemically pure compounds may be obtained by chiral separation

from a stereoisomers mixture, or (whenever there are two or more stereogenic centres -i.e. chiral center- in compounds of formula (I)) stepwise by chromatographic separation of diastereoisomers followed by further chiral separation into single stereoisomers.

SCHEME 1

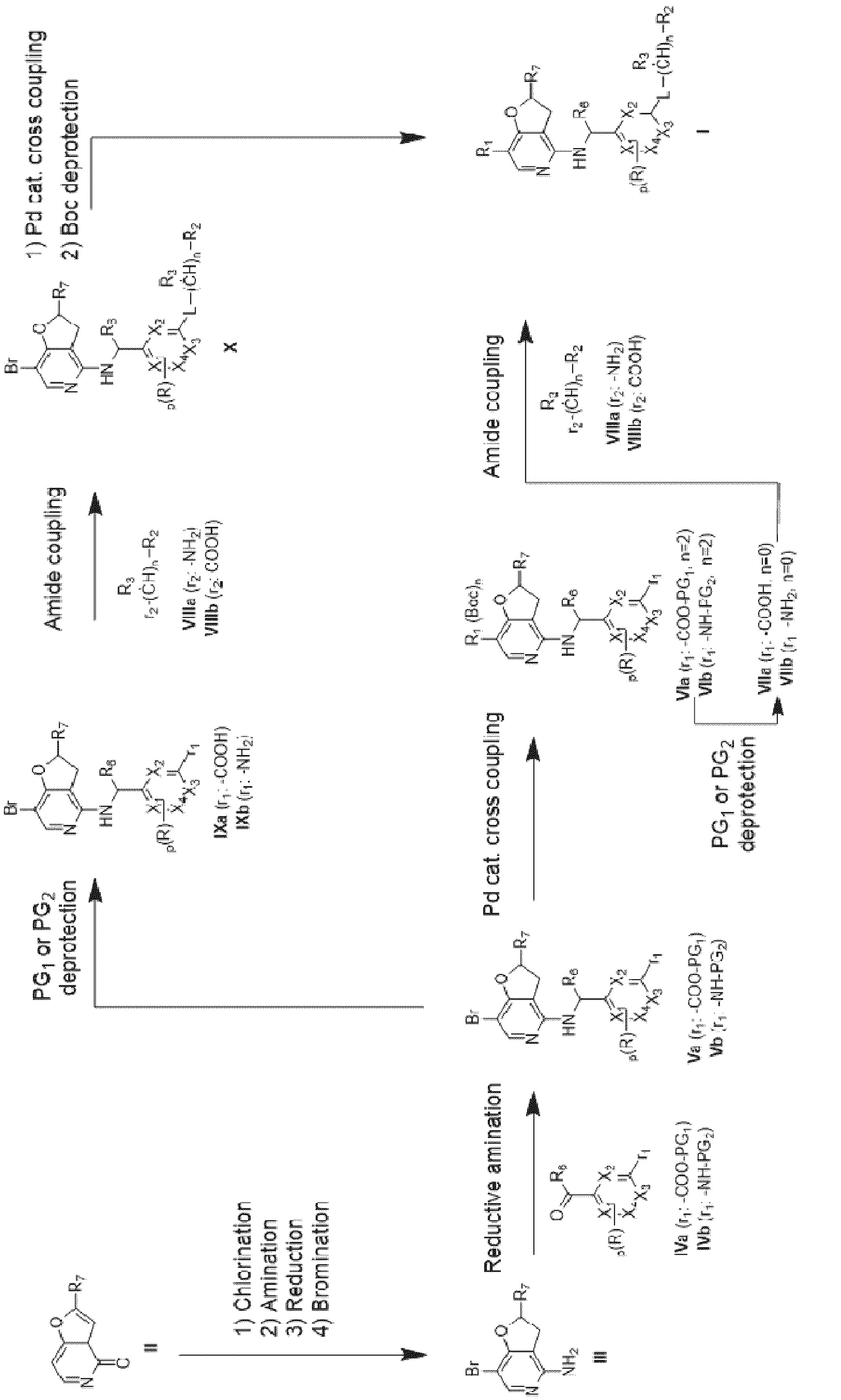


Compounds of formula (I) can be prepared according to scheme 1 starting from comercially available intermediate **II** or easily obtainable by those skilled in art.

Intermediate **II** can be converted into intermediate **III** by means of four consecutive steps including 1) chlorination, 2) amination, 3) reduction and 4) bromination.

For example, the chlorination step may be carried out by refluxing intermediate **II** with an appropriate chlorinating agent (neat or in solution with an organic solvent such as DCM or dioxane) such as $POCl_3$ or $SOCl_2$.

The amination step can be carried out by introducing a masked ammonia such as benzophenone imine through a Buchwald type palladium catalyzed reaction using, for example, tris(dibenzylideneacetone)dipalladium(0)/BINAP catalytic system followed by hydrolysis of the benzophenone imine by using hydroxylamine to give the corresponding furo [3,2-c]pyridin-4-amine. Alternatively, the amination step can be carried out by introducing 4-methoxybenzylamine by means of $S_NAr$ reaction (nucleophilic aromatic substitution) followed by deprotection with a strong acid such as trifluoroacetic acid or methansulphonic acid. Reduction of furo[3,2-c]pyridin-4-amine to give 2,3-dihydrofuro[3,2-c] pyridin-4-amine (step 3) can be carried out, for example, by hydrogenation of a solution of furo[3,2-c]pyridin-4-amine in methanol / acetic acid in the presence of a Pd/C catalyst under high $H_2$ pressure (e.g. 10 bar) and at a temperature of 50°C or higher. Finally, intermediate **III** can be obtained by means of bromination of 2,3-dihydrofuro[3,2-c]pyridin-4-amine (step 4) by reaction with a brominating agent such as N-bromosuccinimide in a polar aprotic solvent such as acetonitrile or tetrahydrofuran for a few hours at low temperature (e.g. -10 - 0 °C).

Intermediate **III** and carbonyl intermediate **IVa** (or **IVb**) can be combined to give intermediate **Va** (or **Vb**) through a reductive amination reaction that can be performed in an appropriate solvent such as DCM or THF, in the presence of a Lewis acid such as chloro(triisopropoxy)titanium(IV) or titanium tetraisopropoxide(IV) followed by addition of a reducing agent such as sodium triacetoxyborohydride or sodium cyanoborohydride, in the presence of an organic acid such as acetic acid or trifluoroacetic acid.

Intermediate **Va** (or **Vb**) can be converted into intermediate **VIa** (or **VIb**) by a direct introduction of group $R_1$ through a metal/palladium catalyzed cross coupling reaction such as Stille coupling, Suzuki coupling or similar (Strategic application of named reactions in organic synthesis, L. Kurti, B. Czako, Ed. 2005). For example a suitable palladium catalyzed cross coupling for introducing $R_1$ when it is an 2-aminopyrimidin-4-yl, is a Stille coupling. A Stille coupling can be performed by reacting intermediate **Va** (or **Vb**) with the corresponding organostannane of group $R_1$, in the presence of a Pd catalyst such as tetrakistriphenylphosphinepalladium(0), tris(dibenzylideneacetone)dipalladium(0), or $PdCl_2(dppf)_2$, in an organic solvent such as dioxane or THF or DMF, in the presence of a copper(I) salt such as copper(I) thiophene-2-carboxylate, under heating (90-150°C). Organostannanes are generally commercially available or may be readily prepared by those skilled in the art starting from corresponding commercially available halides. Experimental procedures for the preparation of organostannane not commercially available are reported in the experimental section. When $R_1$ is a 2-aminopyrimidin-4-yl, for synthetic convenience, the amino group needs to be masked/protected during the Stille coupling. Said amino group may be suitably protected by one or even two Boc groups and removed when convenient trougthout the synthetic sequence.

Removal of $PG_1$ (when $PG_1$ is methyl or isopropyl) from intermediate **VIa** to give the intermediate **VIIa** may be carried out by hydrolysis, using an inorganic base such as LiOH or NaOH in a mixture of an organic solvent such as THF and/or methanol with water, generally at RT and for a time ranging from 1 h to overnight. In the above mentioned reaction condition, whether $R_1$ is a N-bis-Boc protected 2-aminopyrimidin-4-yl, one Boc group could undergo cleavage; then complete Boc removal could be performed by treatment with a strong acid such as trifluoroacetic acid or concentrated hydrochloric acid.

Removal of $PG_2$ (when $PG_2$ is Boc) from intermediate **VIb** to give the intermediate **VIIb** may be carried out by acidic deprotection. For example, an acidic Boc cleavage may be carried out by means of concentrated hydrochloric acid or trifluoroacetic acid. With these conditions Boc groups on bis-Boc protected 2-aminopyrimidin-4-yl can also be cleaved.

Reaction between acid intermediate **VIIa** and amino intermediate **VIIIa** (or acid **VIIIb** and amine **VIIb**) to give a compound of formula (I) may be carried out under suitable amide coupling reaction conditions. For example, acid intermediate **VIIa** may be reacted in the presence of an activating agent such as TBTU, HATU or COMU, with an organic base such as DIPEA or TEA, in a suitable organic solvent such as DCM or DMF, and at temperature generally around RT for a time ranging from a few hours to overnight. An alternative condition for amide coupling may be carried out by reacting intermediate **VIIa** and **VIIIa** in the presence of 1-(methylsulfonyl)-1H-benzotriazole as a coupling agent, with an organic base such as TEA, at a temperature up to 150°C for a few hours (for example 4 h).

Wherein a compound of formula (I) contains in $R_2$ or $R_3$ a primary or secondary amine, this amino moiety needs to be masked during the amide coupling step by using suitably protected (generally Boc) intermediates **VIIIa** or **VIIIb**. The Boc protecting group can be removed by using similar methods to those described above for intermediates **VIb** after amide coupling.

In some cases, wherein a compound of formula (I) contains in $R_2$ or $R_3$ a tertiary amine or a tertiary amide, such compounds can be obtained by further elaboration of a compound of formula (I) (wherein $R_2$ or $R_3$ contain a secondary amine) by a reductive amination reaction or an amidation of the corresponding secondary amine using generally known methods.

Compounds of formula (I) can be obtained from intermediate **X** by a direct introduction of group $R_1$ in the same

way (scheme 1) as that described for transformation of intermediate **Va** into **VIa** (or **Vb** into **VIb**). When $R_1$ is an 2-aminopyrimidin-4-yl, for synthetic convenience the amino group needs to be protected during the Stille coupling. Said amino group may be suitably protected by one or even two Boc groups and removed by acidic cleavage as already described for intermediates **VIIa** or **VIIb.**

Intermediate **X** can be obtained by amide coupling of acid intermediate **IXa** and amino intermediate **VIIIa** (or acid **VIIIb** and amine **IXb**) using similar conditions to that described above for the reaction of **VIIa** and intermediate **VIIIa** (or **VIIIb** and **VIIb**). Intermediates **IXa** and **IXb** can be obtained from **Va** and **Vb** respectively by deprotection of $PG_1$ and $PG_2$ according to conditions already reported above for intermediate **VIa** and **VIb**.

The invention is also directed to a compound of formula **Va** or **Vb,** and to its use as intermediate in the preparation of compounds of formula (I) wherein $PG_1$ and $PG_2$ are suitable protective groups and all the other variables are defined as for compounds of formula (I) above. Particularly for intermediate compound **Va,** carboxylic acid protection via ester formation, $PG_1$ is a $(C_1-C_4)$ alkyl group, preferably selected from methyl, isopropyl, tert-butyl or ethyl, even more preferably $PG_1$ is methyl. A suitable protective group for intermediate compounds **Vb,** amino protection via carbamate formation, $PG_2$ is preferably selected from Boc (tert-butoxycarbonyl), Cbz (benzyloxycarbonyl), ethyloxycarbonyl or methoxycarbonyl.

The invention is also directed to the use of compounds **Va** or **Vb** as an intermediate in the preparation of compounds of formula (I) according to the process as described above.

As herein described in details, the compounds of the invention are inhibitors of kinase activity, in particular Rho-kinase activity.

In one aspect the invention provides a compound of formula (I) for use as a medicament, preferably for the prevention and /or treatment of pulmonary disease.

In a further aspect the invention provides the use of a compound (I), or a pharmaceutically acceptable salt thereof, in the manufacture of a medicament for the treatment of disorders associated with ROCK enzyme mechanisms, including immune system disorders and particularly for the treatment of disorders such as pulmonary diseases.

In particular the invention provides compounds of formula (I) for use in the prevention and /or treatment of immune system disorders including Graft-versus-host disease (GVHD), and for pulmonary disease selected from the group consisting of asthma, chronic obstructive pulmonary disease COPD, idiopathic pulmonary fibrosis (IPF), pulmonary hypertension (PH) and specifically Pulmonary Arterial Hypertension (PAH).

Moreover the invention provides a compound of formula (I) for use in a method for the prevention and/or treatment of disorders associated with ROCK enzymes mechanisms, said method comprising administering to a patient in need of such treatment a therapeutically effective amount of a compound of the invention.

In particular the invention provides a compound of formula (I) for use in methods for the prevention and/or treatment wherein the disorder is an immune system disorder such as Graft-versus-host disease (GVHD), and/or a respiratory disease selected from asthma, chronic obstructive pulmonary disease (COPD), idiopathic pulmonary fibrosis (IPF), Pulmonary hypertension (PH) and specifically Pulmonary Arterial Hypertension (PAH).

Preferred is the use of the compounds of the invention for the prevention of the aforesaid disorders.

Equally preferred is the use of the compounds of the invention for the treatment of the aforesaid disorders.

Generally speaking, compounds which are ROCK inhibitors may be useful in the treatment of many disorders associated with ROCK enzyme mechanisms.

In one embodiment, the disorders that can be treated by the compounds of the present invention include glaucoma, inflammatory bowel disease (IBD), immune system disorders including Graft-versus-host disease (GVHD), and pulmonary diseases selected from asthma, chronic obstructive pulmonary disease (COPD), interstitial lung disease such as idiopathic pulmonary fibrosis (IPF) and pulmonary arterial hypertension (PAH).

In another embodiment, the disorder that can be treated by the compound of the present invention is selected from the group consisting of asthma, chronic obstructive pulmonary disease (COPD) and interstitial lung disease such as idiopathic pulmonary fibrosis (IPF) and pulmonary arterial hypertension (PAH).

In a further embodiment, the disorder is selected from idiopathic pulmonary fibrosis (IPF) and pulmonary arterial hypertension (PAH).

The methods of treatment referred to in the description comprise administering a safe and effective amount of a compound of formula (I) or a pharmaceutically acceptable salt thereof to a patient in need thereof. As used herein, "safe and effective amount" in reference to a compound of formula (I) or a pharmaceutically acceptable salt thereof or other pharmaceutically-active agent means an amount of the compound sufficient to treat the patient's condition but low enough to avoid serious side effects and it can nevertheless be routinely determined by the skilled artisan. The compounds of formula (I) or pharmaceutically acceptable salts thereof may be administered once or according to a dosing regimen wherein a number of doses are administered at varying intervals of time for a given period of time. Typical daily dosages may vary depending upon the particular route of administration chosen.

The invention also provides pharmaceutical compositions of compounds of formula (I) in admixture with one or more pharmaceutically acceptable carrier or excipient, for example those described in Remington's Pharmaceutical Sciences Handbook, XVII Ed., Mack Pub., N.Y., U.S.A.

The present invention is also directed to use of the compounds of the invention and their pharmaceutical compositions for various route of administration

Administration of the compounds of the invention and their pharmaceutical compositions may be accomplished according to patient needs, for example, orally, nasally, parenterally (subcutaneously, intravenously, intramuscularly, intrasternally and by infusion), by inhalation, rectally, vaginally, topically, locally, transdermally, and by ocular administration.

Various solid oral dosage forms can be used for administering compounds of the invention including such solid forms as tablets, gelcaps, capsules, caplets, granules, lozenges and bulk powders. The compounds of the present invention can be administered alone or combined with various pharmaceutically acceptable carriers, diluents (such as sucrose, mannitol, lactose, starches) and known excipients, including suspending agents, solubilizers, buffering agents, binders, disintegrants, preservatives, colorants, flavorants, lubricants and the like. Time release capsules, tablets and gels are also advantageous.

Various liquid oral dosage forms can also be used for administering compounds of the invention, including aqueous and non-aqueous solutions, emulsions, suspensions, syrups, and elixirs. Such dosage forms can also contain suitable known inert diluents such as water and suitable known excipients such as preservatives, wetting agents, sweeteners, flavorants, as well as agents for emulsifying and/or suspending the compounds of the invention. The compounds of the present invention may be formulated as injectable composition, for example to be injected intravenously, in the form of an isotonic sterile solution. Other preparations are also possible.

Suppositories for rectal administration of the compounds of the invention can be prepared by mixing the compound with a suitable excipient such as cocoa butter, salicylates and polyethylene glycols.

Formulations for vaginal administration can be in the form of cream, gel, paste, foam, or spray formula containing, in addition to the active ingredient, such as suitable carriers, are also known.

For topical administration the pharmaceutical composition can be in the form of creams, ointments, liniments, lotions, emulsions, suspensions, gels, solutions, pastes, powders, sprays, and drops suitable for administration to the skin, eye, ear or nose. Topical administration may also involve transdermal administration via means such as transdermal patches.

Some preferred compounds of the invention exhibit profile suitable for inhalatory route administration.

Drugs optimized for inhaled delivery require certain characteristics that allow the compound, when administered to the lung to maintain a sufficient local concentration (lung retention) to exert a pharmacological effect of the desired duration, and non-relevant levels in unwanted compartments (i.e. plasma). To attenuate lung absorpion, one or more features of a compounds need to be optimized such as, and not limited to, membrane permeability, dissolution rate and the degree of basicity. In this respect, to attain lung retention, permeability is low, dissolution rate is sufficiently slow, and a basic group is present to enhance binding to the phospholipid-rich lung tissue or toallow lysosomial trapping. In some embodiments, compounds of the invention show one or more of the features above that are desirable for an inhaled compound.

Other preferred compounds of the invention exhibit a profile suitable for the oral route of administration. Drugs optimized for oral delivery require certain characteristics that allow the orally administered compound to be absorbed by the GI (gastrointestinal) tract and to be poorly cleared in order to give a good bioavailability (F%), thus to maintain a sufficient concentration in plasma and target tissues for a time adequate to sustain pharmacological effect. To enhance oral bioavalability, one or more features of the compounds need to be optimized such as, and not limited to, membrane permeabilty and in vivo clearance. In this respect, to attain high oral bioavailability membrane permeability is high and compounds have reduced metabolic hot spots to (optimized in-vitro clearance). In some embodiments, compounds of the invention show one or more of the features above for an oral compound.

For the treatment of the diseases of the respiratory tract, the compounds according to the invention, as above said, may be administered by inhalation.

Inhalable preparations include inhalable powders, propellant-containing metering aerosols or propellant-free inhalable formulations.

For administration as a dry powder, single- or multi-dose inhalers known from the prior art may be utilized. In that case the powder may be filled in gelatine, plastic or other capsules, cartridges or blister packs or in a reservoir.

A diluent or carrier, usually non-toxic and chemically inert to the compounds of the invention, e.g. lactose or any other additive suitable for improving the respirable fraction may be added to the powdered compounds of the invention.

Inhalation aerosols containing propellant gas such as hydrofluoroalkanes may contain the compounds of the invention either in solution or in dispersed form. The propellant-driven formulations may also contain other ingredients such as co-solvents, stabilizers and optionally other excipients.

The propellant-free inhalable formulations comprising the compounds of the invention may be in the form of solutions or suspensions in an aqueous, alcoholic or hydroalcoholic medium and they may be delivered by jet or ultrasonic nebulizers known from the prior art or by soft-mist nebulizers such as Respimat®.

Further preferably the invention provides compounds of formula (I) and/or pharmaceutical compositions thereof,

for use via inhalatory route of administration particularly in the prevention and /or treatment of asthma, chronic obstructive pulmonary disease COPD, idiopathic pulmonary fibrosis (IPF), pulmonary hypertension (PH) and specifically Pulmonary Arterial Hypertension (PAH), preferably in the prevention and /or treatment of asthma, chronic obstructive pulmonary disease COPD, idiopathic pulmonary fibrosis (IPF).

Further preferably the invention provides compounds of formula (I) and/or pharmaceutical compositions thereof, for use via oral route of administration particularly in the prevention and /or treatment of asthma, chronic obstructive pulmonary disease COPD, idiopathic pulmonary fibrosis (IPF), pulmonary hypertension (PH) and specifically Pulmonary Arterial Hypertension (PAH), preferably in the prevention and /or treatment of pulmonary hypertension (PH) and specifically Pulmonary Arterial Hypertension (PAH).The compounds of the invention, regardless of the route of administration, can be administered as the sole active agent or in combination (i.e. as co-therapeutic agents administered in fixed dose combination or in combined therapy of separately formulated active ingredients) with other pharmaceutical active ingredients selected from organic nitrates and NO donors; inhaled NO; stimulator of soluble guanylate cyclase (sGC); prostaciclin analogue PGI2 and agonist of prostacyclin receptors; compounds that inhibit the degradation of cyclic guanosine monophosphate (cGMP) and/or cyclic adenosine monophosphate (cAMP), such as inhibitors of phosphodiesterases (PDE) 1, 2, 3, 4 and/or 5, especially PDE 5 inhibitors; human neutrophilic elastase inhibitors; compounds inhibiting the signal transduction cascade, such as tyrosine kinase and/or serine/threonine kinase inhibitors; antithrombotic agents, for example platelet aggregation inhibitors, anticoagulants or profibrinolytic substances; active substances for lowering blood pressure, for example calcium antagonists, angiotensin II antagonists, ACE inhibitors, endothelin antagonists, renin inhibitors, aldosterone synthase inhibitors, alpha receptor blockers, beta receptor blockers, mineralocorticoid receptor antagonists; neutral endopeptidase inhibitor; osmotic agents; ENaC blockers; anti-inflammatories including corticosteroids and antagonists of chemokine receptors; antihistamine drugs; anti-tussive drugs; antibiotics such as macrolide and DNase drug substance and selective cleavage agents such as recombinant human deoxyribonuclease I (rhDNase); agents that inhibit ALK5 and/or ALK4 phosphorylation of Smad2 and Smad3; tryptophan hydroylase 1 (TPH1) inhibitors and multi-kinase inhibitors, beta2-agonists, corticosteroids, anticholinergic or antimuscarinic agents, mitogen-activated protein kinases (P38 MAP kinase) inhibitors, nuclear factor kappa-B kinase subunit beta (IKK2) inhibitors, leukotriene modulators, non-steroidal anti-inflammatory agents (NSAIDs), mucus regulators, mucolytics, expectorant/mucokinetic modulators, peptide mucolytics, inhibitors of JAK, SYK inhibitors, inhibitors of PI3Kdelta or PI3Kgamma and combinations thereof.

In a preferred embodiment, the compounds of the invention are dosed in combination with phosphodiesterase V such as sildenafil, vardenafil and tadalafil; organic nitrates and NO donors (for example sodium nitroprusside, nitroglycerin, isosorbide mononitrate, isosorbide dinitrate, molsidomine or SIN-1 , and inhaled NO); synthetic prostacyclin analogue PGI2 such as iloprost, treprostinil, epoprostenol and beraprost; agonist of prostacyclin receptors such as selexipag and compounds of WO 2012/007539; stimulators of soluble guanylate cyclase (sGC) like riociguat and tyrosine kinase like imatinib, sorafenib and nilotinib and endothelin antagonist (for example macitentan, bosentan, sitaxentan and ambrisentan).

In a further embodiment the compounds of the invention are dosed in combination with beta2-agonists such as salbutamol, salmeterol, and vilanterol, corticosteroids such as fluticasone propionate or furoate, flunisolide, mometasone furoate, rofleponide and ciclesonide, dexametasone, anticholinergic or antimuscarinic agents such as ipratropium bromide, oxytropium bromide, tiotropium bromide, oxybutynin, and combinations thereof.

In a further embodiment the compounds of the invention are dosed in combination with mitogen-activated protein kinases (P38 MAP kinase) inhibitors, nuclear factor kappa-B kinase subunit beta (IKK2) inhibitors, leukotriene modulators, non-steroidal anti-inflammatory agents (NSAIDs), mucus regulators, mucolytics, expectorant/mucokinetic modulators, peptide mucolytics inhibitors of JAK, SYK inhibitors, inhibitors of PI3Kdelta or PI3Kgamma.

The invention is also directed to a kit comprising the pharmaceutical compositions of compounds of the invention alone or in combination with or in admixture with one or more pharmaceutically acceptable carriers and/or excipients and a device which may be a single- or multi-dose dry powder inhaler, a metered dose inhaler or a nebulizer.

The dosages of the compounds of the invention depend upon a variety of factors including the particular disease to be treated, the severity of the symptoms, the route of administration, the frequency of the dosage interval, the particular compound utilized, the efficacy, toxicology profile, and pharmacokinetic profile of the compound.

Advantageously, the compounds of formula (I) can be administered for example, at a dosage comprised between 0.001 and 10000 mg/day, preferably between 0.1 and 500 mg/day.

When the compounds of formula (I) are administered by inhalation route, they are preferably given at a dosage comprised between 0.001 and 500 mg/day, preferably between 0.1 and 100 mg/day.

A pharmaceutical composition comprising a compound of the invention suitable to be administered by inhalation is in various respirable forms, such as inhalable powders (DPI), propellant-containing metering aerosols (PMDI) or propellant-free inhalable formulations (e.g. UDV).

The invention is also directed to a device comprising the pharmaceutical composition comprising a compound according to the invention, which may be a single- or multi-dose dry powder inhaler, a metered dose inhaler and a nebulizer

particularly soft mist nebulizer.

Although for the treatment of the diseases of the respiratory tract, the compounds according to the invention can be administered by inhalation; they may be in some case preferably be administered by the oral route.

When the compounds of formula (I) are administered by oral route, they are preferably given at a dosage comprised from 0.001 mg to 100 mg per kg body weight of a human, often 0.01 mg to about 50 mg per kg, for example 0.1 to 10 mg per kg, in single or multiple doses per day.

A pharmaceutical composition comprising a compound of the invention suitable to be administered by the oral route can be in various solid or liquid forms, such as tablets, gelcaps, capsules, caplets, granules, lozenges and bulk powders or aqueous and non-aqueous solutions, emulsions, suspensions, syrups, and elixirs formulations.

The following examples illustrate the invention in more detail.

## PREPARATION OF INTERMEDIATES AND EXAMPLES

### General Experimental details

Chemical Names of the compounds were generated with Structure To Name Enterprise 10.0 Cambridge Software or latest.

Purification by 'chromatography' or 'flash chromatography' refers to purification using the Biotage SPI purification system or equivalent MPLC system using a pre-packed polypropylene column containing unbounded activated silica with irregular particles with average size of 50 μm and nominal 60Å porosity. When 'NH-silica' and 'C18-silica' are specified, they refer respectively to aminopropyl chain bonded silica and octadecyl carbon chain (C18)-bonded silica. Fractions containing the required product (identified by TLC and/or LCMS analysis) were pooled and concentrated *in vacuo* or freeze-dried.

Where an Isolute® SCX-2 cartridge was used, 'Isolute® SCX-2 cartridge' refers to a pre-packed polypropylene column containing a non-end-capped propylsulphonic acid functionalised silica strong cation exchange sorbent.

### LCMS Methods

#### Method 1

| | | | |
|---|---|---|---|
| Instrumentation | Acquity H-Class (quaternary pump/PDA detector) + QDa Mass Spectrometer | | |
| Column | Acquity UPLC CSH C18 1.7μm, 50 × 2.1mm at 40°C | | |
| Mobile Phase A | 0.1% Formic acid (v/v) in water | | |
| Mobile Phase B | 0.1% Formic acid in acetonitrile (v/v) | | |
| Flow | 1.0 mL/min | | |
| Gradient Program | Time (mins) | % A | %B |
| | 0.0 | 97 | 03 |
| | 1.5 | 01 | 99 |
| | 1.9 | 01 | 99 |
| | 2.0 | 97 | 03 |
| | 2.5 | 97 | 03 |
| Detectors | UV, diode array 190-400nm | | |
| | MS ionisation method - Electrospray (positive/negative ion) | | |

#### Method 2

| | |
|---|---|
| Instrumentation | Acquity H-Class (quaternary pump/PDA detector) + QDa Mass Spectrometer |

(continued)

| | | | |
|---|---|---|---|
| Column | Acquity BEH C18 1.7μm, 50 × 2.1mm at 40°C | | |
| Mobile Phase C | 0.03% Aqueous ammonia (v/v) | | |
| Mobile Phase D | 0.03% Ammonia in Acetonitrile (v/v) | | |
| Flow | 0.8 mL/min | | |
| Gradient Program | Time (mins) | % A | %B |
| | 0.0 | 97 | 03 |
| | 1.5 | 03 | 97 |
| | 1.9 | 03 | 97 |
| | 2.0 | 97 | 03 |
| | 2.5 | 97 | 03 |
| Detectors | UV, diode array 190-400nm | | |
| | MS ionisation method - Electrospray (positive/negative ion) | | |

Method 3

| | | | |
|---|---|---|---|
| Instrumentation | Acquity H-Class (quaternary pump/PDA detector) + QDa Mass Spectrometer | | |
| Column | Acquity BEH C18 1.7μm, 50 × 2.1mm at 40°C | | |
| Mobile Phase C | 0.03% Aqueous ammonia (v/v) (7.66mM) | | |
| Mobile Phase D | 0.03% Ammonia in Acetonitrile (v/v) (7.66mM) | | |
| Flow | 0.8 mL/min | | |
| Gradient Program | Time (mins) | % A | %B |
| | 0.0 | 97 | 03 |
| | 4.0 | 03 | 97 |
| | 4.4 | 03 | 97 |
| | 4.5 | 97 | 03 |
| | 5.0 | 97 | 03 |
| Detectors | UV, diode array 190-400nm | | |
| | MS ionisation method - Electrospray (positive/negative ion) | | |

Method 4

| | |
|---|---|
| Instrumentation | UPLC + Waters DAD + Waters SQD2, single quadrupole UPLC-MS |
| Column | Acquity UPLC HSS C18 1.8μm 100 x 2.1mm. (Plus guard cartridge), maintained at 40°C |
| Mobile Phase A | 0.1% Formic acid (v/v) in water |
| Mobile Phase B | 0.1% Formic acid (v/v) in acetonitrile |
| Flow | 0.4 mL/min |

(continued)

| | | | |
|---|---|---|---|
| Gradient Program | Time (mins) | % A | %B |
| | 0.0 | 95 | 05 |
| | 0.4 | 95 | 05 |
| | 6.0 | 05 | 95 |
| | 6.8 | 05 | 95 |
| | 7.0 | 95 | 05 |
| | 8.0 | 95 | 05 |
| Detectors | UV, diode array 210nm-400nm | | |
| | MS ionisation method - Electrospray (positive/negative ion) | | |

Method 5

| | | | |
|---|---|---|---|
| Instrumentation | UPLC + Waters DAD + Waters SQD2, single quadrupole UPLC-MS | | |
| Column | Acquity UPLC BEH Shield RP18 1.7μm 100 x 2.1mm. (Plus guard cartridge), maintained at 40°C | | |
| Mobile Phase A | Aqueous ammonium hydrogen carbonate 10mM | | |
| Mobile Phase B | Acetonitrile | | |
| Flow | 0.4 mL/min | | |
| Gradient Program | Time (mins) | % A | %B |
| | 0.0 | 95 | 05 |
| | 0.4 | 95 | 05 |
| | 6.0 | 05 | 95 |
| | 6.8 | 05 | 95 |
| | 7.0 | 95 | 05 |
| | 8.0 | 95 | 05 |
| Detectors | UV, diode array 210nm-400nm | | |
| | MS ionisation method - Electrospray (positive/negative ion) | | |

Method 6

| | |
|---|---|
| Instrumentation | Acquity i-Class (quarternary pump/PDA detector) + Quattro Micro Mass Spectrometer |
| Column | Acquity UPLC BEH $C_{18}$ 1.7μm, 100 × 2.1mm, maintained at 40°C |
| Mobile Phase A | 0.1% Formic acid (v/v) in water |
| Mobile Phase B | 0.1% Formic acid in acetonitrile (v/v) |
| Flow | 0.4 mL/min |

(continued)

| | | | |
|---|---|---|---|
| Gradient Program | Time (mins) | % A | %B |
| | 0.0 | 95 | 05 |
| | 0.4 | 95 | 05 |
| | 6.0 | 05 | 95 |
| | 6.8 | 05 | 95 |
| | 7.0 | 95 | 05 |
| | 8.0 | 95 | 05 |
| Detectors | UV, diode array 200-500nm | | |
| | MS ionisation method - Electrospray (positive/negative ion) | | |

Method 7

| | | | |
|---|---|---|---|
| Instrumentation | Acquity UPLC (binary pump/PDA detector) + ZQ Mass Spectrometer | | |
| Column | Acquity UPLC BEH $C_{18}$ 1.7μm, 100 × 2.1mm, maintained at 40°C | | |
| Mobile Phase A | 0.1% Aqueous ammonia (v/v) | | |
| Mobile Phase B | 0.1% Ammonia in acetonitrile (v/v) | | |
| Flow | 0.4 mL/min | | |
| Gradient Program | Time (mins) | % A | %B |
| | 0.0 | 95 | 05 |
| | 0.4 | 95 | 05 |
| | 6.0 | 05 | 95 |
| | 6.8 | 05 | 95 |
| | 7.0 | 95 | 05 |
| | 8.0 | 95 | 05 |
| Detectors | UV, diode array 200-500nm | | |
| | MS ionisation method - Electrospray (positive/negative ion) | | |

Method 8

| | |
|---|---|
| Instrumentation | HP1100 (quaternary pump/PDA detector) + ZQ Mass Spectrometer |
| Column | Xbridge BEH $C_{18}$ 3.5μm, 4.6x50mm 40°C |
| Mobile Phase A | 0.03% Aqueous ammonia (v/v) |
| Mobile Phase B | 0.03% Ammonia in acetonitrile (v/v) |
| Flow | 2.0 mL/min |

(continued)

| Gradient Program | Time (mins) | % A | %B |
|---|---|---|---|
| | 0.0 | 95 | 05 |
| | 0.3 | 95 | 05 |
| | 4.3 | 05 | 95 |
| | 5.3 | 05 | 95 |
| | 5.8 | 95 | 05 |
| | 6.0 | 95 | 05 |
| Detectors | UV, diode array 190-450nm | | |
| | MS ionisation method - Electrospray (positive/negative ion) | | |

Method 9

| | | | |
|---|---|---|---|
| Instrumentation | Acquity UPLC (binary pump/PDA detector) + QDa Mass Spectrometer | | |
| Column | CSH $C_{18}$ 1.7μm, 50 × 2.1mm, at 40°C | | |
| Mobile Phase A | 0.05% Formic acid (v/v) in 95/5 water/acetonitrile | | |
| Mobile Phase B | 0.05% Formic acid (v/v) in 5/95 water/acetonitrile | | |
| Flow | 1.0 mL/min | | |
| Gradient Program | Time (mins) | % A | %B |
| | 0.0 | 95 | 05 |
| | 1.50 | 05 | 95 |
| | 1.90 | 05 | 95 |
| | 2.0 | 05 | 95 |
| | 2.3 | 05 | 95 |
| Detectors | UV, diode array 200-500nm | | |
| | MS ionisation method - Electrospray (positive/negative ion) | | |

Method 10 and Method 11

| | |
|---|---|
| Instrumentation | Shimadzu LCMS-2020 Single Quadrupole Liquid Chromatograph Mass Spectrometer |
| Column | Aquity HSS $C_{18}$ 1.8μm, 50 × 2.1mm, at 25°C |
| Mobile Phase A | 0.1% Formic acid (v/v) in water |
| Mobile Phase B | 0.1% Formic acid (v/v) in acetonitrile |
| Flow | 0.5 mL/min |

(continued)

| | | | |
|---|---|---|---|
| Gradient Program | Time (mins) | % A | %B |
| | 0.00 | 95 | 05 |
| | 4.00 | 05 | 95 |
| | 5.00 | 05 | 95 |
| | 5.20 | 95 | 05 |
| | 6.00 | 95 | 05 |
| Detectors | UV, 254 nm and 214 nm (method 10)<br>UV, 254 nm and 220 nm (method 11) | | |
| | MS ionisation method - Electrospray (positive/negative ion) | | |

Method 12

| | | | |
|---|---|---|---|
| Instrumentation | Shimadzu LCMS-2020 Single Quadrupole Liquid Chromatograph Mass Spectrometer | | |
| Column | Aquity HSS $C_{18}$ 1.8μm, 50 × 2.1mm, at 25°C | | |
| Mobile Phase A | 0.1% Formic acid (v/v) in water | | |
| Mobile Phase B | 0.1% Formic acid (v/v) in acetonitrile | | |
| Flow | 0.5 mL/min | | |
| Gradient Program | Time (mins) | % A | %B |
| | 0.00 | 95 | 05 |
| | 10.00 | 05 | 95 |
| | 10.50 | 05 | 95 |
| | 11.00 | 95 | 05 |
| | 12.00 | 95 | 05 |
| Detectors | UV, 254 nm and 214 nm | | |
| | MS ionisation method - Electrospray (positive/negative ion) | | |

Method 13

| | |
|---|---|
| Instrumentation | Agilent Technologies 1260 Infinity II with DAD detector / Agilent Technologies InfinityLab LC/MSD |
| Column | BEH $C_{18}$ 1.7μm, 50 × 2.1mm, at 25°C |
| Mobile Phase A | 0.05% Aqueous ammonia (v/v) |
| Mobile Phase B | Acetonitrile |
| Flow | 0.5 mL/min |

(continued)

| Gradient Program | Time (mins) | % A | %B |
|---|---|---|---|
| | 0.00 | 80 | 20 |
| | 5.00 | 70 | 30 |
| | 5.60 | 70 | 30 |
| | 5.90 | 05 | 95 |
| | 7.10 | 05 | 95 |
| | 7.50 | 80 | 20 |
| | 9.00 | 80 | 20 |
| Detectors | UV, Diode array 190 - 400 nm | | |
| | MS ionisation method - Electrospray (positive/negative ion) | | |

NMR Methods

NMR spectra were obtained on a Bruker Avance 400 MHz, 5mm QNP probe H, C, F, P, single Z gradient, two channel instrument running TopSpin 2.1, or on a Bruker Avance III 400 MHz, 5mm BBFO Plus probe, single Z gradient, two channel instrument running TopSpin 3.0, or on a Varian Unity Inova 400 spectrometer with a 5 mm inverse detection triple resonance probe operating at 400 MHz. Chemical shift are reported as $\delta$ values in ppm relative to tetramethylsilane. Coupling constants (J values) are given in hertz (Hz) and multiplicities are reported using the following abbreviation: s=singlet, d=doublet, t=triplet, q=quartet, m=multiplet, br=broad, nd=not determined.

SFC Methods

Where compounds were purified using Supercritical Fluid Chromatography (SFC) either a Waters Thar Prep100 preparative SFC system (P200 $CO_2$ pump, 2545 modifier pump, 2998 UV/VIS detector, 2767 liquid handler with Stacked Injection Module) or a Waters Thar Investigator semi preparative system (Waters Fluid Delivery Module, 2998 UV/VIS detector, Waters Fraction Collection Module) was used. The compounds were purified using the column and conditions specified and fractions that contained the desired product were concentrated by vacuum centrifugation.

The modifier used under basic conditions was diethyl amine (0.1% V/V). Alternate modifiers such as formic acid (0.1% V/V), acetic acid (0.1% V/V), were used as an acidic modifier.

MDAP Methods

Compounds were purified by reverse phase HPLC using a Waters Fractionlynx preparative HPLC system (2525 pump, 2996/2998 UV/VIS detector, 2767 liquid handler) or Gilson preparative HPLC system (322 pump, 155 UV/VIS detector, GX-281 liquid handler) or equivalent system. Collection was triggered by a threshold absorbance value at 260 nm and the presence of target molecular ion as observed under ESI conditions. The fractions that contained the desired product were lyophilized. The specific details of the conditions used, including the column, solvents, gradient and modifier (acidic or basic), are provided for some examples and merely provided for assistance. When specific conditions are not provided, they can be readily optimized by those skilled in the art.

In the procedures that follow, some of the starting materials are identified through an "Intermediate" or "Example" number with indications on step name. When reference is made to the use of a "similar" or "analogous" procedure, as will be appreciated by those skilled in the art, such a procedure may involve minor variations, for example reaction temperature, reagent/solvent amount, reaction time, work-up conditions or chromatographic purification conditions.

The stereochemistry of the compounds in the Examples, where indicated, has been assigned on the assumption that absolute configuration at resolved stereogenic centers of starting materials is maintained throughout any subsequent reaction conditions.

All solvents and commercial reagents were used as received. Where the preparation of starting materials is not described, these are commercially available, known in the literature, or readily obtainable by those skilled in the art using standard procedures.

Abbreviations

ACN (acetonitrile), BINAP (2,2'-Bis(diphenylphosphino)-1,1'-binaphthalene), COMU ((1-Cyano-2-ethoxy-2-oxoethylidenaminooxy)dimethylamino-morpholino-carbenium hexafluorophosphate), DCM (dichloromethane), DIPEA or DIEA (N-Ethyldiisopropylamine), DMF (N,N-Dimethylformamide), DMSO (Dimethylsulfoxide), dppf (1,1'-Ferrocenediyl-bis(diphenylphosphine)), EtOH (ethanol), EtOAc (ethyl acetate), FA (Formic acid), HATU (1-[Bis(dimethylamino) methylene]-1H-1,2,3-triazolo[4,5-b]pyridinium 3-oxid hexafluorophosphate, N-[(Dimethylamino)-1H-1,2,3-triazolo-[4,5-b]pyridin-1-ylmethylene]-N-methylmethanaminium hexafluorophosphate N-oxide), HPLC (High performance liquid chromatography), LCMS (Liquid chromatography - mass spectrometry), MDAP (Mass-directed auto-purification), MeOH (methanol), Me-THF (2-Methyltetrahydrofuran), MTBE (methyl tert-butyl ether), NMP (N-methylpyrrolidone), NMR (Nuclear magnetic resonance), Rt (Retention time), RT (Room temperature), SCX (Strong cation exchange), STAB (Sodium triacetoxyborohydride), TBTU (2-(1H-Benzotriazole-1-yl)-1,1,3,3-tetramethylaminium tetrafluoroborate), TEA (Triethylamine), TFA (Trifluoroacetic acid), THF (Tetrahydrofuran).

**PREPARATION OF INTERMEDIATES AND EXAMPLES**

**Example 1**

***Step A***

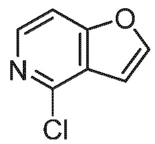

**4-Chlorofuro[3,2-c]pyridine (Intermediate 1A)**

A mixture of furo[3,2-c]pyridin-4-ol (70.4 g, 0.52 mol) in phosphoryl trichloride (430 mL) was heated at reflux for 1 h. Phosphoryl trichloride was distilled off, the residue poured into ice/water and neutralized to pH~6 with aqueous saturated $NaHCO_3$. The aqueous phase was extracted twice with DCM, then the organic layer was washed with saturated aqueous NaCl and evaporated to dryness. The crude material was purified by column chromatography on silica gel eluting with EtOAc-hexane to give the title compound (72.8 g).
LCMS (Method 10): Rt = 2.71 min, m/z 153.9 $[M+H]^+$

***Step B***

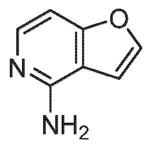

**Furo[3,2-c]pyridin-4-amine (Intermediate 1B)**

A solution of Intermediate 1A (72.8 g, 0.47 mol) in dry toluene (730 mL) was purged with argon over 20 min, then racemic BINAP (17.72 g, 0.028 mol), tris(dibenzylideneacetone)dipalladium(0) (8.69 g, 0.0095 mol) and potassium tert-butoxide (74.50 g, 0.66 mol) were added. After addition of benzophenone imine (95.5 mL, 0.57 mol), the mixture was heated at 90°C for 1.5 h. The reaction mixture was cooled to RT, diluted with THF and filtered through a pad of diatomaceous earth followed by washing with THF and diethyl ether. The combined filtrate was evaporated and the residue taken into MeOH (260 mL) and added dropwise to a solution of hydroxylamine hydrochloride (98.87 g, 1.42 mol) in MeOH (1200 mL) which had previously been neutralized in an ice bath with NaOH (56.91 g, 1.42 mol). The reaction mixture was stirred at RT for 1 h and evaporated to dryness. The crude material was purified by chromatography on silica by eluting with 10-100% EtOAc in hexane to give a solid that was further purified by trituration and filtration in a mixture of MTBE and

DCM. A second purification by chromatography on silica by eluting with 0-10% MeOH in DCM afforded the pure title compound (45.1 g).
LCMS (Method 11): Rt = 0.83 min, m/z 135.0 [M+H]+

***Step C***

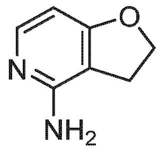

**2,3-Dihydrofuro[3,2-c]pyridin-4-amine (Internediate 1C)**

Intermediate 1B (44.1 g, 0.33 mol) was dissolved in MeOH (530 mL) and acetic acid (56.4 mL), then 10% Pd/C (50% wet, 17.74 g) was added and the reaction mixture purged with argon before being hydrogenated at a pressure of 10 bar of $H_2$ at 50°C under vigorous stirring. After 20 h a further half equivalent of 10% Pd/C (50% wet) and further 3 h of hydrogenation were needed in order to achieve full conversion. The reaction mixture was filtered and washed with MeOH. The combined filtrate was evaporated and the residue partitioned between EtOAc (500 mL) and water (500 mL). The aqueous layer was washed with further EtOAc (300 mL), neutralized with solid $NaHCO_3$ and saturated with NaCl. This aqueous mixture was extracted with DCM (8 x 300 mL) and the combined organic layers washed with saturated aqueous NaCl (800 mL), dried over $Na_2SO_4$ and evaporated to afford the title compound (24.57 g).
LCMS (Method 12): Rt = 0.81 min, m/z 137.1 [M+H]+

***Step D***

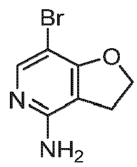

**7-Bromo-2,3-dihydrofuro[3,2-c]pyridin-4-amine (Intermediate 1D)**

Intermediate 1C (24.57 g, 0.180 mol) was dissolved in ACN (1230 mL) and then a solution of N-bromosuccinimide (35.33 g, 0.198 mol) in ACN (490 mL) was added dropwise over 3 h at -10°C in darkness. The reaction was quenched with aqueous saturated $NaHCO_3$ (500 mL), water (500 mL), EtOAc (1000 mL) and aqueous 5% NaCl (500 mL). The resulting organic and aqueous phases were separated, and the aqueous layer further washed with EtOAc (1000 mL). The combined organic layers were washed with aqueous 5% NaCl (7 x 2000 mL) and concentrated to dryness. The residual solid was treated with a mixture of EtOAc (500 mL) and water (200 mL), placed in a sonic bath for some minutes and acidified with aqueous 10% $KHSO_4$ (300 mL). The solid that appeared was collected by filtration. The biphasic filtrate was partitioned, and the organic layer washed twice with aqueous 10% $KHSO_4$ (200 mL each). The combined aqueous layer was washed with EtOAc (3 x 500 mL) and mixed with the previous collected solid. The resulting aqueous mixture was neutralized to pH7 with $NaHCO_3$ and extracted with EtOAc (3 x 1000 mL). The combined organic phase was washed with saturated aqueous NaCl (500 mL), dried over anhydrous $MgSO_4$, and concentrated to give the title compound (27.1 g).
LCMS (Method 13): Rt = 1.69 min, m/z 215.0/217.0 [M+H]+

***Step E***

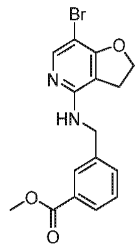

**Methyl 3-(((7-bromo-2,3-dihydrofuro[3,2-c]pyridin-4-yl)amino)methyl)-benzoate (Intermediate 1E)**

Intermediate 1D (15.6 g, 0.074 mol) and methyl 3-formylbenzoate (18.1g, 0.11 mol) were dissolved in anhydrous DCM (470 mL) with molecular sieves and kept under inert atmosphere. After 10 min, chloro(triisopropoxy)titanium(IV) (35.4 mL, 0.148 mol) was added dropwise and the resulting mixture stirred at RT over 2.5 h. Sodium triacetoxyborohydride (31.4g, 0.148 mol) followed by acetic acid (8.5 mL, 0.148 mol) were added and the mixture stirred at RT overnight. The reaction mixture was quenched with methanol and solvents were evaporated. The residue was dissolved in EtOAc and aqueous saturated $NaHCO_3$ solution. After being stirred for 15 min, the mixture was filtered through a thin pad of diatomaceous earth and washed with EtOAc. The combined filtrate was collected and organic-aqueous phases were separated. The organic layer was dried over $Na_2SO_4$ and evaporated. The crude material was purified by chromatography on silica by eluting with 20% - 40% EtOAc in hexane to give the title compound (19.3g).
LCMS (Method 9): Rt = 0.85 min, m/z 362.9/364.9 $[M+H]^+$

***Step F***

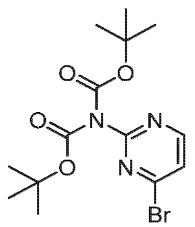

**tert-Butyl (4-bromopyrimidin-2-yl)(tert-butoxycarbonyl)carbamate (Intermediate 1F)**

A solution of 4-bromopyrimidin-2-amine (0.5 g, 2.87 mmol), di-tert-butyl dicarbonate (0.63 g, 2.87 mmol), potassium carbonate (0.79 g, 5.75 mmol) and a catalytic amount of DMAP in dioxane (4 mL) was stirred at ambient temperature for 18 h. Di-tert-butyl dicarbonate (0.94 g, 4.3 mmol) and potassium carbonate (1.58 g, 11.5 mmol) were added and the reaction mixture was stirred at 40°C for 4 h. The reaction mixture, diluted with EtOAc, was washed with saturated aqueous NaCl, the organic layer was dried with sodium sulphate and concentrated *in vacuo.* The residue was purified by flash chromatography on silica gel by eluting with 0-40% EtOAc in cyclohexane, the relevant fractions were combined and concentrated to give the title product (416 mg).
LCMS (Method 8): Rt = 3.69 min, m/z 396.0/398.0 $[M+Na]^+$

***Step G***

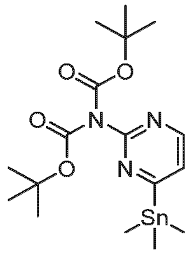

**tert-Butyl (tert-butoxycarbonyl)(4-(trimethylstannyl)pyrimidin-2-yl)-carbamate (Intermediate 1G)**

A degassed mixture of Intermediate 1F (310 mg, 0.828 mmol), hexamethylditin (0.19 mL, 0.911 mmol) and tetrakis(triphenylphosphine)palladium(0) (48 mg, 0.042 mmol) in THF (4 mL) was stirred at 80 °C for 6 h. The reaction mixture, diluted with EtOAc, was washed with saturated aqueous NaCl, the organic layer was dried with sodium sulphate and concentrated *in vacuo.* The solution was concentrated *in vacuo* and the residue was purified by flash chromatography on silica gel by eluting with 0-50% EtOAc in cyclohexane, the relevant fractions were combined and concentrated to give the desired product (240 mg).
LCMS (Method 3): Rt = 3.30 min, m/z 458.3-460.3 $[M+H]^+$

***Step H***

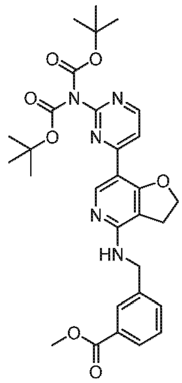

**Methyl 3-(((7-(2-(bis(tert-butoxycarbonyl)amino)pyrimidin-4-yl)-2,3-di-hydrofuro[3,2-c]pyridin-4-yl)amino)methyl)benzoate (Intermediate 1H)**

A degassed mixture of Intermediate 1E (1 g, 2.75 mmol), Intermediate 1G (1.39 g, 3.03 mmol), tetrakis(triphenylphosphine)palladium(0) (160 mg, 0.138 mmol) and copper(I) thiophene-2-carboxylate (53 mg, 0.275 mmol) in dioxane (15 mL) was heated at 130 °C under microwave irradiation for 1.5 h. The reaction mixture, diluted with ethyl acetate, was filtered through a pad of diatomaceous earth. The solution was concentrated *in vacuo* and the residue was purified by cromatography on a silica gel cartridge eluting with 0-100% ethyl acetate in cyclohexane to give the title product (929 mg).
LCMS (Method 3): Rt = 3.21 min, m/z 578.5 $[M+H]^+$

***Step I***

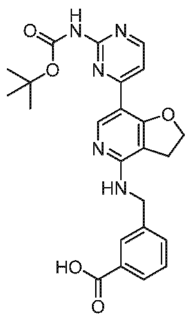

**3-(((7-(2-((tert-Butoxycarbonyl)amino)pyrimidin-4-yl)-2,3-dihydrofuro[3,2-c]pyridin-4-yl)amino)methyl)benzoic acid (Intermediate 11)**

A mixture of Intermediate 1H (929 mg, 1.62 mmol), lithium hydroxide monohydrate (0.075 g, 1.78 mmol) in THF (3 mL), methanol (3 mL) and water (6 mL) was stirred at ambient temperature for 18 h. Further lithium hydroxide monohydrate

(0.15 g, 3.56 mmol) was added and the reaction mixture was stirred for a further 5 h. The resulting mixture was diluted with water and extracted with ethyl acetate. The pH of the aqueous phase was adjusted to pH ~6-7 with aqueous 1M HCl. The organic layer was dried with $Na_2SO_4$ and evaporated *in vacuo* to give the title product (750 mg).
LCMS (Method 3): Rt = 1.49 min, m/z 464.3 $[M+H]^+$

***Step J***

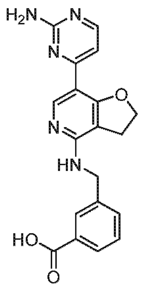

**3-(((7-(2-Aminopyrimidin-4-yl)-2,3-dihydrofuro[3,2-c]pyridin-4-yl)amino)methyl)benzoic acid (Intermediate 1J)**

A mixture of Intermediate 1I (2 g, 4.32 mmol) in TFA (10 mL) and dichloromethane (40 mL) was stirred at ambient temperature for 2 h. The reaction mixture, diluted with MeOH, was purified by an SCX-2 cartridge by eluting with MeOH and then 2M methanolic ammonia. The ammonia solution was concentrated *in vacuo* to give the title product (1.5 g).
LCMS (Method 4): Rt = 2.15 min, m/z 364.0 $[M+H]^+$

***Step K***

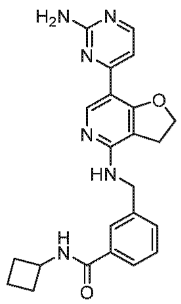

**3-(((7-(2-Aminopyrimidin-4-yl)-2,3-dihydrofuro[3,2-c]pyridin-4-yl)amino)methyl)-N-cyclobutylbenzamide (Example 1)**

A solution of Intermediate 1J (50 mg, 0.138 mmol), cyclobutylamine (13 μL, 0.151 mmol), TBTU (57 mg, 0.179 mmol) and DIPEA (72 μL, 0.413 mmol) in DMF (1.0 mL) was stirred at RT for 66 h. The reaction mixture was diluted with EtOAc and washed with water. The organic layer was passed through a phase separator, loaded onto an SCX-2 cartridge, washed with MeOH and eluted with 2N methanolic ammonia, and the latter was concentrated *in vacuo.* The residue was purified by flash chromatography on a silica gel cartridge eluting with 0 to 6% 2N methanolic ammonia in DCM, concentrated and freeze-dried to afford the desired product (49 mg).

LCMS (Method 6): Rt = 2.40 min, m/z 417.1 $[M+H]^+$
$^1$H NMR (400 MHz, d6-DMSO) δ 8.66 (s, 1H), 8.58 (d, J=7.5 Hz, 1H), 8.15 (d, J=5.2 Hz, 1H), 7.82 (s, 1H), 7.68 (d, J=7.8 Hz, 1H), 7.47 (d, J=7.8 Hz, 1H), 7.39-7.35 (m, 1H), 7.18-7.14 (m, 1H), 7.06 (d, J=5.2 Hz, 1H), 6.41 (s, 2H), 4.74 (t, J=8.9 Hz, 2H), 4.67 (d, J=5.8 Hz, 2H), 4.40 (dt, J=8.4, 16.4 Hz, 1H), 3.03 (t, J=8.9 Hz, 2H), 2.24-2.17 (m, 2H), 2.11-2.01 (m, 2H), 1.70-1.61 (m, 2H).

**Example 2 to 50**

The following examples were prepared from intermediate 1J and the given amine using a method similar to that used for *example 1*.

Example 32-33, 34-35 and 45-47 were prepared as racemic mixture by using the amine indicated in the table and then resolved by chiral SFC according to the methods described below.

| Example | Structure | Amine | 1H NMR | LC-MS |
|---|---|---|---|---|
| 2 | 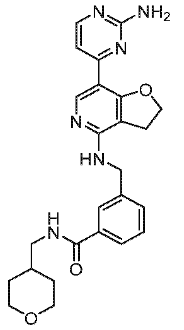 3-(((7-(2-Aminopyrimidin-4-yl)-2,3-dihydrofuro[3,2-c] pyridin-4-yl)amino) methyl)-N-((tetrahydro-2H-pyran-4-yl)methyl)benzamide | (Tetrahydro-2H-pyran-4-yl)methanamine | (400 MHz, d6-DMSO) δ 8.66 (s, 1H), 8.44 (dd, J=5.8, 5.8 Hz, 1H), 8.15 (d, J=5.2 Hz, 1H), 7.83 (s, 1H), 7.68 (d, J=7.8 Hz, 1H), 7.47 (d, J=7.8 Hz, 1H), 7.39-7.35 (m, 1H), 7.15 (t, J=6.1 Hz, 1H), 7.05 (d, J=5.2 Hz, 1H), 6.40 (s, 2H), 4.74 (t, J=8.9 Hz, 2H), 4.67 (d, J=6.3 Hz, 2H), 3.83 (dd, J=2.5, 11.3 Hz, 2H), 3.25 (dt, J=1.8, 11.6 Hz, 2H), 3.14 (t, J=6.4 Hz, 2H), 3.03 (t, J=8.9 Hz, 2H), 1.83-1.73 (m, 1H), 1.57 (dd, J=1.8, 12.8 Hz, 2H), 1.24-1.12 (m, 2H). | Rt = 2.42 min, m/z 461.1 $[M+H]^+$ (Method 6) |
| 3 | 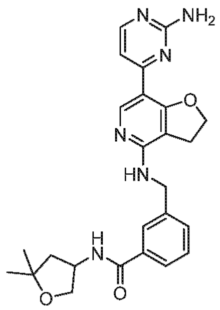 3-(((7-(2-Aminopyrimidin-4-yl)-2,3-dihydrofuro[3,2-c] pyridin-4-yl)amino) methyl)-N-(5,5-dimethyltetrahydrofuran-3-yl)benzamide | 5,5-Dimethyltetrahy drofuran-3-amine | (400 MHz, d6-DMSO) δ 8.66 (s, 1H), 8.46 (d, J=6.5 Hz, 1H), 8.15 (d, J=5.3 Hz, 1H), 7.83 (s, 1H), 7.69 (d, J=7.8 Hz, 1H), 7.48 (d, J=7.8 Hz, 1H), 7.40-7.35 (m, 1H), 7.18-7.13 (m, 1H), 7.05 (d, J=5.3 Hz, 1H), 6.40 (s, 2H), 4.74 (t, J=8.8 Hz, 2H), 4.68 (d, J=6.0 Hz, 2H), 4.52 (dt, J=7.0, 14.4 Hz, 1H), 3.98 (dd, J=7.0, 8.8 Hz, 1H), 3.60 (dd, J=6.3, 8.9 Hz, 1H), 3.03 (t, J=8.9 Hz, 2H), 2.07 (dd, J=8.4, 12.5 Hz, 1H), 1.80 (dd, J=7.2, 12.5 Hz, 1H), 1.27 (s, 3H), 1.17 (s, 3H). | Rt = 2.34 min, m/z 461.2 $[M+H]^+$ (Method 6) |
| 4 | 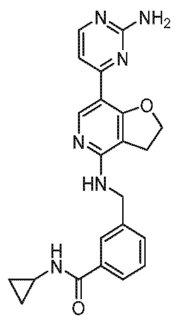 3-(((7-(2-Aminopyrimidin-4-yl)-2,3-dihydrofuro[3,2-c] pyridin-4-yl)amino) methyl)-N-cyclopropylbenzamide | Cyclopropanami ne | (400 MHz, d6-DMSO) δ 8.66 (s, 1H), 8.40 (d, J=4.1 Hz, 1H), 8.15 (d, J=5.3 Hz, 1H), 7.81 (s, 1H), 7.64 (d, J=7.8 Hz, 1H), 7.46 (d, J=7.8 Hz, 1H), 7.38-7.33 (m, 1H), 7.15 (t, J=6.0 Hz, 1H), 7.05 (d, J=5.2 Hz, 1H), 6.40 (s, 2H), 4.73 (t, J=9.0 Hz, 2H), 4.66 (d, J=6.2 Hz, 2H), 3.03 (t, J=8.9 Hz, 2H), 2.83 (dt, J=4.0, 7.3 Hz, 1H), 0.71-0.65 (m, 2H), 0.58-0.53 (m, 2H). | Rt = 2.11 min, m/z 403.1 $[M+H]^+$ (Method 6) |

(continued)

| Example | Structure | Amine | 1H NMR | LC-MS |
|---|---|---|---|---|
| 5 | 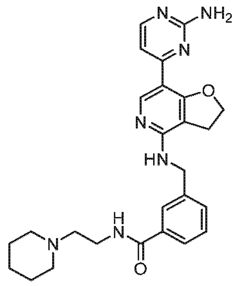 3-(((7-(2-Aminopyrimidin-4-yl)-2,3-dihydrofuro[3,2-c] pyridin-4-yl)amino) methyl)-N-(2-(piperidin-1-yl) ethyl)benzamide | 2-(Piperidin-1-yl) ethan-1-amine | (400 MHz, d6-DMSO) δ 8.66 (s, 1H), 8.30 (dd, J=5.6, 5.6 Hz, 1H), 8.15 (d, J=5.2 Hz, 1H), 7.81 (s, 1H), 7.66 (d, J=7.8 Hz, 1H), 7.47 (d, J=7.8 Hz, 1H), 7.40-7.35 (m, 1H), 7.15 (dd, J=6.1, 6.1 Hz, 1H), 7.05 (d, J=5.3 Hz, 1H), 6.40 (s, 2H), 4.73 (t, J=9.0 Hz, 2H), 4.67 (d, J=6.1 Hz, 2H), 3.36 (t, J=5.9 Hz, 2H), 3.03 (t, J=8.9 Hz, 2H), 2.41 (t, J=7.1 Hz, 2H), 2.38-2.33 (m, 4H), 1.51-1.43 (m, 4H), 1.39-1.34 (m, 2H). | Rt = 1.77 min, m/z 474.3 $[M+H]^+$ (Method 6) |
| 6 | 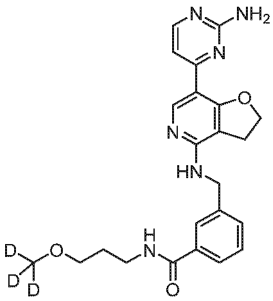 3-(((7-(2-aminopyrimidin-4-yl)-2,3-dihydrofuro[3,2-c] pyridin-4-yl)amino) methyl)-N-(3-(methoxy-d3) propyl)benzamide | Intermediate 6B | (400 MHz, d6-DMSO) δ 8.68 (s, 1H), 8.43 (t, J=5.6 Hz, 1H), 8.16 (d, J=5.3 Hz, 1H), 7.84 (s, 1H), 7.68 (d, J=7.8 Hz, 1H), 7.48 (d, J=7.9 Hz, 1H), 7.41 - 7.36 (m, 1H), 7.17 (t, J=6.1 Hz, 1H), 7.07 (d, J=5.3 Hz, 1H), 6.42 (s, 2H), 4.75 (t, J=9.0 Hz, 2H), 4.68 (d, J=6.0 Hz, 2H), 3.37 (t, J=6.3 Hz, 2H), 3.33 - 3.26 (m, 2H), 3.04 (t, J=9.0 Hz, 2H), 1.79 - 1.72 (m, 2H). | Rt = 2.32 min, m/z 438.0 $[M+H]^+$ (Method 4) |
| 7 | 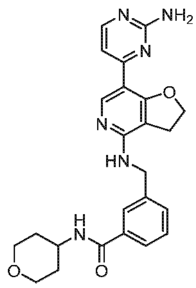 3-(((7-(2-Aminopyrimidin-4-yl)-2,3-dihydrofuro[3,2-c] pyridin-4-yl)amino) methyl)-N-(tetrahydro-2H-pyran-4-yl)benzamide | Tetrahydro-2H-pyran-4-amine | (400 MHz, d6-DMSO) δ 8.66 (s, 1H), 8.28 (d, J=7.7 Hz, 1H), 8.15 (d, J=5.2 Hz, 1H), 7.83 (s, 1H), 7.69 (d, J=7.8 Hz, 1H), 7.47 (d, J=7.8 Hz, 1H), 7.40-7.35 (m, 1H), 7.16 (t, J=6.1 Hz, 1H), 7.06 (d, J=5.2 Hz, 1H), 6.40 (s, 2H), 4.73 (t, J=8.9 Hz, 2H), 4.68 (d, J=6.0 Hz, 2H), 4.03-3.93 (m, 1H), 3.90-3.84 (m, 2H), 3.37 (t, J=11.8 Hz, 2H), 3.03 (t, J=8.9 Hz, 2H), 1.76-1.71 (m, 2H), 1.63-1.46 (m, 2H). | Rt = 2.09 min, m/z 447.2 $[M+H]^+$ (Method 6) |

(continued)

| Example | Structure | Amine | 1H NMR | LC-MS |
|---|---|---|---|---|
| 8 | 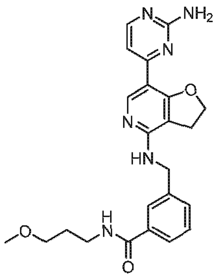 3-(((7-(2-Aminopyrimidin-4-yl)-2,3-dihydrofuro[3,2-c] pyridin-4-yl)amino) methyl)-N-(3-methoxypropyl)benzamide | 3-Methoxypropan-1-amine | (400 MHz, d6-DMSO) δ 8.66 (s, 1H), 8.42 (dd, J=5.6, 5.6 Hz, 1H), 8.15 (d, J=5.3 Hz, 1H), 7.84-7.81 (m, 1H), 7.67 (d, J=7.7 Hz, 1H), 7.47 (d, J=7.8 Hz, 1H), 7.40-7.35 (m, 1H), 7.16 (t, J=6.1 Hz, 1H), 7.06 (d, J=5.2 Hz, 1H), 6.40 (s, 2H), 4.73 (t, J=8.9 Hz, 2H), 4.67 (d, J=6.1 Hz, 2H), 3.36 (t, J=6.1 Hz, 2H), 3.28 (dd, J=6.8, 12.9 Hz, 2H), 3.23 (s, 3H), 3.03 (t, J=8.9 Hz, 2H), 1.78-1.70 (m, 2H). | Rt = 2.17 min, m/z 435.2 $[M+H]^+$ (Method 6) |
| 9 | 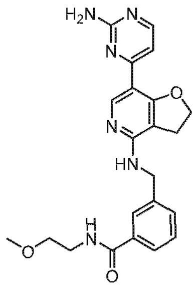 3-(((7-(2-Aminopyrimidin-4-yl)-2,3-dihydrofuro[3,2-c] pyridin-4-yl)amino) methyl)-N-(2-methoxyethyl) benzamide | 2-Methoxyethan-1-amine | (400 MHz, d6-DMSO) δ 8.68 (s, 1H), 8.49 (t, J = 5.0Hz, 1H), 8.06 (d, J = 5.4Hz, 1H), 7.86-7.84 (m, 1H), 7.72-7.68 (m, 1H), 7.51-7.46 (m, 1H), 7.39 (t, J = 7.5Hz, 1H), 7.17 (t, J = 7.2Hz, 1H), 7.07 (d, J = 5.0Hz, 1H), 6.42 (s, 2H), 4.75 (t, J = 8.9Hz, 2H), 4.69 (d, J = 5.7Hz, 2H), 3.49-3.38 (m, 4H), 3.27 (s, 3H), 3.04 (t, J = 8.8Hz, 2H). | Rt = 2.14 min, m/z 421.0 $[M+H]^+$ (Method 4) |
| 10 | 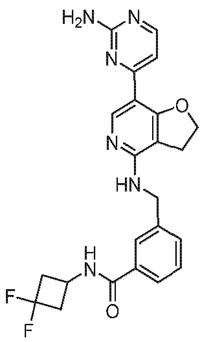 3-(((7-(2-Aminopyrimidin-4-yl)-2,3-dihydrofuro[3,2-c] pyridin-4-yl)amino) methyl)-N-(3,3-difluorocyclobutyl)benzam ide | 3,3-Difluorocyclobu tan-1-amine | (400 MHz, d6-DMSO) δ 8.82 (d, J = 7.2Hz, 1H), 8.67 (s, 1H), 8.16 (d, J = 5.7Hz, 1H), 7.85 (s, 1H), 7.73-7.69 (m, 1H), 7.54-7.49 (m, 1H), 7.42 (t, J = 8.0Hz, 1H), 7.18 (t, J = 6.3Hz, 1H), 7.07 (d, J = 5.3Hz, 1H), 6.42 (s, 2H), 4.75 (t, J = 8.9Hz, 2H), 4.69 (d, J = 5.8 Hz, 2H), 4.33-4.21 (m, 1H), 3.04 (t, J = 8.8Hz, 2H), 3.00-2.88 (m, 2H), 2.84-2.70 (m, 2H). | Rt = 2.62 min, m/z 453.0 $[M+H]^+$ (Method 4) |

(continued)

| Example | Structure | Amine | 1H NMR | LC-MS |
|---|---|---|---|---|
| 11 | 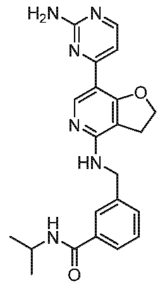 3-(((7-(2-Aminopyrimidin-4-yl)-2,3-dihydrofuro[3,2-c] pyridin-4-yl)amino) methyl)-N-isopropylbenzamide | Propan-2-amine | (400 MHz, d6-DMSO) δ 8.68 (s, 1H), 8.19 (d, J = 7.3Hz, 1H), 8.16 (d, J = 5.1, 1H), 7.85-7.82 (m, 1H), 7.71-7.67 (m, 1H), 7.50-7.45 (m, 1H), 7.38 (t, J = 7.6Hz, 1H), 7.17 (t, J = 5.9Hz, 1H), 7.07 (d, J = 5.1Hz, 1H), 6.42 (s, 2H), 4.75 (t, J = 4.8Hz, 2H), 4.68 (d, J = 5.9Hz, 2H), 4.14-4.03 (m, 1H), 3.04 (t, J = 8.8Hz, 2H), 1.17 (d, J = 6.7Hz, 6H). | Rt = 2.38 min, m/z 405.0 [M+H]+ (Method 4) |
| 12 | 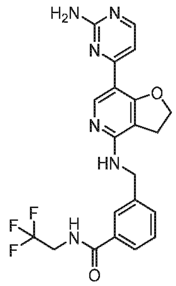 3-(((7-(2-Aminopyrimidin-4-yl)-2,3-dihydrofuro[3,2-c] pyridin-4-yl)amino) methyl)-N-(2,2,2-trifluoroethyl)benzamide | 2,2,2-Trifluoroethan-1-amine | (400 MHz, d6-DMSO) δ 9.08 (t, J = 6.2Hz, 1H), 8.68 (s, 1H), 8.16 (d, J = 5.2Hz, 1H), 7.90 (s, 1H), 7.77-7.73 (m, 1H), 7.57-7.52 (m, 1H), 7.44 (t, J = 7.7Hz, 1H), 7.19 (t, J = 5.7Hz, 1H), 7.07 (d, J = 5.3Hz, 1H), 6.42 (s, 2H), 4.75 (t, J = 9.1Hz, 2H), 4.70 (d, J = 6.1Hz, 2H), 4.15-4.02 (m, 2H), 3.05 (t, J = 8.9Hz, 2H). | Rt = 2.54 min, m/z 445.0 [M+H]+ (Method 4) |
| 13 | 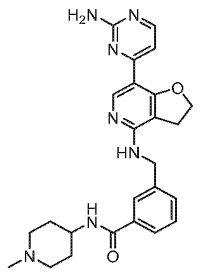 3-(((7-(2-Aminopyrimidin-4-yl)-2,3-dihydrofuro[3,2-c] pyridin-4-yl)amino) methyl)-N-(1-methylpiperidin-4-yl)benzamide | 1-Methylpiperidin-4-amine | (400 MHz, d6-DMSO) δ 8.68 (s, 1H), 8.22 (d, J = 7.8Hz, 1H), 8.16 (d, J = 5.4Hz, 1H), 7.84 (s, 1H), 7.71-7.67 (m, 1H), 7.50-7.45 (m, 1H), 7.38 (t, J = 7.2Hz, 1H), 7.17 (t, J = 5.5Hz, 1H), 7.07 (d, J = 5.0Hz, 1H), 6.42 (s, 2H), 4.75 (t, J = 9.0Hz, 2H), 4.66 (d, J = 5.8Hz, 2H), 3.72 (s, 1H), 3.05 (t, J = 9.3Hz, 2H), 2.80-2.71 (m, 2H), 2.16 (s, 3H), 1.98-1.88 (m, 2H), 1.79-1.71 (m, 2H), 1.64-1.52 (m, 2H). | Rt = 1.72 min, m/z 460.0 [M+H]+ (Method 4) |

(continued)

| Example | Structure | Amine | 1H NMR | LC-MS |
|---|---|---|---|---|
| 14 | 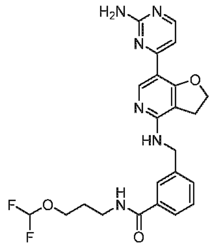 3-(((7-(2-Aminopyrimidin-4-yl)-2,3-dihydrofuro[3,2-c] pyridin-4-yl)amino) methyl)-N-(3-(difluoromethoxy)propyl)b enzamide | 3-(Difluoromethox y) propan-1-amine | $^1$H NMR (400 MHz, d6-DMSO) δ 8.68 (s, 1H), 8.51 (t, J=5.6 Hz, 1H), 8.17 - 8.15 (m, 1H), 7.84 (s, 1H), 7.69 (d, J=7.7 Hz, 1H), 7.51 - 7.47 (m, 1H), 7.40 (t, J=7.7 Hz, 1H), 7.17 (t, J=6.1 Hz, 1H), 7.07 (d, J=5.3 Hz, 1H), 6.67 (t, J=76.9 Hz, 1H), 6.44 - 6.41 (m, 2H), 4.75 (t, J=8.9 Hz, 2H), 4.69 (d, J=6.0 Hz, 2H), 3.89 (t, J=6.4 Hz, 2H), 3.05 (t, J=8.9 Hz, 2H), 1.88 - 1.83 (m, 2H). | Rt = 4.07 min, m/z 471.0 $[M+H]^+$ (Method 5) |
| 15 | 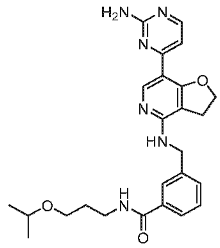 3-(((7-(2-Aminopyrimidin-4-yl)-2,3-dihydrofuro[3,2-c] pyridin-4-yl)amino) methyl)-N-(3-isopropoxypropyl)benzami de | 3-Isopropoxypropa n-1-amine | $^1$H NMR (400 MHz, d6-DMSO) δ 8.68 (s, 1H), 8.40 (t, J=5.6 Hz, 1H), 8.16 (d, J=5.4 Hz, 1H), 7.83 (s, 1H), 7.69 - 7.66 (m, 1H), 7.50 - 7.46 (m, 1H), 7.39 (t, J=7.7 Hz, 1H), 7.17 (t, J=6.1 Hz, 1H), 7.07 (d, J=5.3 Hz, 1H), 6.42 (s, 2H), 4.75 (t, J=9.0 Hz, 2H), 4.69 (d, J=6.0 Hz, 2H), 3.57 - 3.47 (m, 1H), 3.41 (t, J=6.3 Hz, 2H), 3.32 - 3.27 (m, 2H), 3.08 - 3.01 (m, 2H), 1.77 - 1.68 (m, 2H), 1.08 (d, J=6.0 Hz, 6H). | Rt = 2.75 min, m/z 463.0 $[M+H]^+$ (Method 4) |
| 16 | 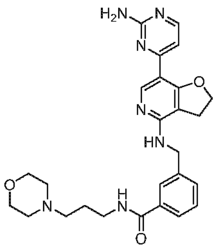 3-(((7-(2-Aminopyrimidin-4-yl)-2,3-dihydrofuro[3,2-c] pyridin-4-yl)amino) methyl)-N-(3-morpholinopropyl)benzam ide | 3-Morpholinoprop an-1-amine | $^1$H NMR (400 MHz, d6-DMSO) δ 8.68 (br s, 1H), 8.46 (t, J=5.5 Hz, 1H), 8.16 (d, J=5.2 Hz, 1H), 7.83 (s, 1H), 7.68 (d, J=7.6 Hz, 1H), 7.48 (d, J=7.7 Hz, 1H), 7.39 (t, J=7.7 Hz, 1H), 7.17 (t, J=6.1 Hz, 1H), 7.07 (d, J=5.2 Hz, 1H), 6.42 (br s, 2H), 4.75 (t, J=9.0 Hz, 2H), 4.69 (d, J=6.0 Hz, 2H), 3.57 (t, J=4.6 Hz, 4H), 3.29 (q, J=6.6 Hz, 2H), 3.04 (t, J=8.8 Hz, 2H), 2.30-2.38 (m, 6H), 1.68 (p, J=6.9 Hz, 2H). | Rt = 1.78 min, m/z 490.3 $[M+H]^+$ (Method 4) |

(continued)

| Example | Structure | Amine | 1H NMR | LC-MS |
|---|---|---|---|---|
| 17 | 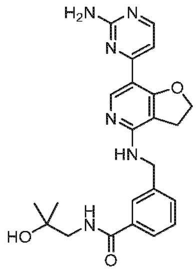 3-(((7-(2-Aminopyrimidin-4-yl)-2,3-dihydrofuro[3,2-c] pyridin-4-yl)amino) methyl)-N-(2-hydroxy-2-methylpropyl)benzamide | 1-Amino-2-methylpro-pan-2-ol | $^1$H NMR (400 MHz, d6-DMSO) δ 8.68 (br s, 1H), 8.15-8.22 (m, 2H), 7.86 (s, 1H), 7.72 (d, J=7.8 Hz, 1H), 7.49 (d, J=7.6 Hz, 1H), 7.40 (t, J=7.7 Hz, 1H), 7.17 (t, J=6.1 Hz, 1H), 7.07 (d, J=5.3 Hz, 1H), 6.42 (br s, 1H), 4.75 (t, J=8.9 Hz, 2H), 4.70 (d, J=6.0 Hz, 2H), 4.57 (br s, 1H), 3.26 (d, J=6.1 Hz, 2H), 3.05 (t, J=8.8 Hz, 2H), 1.11 (s, 6H). | Rt = 2.13 min, m/z 435.3 $[M+H]^+$ (Method 4) |
| 18 | 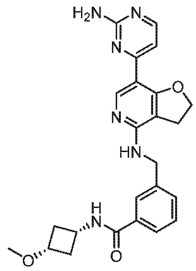 3-(((7-(2-aminopyrimidin-4-yl)-2,3-dihydrofuro[3,2-c] pyridin-4-yl)amino) methyl)-N-((1s,3s)-3-methoxycyclobutyl)benza mide | (1S,3 S)-3-Methoxy-cyclobu tan-1-amine | $^1$H NMR (400 MHz, d6-DMSO) δ 8.68 (s, 1H), 8.59 (d, J=7.7 Hz, 1H), 8.16 (d, J=5.3 Hz, 1H), 7.84 (s, 1H), 7.70 (d, J=7.9 Hz, 1H), 7.49 (d, J=7.9 Hz, 1H), 7.39 (t, J=7.7 Hz, 1H), 7.17 (t, J=6.0 Hz, 1H), 7.07 (d, J=5.3 Hz, 1H), 6.42 (s, 2H), 4.75 (t, J=8.7 Hz, 2H), 4.68 (d, J=6 Hz, 2H), 4.07-3.99 (m, 1H), 3.62 (d, J=7.26 Hz, 1H), 3.16 (s, 3H), 3.04 (t, J=9.1 Hz, 2H), 2.63-2.54 (m, 2H), 2.00-1.91 (m, 2H). | Rt = 2.39 min, m/z 447.0 $[M+H]^+$ (Method 4) |
| 19 | 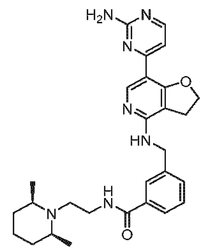 3-(((7-(2-Aminopyrimidin-4-yl)-2,3-dihydrofuro[3,2-c] pyridin-4-yl)amino) methyl)-N-(2-((2R,6S)-2,6-dimethylpiperidin-1-yl)ethyl) benzamide | 2-((2R,6S)-2,6-Dimethylpiperid in-1-yl) ethan-1-amine | $^1$H NMR (400 MHz, d6-DMSO) δ 8.68 (s, 1H), 8.43 (t, J=5.7 Hz, 1H), 8.16 (d, J=5.3 Hz, 1H), 7.83 (s, 1H), 7.67 (d, J=7.9 Hz, 1H), 7.48 (d, J=7.7 Hz, 1H), 7.39 (t, J=7.7 Hz, 1H), 7.17 (t, J=6.2 Hz, 1H), 7.07 (d, J=5.3 Hz, 1H), 6.42 (s, 2H), 4.75 (t, J=8.9 Hz, 2H), 4.69 (d, J=6 Hz, 2H), 3.29-3.22 (m, 2H), 3.04 (t, 2H, J=8.7 Hz, 2H), 2.72-2.66 (m, 2H), 2.50-2.40 (m, 2H), 1.63-1.54 (m, 1H), 1.54-1.47 (m, 2H), 1.35-1.21 (m, 1H), 1.17-1.06 (m, 8H). | Rt = 2.03 min, m/z 502.5 $[M+H]^+$ (Method 4) |

(continued)

| Example | Structure | Amine | 1H NMR | LC-MS |
|---|---|---|---|---|
| 20 | 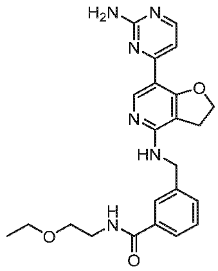 3-(((7-(2-Aminopyrimidin-4-yl)-2,3-dihydrofuro[3,2-c] pyridin-4-yl)amino) methyl)-N-(2-ethoxyethyl) benzamide | 2-Ethoxyethan-1-amine | $^1$H NMR (400 MHz, d6-DMSO) δ 8.68 (s, 1H), 8.48 (t, J=5.5 Hz, 1H), 8.16 (d, J=5.3 Hz, 1H), 7.85 (s, 1H), 7.72 - 7.68 (m, 1H), 7.51 - 7.47 (m, 1H), 7.39 (t, J=7.7 Hz, 1H), 7.17 (t, J=6.1 Hz, 1H), 7.07 (d, J=5.3 Hz, 1H), 6.42 (s, 2H), 4.75 (t, J=8.9 Hz, 2H), 4.69 (d, J=6.0 Hz, 2H), 3.51 - 3.38 (m, 6H), 3.05 (t, J=8.9 Hz, 2H), 1.11 (t, J=7.0 Hz, 3H). | Rt = 3.79 min, m/z 435.0 $[M+H]^+$ (Method 5) |
| 21 | 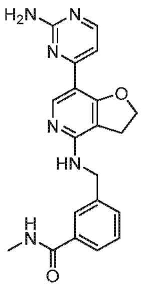 3-(((7-(2-Aminopyrimidin-4-yl)-2,3-dihydrofuro[3,2-c] pyridin-4-yl)amino) methyl)-N-methylbenza-mide | Methylamine | $^1$H NMR (400 MHz, d6-DMSO) δ 8.68 (s, 1H), 8.44-8.37 (m, 1H), 8.16 (d, J=5.3 Hz, 1H), 7.83 (s, 1H), 7.71-7.64 (m, 1H), 7.50-7.46 (m, 1H), 7.41-7.36 (m, 1H), 7.20-7.15 (m, 1H), 7.07 (d, J=5.3 Hz, 1H), 6.42 (s, 2H), 4.75 (t, J=9.1 Hz, 2H), 4.69 (d, J=6.0 Hz, 2H), 3.04 (t, J=8.8 Hz, 2H), 2.78 (d, J=4.6 Hz, 3H). | Rt = 2.01 min, m/z 377.0 $[M+H]^+$ (Method 4) |
| 22 | 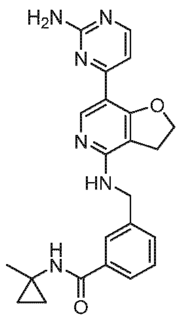 3-(((7-(2-Aminopyrimidin-4-yl)-2,3-dihydrofuro[3,2-c] pyridin-4-yl)amino) methyl)-N-(1-methylcyclopropyl)benza mide | 1-Methylcyclopro pan-1-amine | $^1$H NMR (400 MHz, d6-DMSO) δ 8.68 (s, 1H), 8.62 (s, 1H), 8.16 (d, J=5.3 Hz, 1H), 7.82 (s, 1H), 7.67 - 7.64 (m, 1H), 7.48 - 7.45 (m, 1H), 7.35 (t, J=7.7 Hz, 1H), 7.16 (t, J=6.0 Hz, 1H), 7.07 (d, J=5.3 Hz, 1H), 6.42 (s, 2H), 4.75 (t, J=9.0 Hz, 2H), 4.67 (d, J=6.0 Hz, 2H), 3.04 (t, J=8.9 Hz, 2H), 1.37 (s, 3H), 0.75 - 0.71 (m, 2H), 0.63 - 0.59 (m, 2H). | Rt = 2.44 min, m/z 417.0 $[M+H]^+$ (Method 4) |

(continued)

| Example | Structure | Amine | 1H NMR | LC-MS |
|---|---|---|---|---|
| 23 | 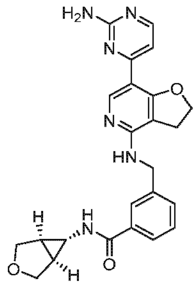 3-(((7-(2-Aminopyrimidin-4-yl)-2,3-dihydrofuro[3,2-c] pyridin-4-yl)amino) methyl)-N-((1R,5S,6r)-3-oxabicyclo[3.1.0]hexan-6-yl) benzamide | (1R,5S,6R)-3-Oxabicyclo[3.1. 0]hexan-6-amine | $^1$H NMR (400 MHz, d6-DMSO) δ 8.67 (s, 1H), 8.47 (d, J=4.1 Hz, 1H), 8.16 (d, J=5.3 Hz, 1H), 7.82 (s, 1H), 7.68 - 7.64 (m, 1H), 7.51 - 7.47 (m, 1H), 7.38 (t, J=7.7 Hz, 1H), 7.18 (t, J=6.1 Hz, 1H), 7.07 (d, J=5.3 Hz, 1H), 6.44 - 6.41 (m, 2H), 4.75 (t, J=8.9 Hz, 2H), 4.68 (d, J=6.0 Hz, 2H), 3.88 - 3.85 (m, 2H), 3.64 (d, J=8.4 Hz, 2H), 3.04 (t, J=8.9 Hz, 2H), 2.62 - 2.59 (m, 1H), 1.90 - 1.89 (m, 2H). | Rt = 2.24 min, m/z 445.0 $[M+H]^+$ (Method 4) |
| 24 | 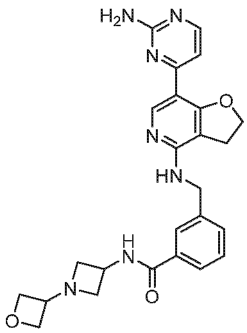 3-(((7-(2-aminopyrimidin-4-yl)-2,3-dihydrofuro[3,2-c] pyridin-4-yl)amino) methyl)-N-(1-(oxetan-3-yl) azetidin-3-yl)benzamide | 1-(oxetan-3-yl)azetidin-3-amine | $^1$H NMR (400 MHz, d6-DMSO) δ 8.79 (d, J=7.2 Hz, 1H), 8.68 (s, 1H), 8.16 (d, J=5.4 Hz, 1H), 7.85 (s, 1H), 7.72 (d, J=7.8 Hz, 1H), 7.50 (d, J=7.8 Hz, 1H), 7.41 (t, J=7.7 Hz, 1H), 7.18 (t, J=6.1 Hz, 1H), 7.07 (d, J=5.3 Hz, 1H), 6.43 (s, 2H), 4.75 (t, J=9.3 Hz, 2H), 4.69 (d, J=6.3 Hz, 2H), 4.60 - 4.53 (m, 3H), 4.44 - 4.40 (m, 2H), 3.79 - 3.72 (m, 1H), 3.56 (t, J=7.3 Hz, 2H), 3.16 (t, J=7.3 Hz, 2H), 3.08 - 3.01 (m, 2H). | Rt = 1.77 min, m/z 474.0 $[M+H]^+$ (Method 4) |
| 25 | 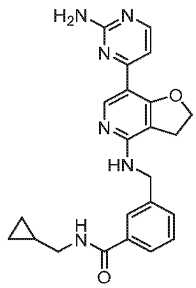 3-(((7-(2-Aminopyrimidin-4-yl)-2,3-dihydrofuro[3,2-c] pyridin-4-yl)amino) methyl)-N-(cyclopropylmethyl)benza mide | Cyclopropylmet hanamine | $^1$H NMR (400 MHz, d6-DMSO) δ 8.64 (s, 1H), 8.49 (t, J=5.6Hz, 1H), 8.12 (d, J=5.3 Hz, 1H), 7.82 (s, 1H), 7.67 (d, J=7.5 Hz, 1H), 7.45 (d, J=7.9 Hz, 1H), 7.38 - 7.33 (m, 1H), 7.14 (t, J=6.0 Hz, 1H), 7.03 (d, J=5.3 Hz, 1H), 6.38 (s, 2H), 4.71 (t, J=8.9 Hz, 2H), 4.66 (d, J=5.8 Hz, 2H), 3.11 (t, J=6.2 Hz, 2H), 3.01 (t, J=8.8 Hz, 2H), 1.10 - 0.96 (m, 1H), 0.42 - 0.37 (m, 2H), 0.22 - 0.17 (m, 2H). | Rt = 2.49 min, m/z 417.0 $[M+H]^+$ (Method 4) |

(continued)

| Example | Structure | Amine | 1H NMR | LC-MS |
|---|---|---|---|---|
| 26 | 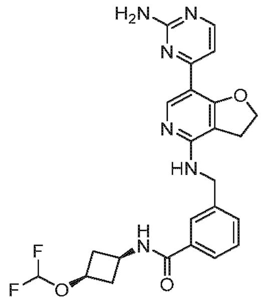 3-(((7-(2-aminopyrimidin-4-yl)-2,3-dihydrofuro[3,2-c] pyridin-4-yl)amino) methyl)-N-((1s,3s)-3-(difluoromethoxy)cyclobut yl) benzamide | (1s,3s)-3-(difluoromethox y)cyclobutan-1-amine | $^1$H NMR (400 MHz, d6-DMSO) δ 8.67 (m, 2H), 8.17 (d, J=5.4 Hz, 1H), 7.85 (s, 1H), 7.71 (d, J=7.8 Hz, 1H), 7.50 (d, J=7.9 Hz, 1H), 7.42 - 7.38 (m, 1H), 7.09 (d, J=5.4 Hz, 1H), 6.51 (s, 2H), 4.76 (t, J=9.0 Hz, 2H), 4.70 (d, J=6.0 Hz, 2H), 4.42 - 4.36 (m, 1H), 4.15 - 4.08 (m, 1H), 3.46 - 3.35 (m, 2H), 3.05 (t, J=9.0 Hz, 2H), 2.72 - 2.65 (m, 2H), 2.23 (m, 2H). | Rt = 2.83 min, m/z 483.3 $[M+H]^+$ (Method 2) |
| 27 | 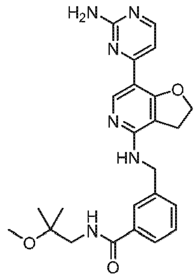 3-(((7-(2-Aminopyrimidin-4-yl)-2,3-dihydrofuro[3,2-c] pyridin-4-yl)amino) methyl)-N-(2-methoxy-2-methylpropyl)benzamide | 2-Methoxy-2-methylpropylam ine | (400 MHz, d6-DMSO) δ 8.67 (s, 1H), 8.19-8.14 (m, 2H), 7.85 (s, 1H), 7.73-7.69 (m, 1H), 7.51-7.46 (m, 1H), 7.39 (t, J = 7.1Hz, 1H), 7.17 (t, J = 5.9Hz, 1H), 7.07 (d, J = 5.3Hz, 1H), 6.42 (s, 2H), 4.75 (t, J = 9.0Hz, 2H), 4.69 (d, J = 5.8Hz, 2H), 3.16 (s, 3H), 3.05 (t, J = 8.9Hz, 2H), 1.11 (s, 6H). | Rt = 2.44 min, m/z 449.0 $[M+H]^+$ (Method 4) |
| 28 | 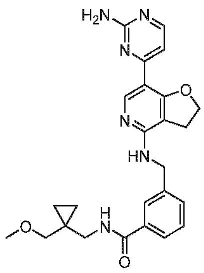 3-(((7-(2-Aminopyrimidin-4-yl)-2,3-dihydrofuro[3,2-c] pyridin-4-yl)amino) methyl)-N-((1-(methoxymethyl)cycloprop yl)methyl) benzamide | (1-(Methoxymethyl ) cyclopropyl)me thanamine | (400 MHz, d6-DMSO) δ 8.68 (s, 1H), 8.33 (t, J=5.9 Hz, 1H), 8.16 (d, J=5.3 Hz, 1H), 7.84 (s, 1H), 7.69 (ddd, J=1.4, 1.4, 7.8 Hz, 1H), 7.49 (ddd, J=1.3, 1.3, 7.7 Hz, 1H), 7.39 (dd, J=7.5, 7.5 Hz, 1H), 7.18 (t, J=5.9 Hz, 1H), 7.07 (d, J=4.9 Hz, 1H), 6.42 (s, 2H), 4.74 (t, J=8.9 Hz, 2H), 4.69 (d, J=5.9 Hz, 2H), 3.31 (d, J=5.9 Hz, 2H), 3.25 (s, 3H), 3.24 (s, 2H), 3.05 (t, J=9.0 Hz, 2H), 0.46 (m, 4H). | Rt = 2.52 min, m/z 461.0 $[M+H]^+$ (Method 4) |

(continued)

| Example | Structure | Amine | 1H NMR | LC-MS |
|---|---|---|---|---|
| 29 | 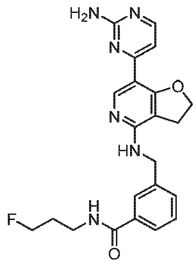 3-(((7-(2-Aminopyrimidin-4-yl)-2,3-dihydrofuro[3,2-c] pyridin-4-yl)amino) methyl)-N-(3-fluoropropyl) benzamide | 3-Fluoropropan-1-amine | (400 MHz, d6-DMSO) δ 8.68 (s, 1H), 8.53 (t, J=5.3 Hz, 1H), 8.16 (d, J=5.5 Hz, 1H), 7.85 (t, J=1.4, Hz, 1H), 7.70 (ddd, J=1.4, 1.4, 7.6 Hz, 1H), 7.49 (ddd, J=1.4, 1.4, 7.6 Hz, 1H), 7.39 (t, J=7.7, Hz, 1H), 7.18 (t, J=6.0 Hz, 1H), 7.07 (d, J=5.3 Hz, 1H), 6.43 (s, 2H), 4.75 (t, J=8.8 Hz, 2H), 4.69 (d, J=6.5 Hz, 2H), 4.52 (td, J=5.8, 47.5 Hz, 2H), 3.40 - 3.36 (m, 2H), 3.05 (t, J=9.1 Hz, 2H), 1.98 - 1.84 (m, 2H). | Rt = 2.27 min, m/z 423.0 $[M+H]^+$ (Method 4) |
| 30 | 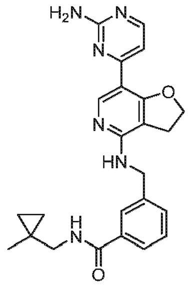 3-(((7-(2-Aminopyrimidin-4-yl)-2,3-dihydrofuro[3,2-c] pyridin-4-yl)amino) methyl)-N-((1-methylcyclopropyl)methyl )benzamide | (1-Methylcyclopro pyl) methanamin e | (400 MHz, d6-DMSO) δ 8.44 (s, 1H), 8.18 (t, J=6.1 Hz, 1H), 7.93 (d, J=5.2 Hz, 1H), 7.62 (t, J=1.5 Hz, 1H), 7.47 (ddd, J=1.4, 1.4, 7.7 Hz, 1H), 7.25 (ddd, J=1.4, 1.4, 7.7 Hz, 1H), 7.16 (t, J=7.6 Hz, 1H), 6.94 (t, J=6.1 Hz, 1H), 6.84 (d, J=5.5 Hz, 1H), 6.18 (s, 2H), 4.52 (t, J=9.1 Hz, 2H), 4.46 (d, J=6.3 Hz, 2H), 2.96 (d, J=6.3 Hz, 2H), 2.81 (t, J=9.1 Hz, 2H), 0.83 (s, 3H), 0.13 (m, 4H). | Rt = 2.81 min, m/z 431.0 $[M+H]^+$ (Method 4) |
| 31 | 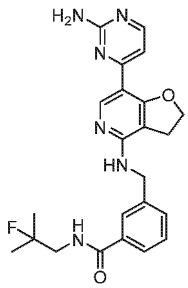 3-(((7-(2-Aminopyrimidin-4-yl)-2,3-dihydrofuro[3,2-c] pyridin-4-yl)amino) methyl)-N-(2-fluoro-2-methylpropyl)benzamide | 2-Fluoro-2-methylpropan-1-amine | (400 MHz, d6-DMSO) δ 8.68 (s, 1H), 8.58 (t, J=5.6 Hz, 1H), 8.16 (d, J=5.6 Hz, 1H), 7.88 (t, J=1.7 Hz, 1H), 7.74 (ddd, J=1.4, 1.4, 7.9 Hz, 1H), 7.50 (ddd, J=1.4, 1.4, 7.6 Hz, 1H), 7.40 (t, J=7.7 Hz, 1H), 7.18 (t, J=6.2 Hz, 1H), 7.07 (d, J=5.3 Hz, 1H), 6.42 (s, 2H), 4.75 (t, J=9.0 Hz, 2H), 4.70 (d, J=6.0 Hz, 2H), 3.48 (dd, J=6.2, 20.0 Hz, 2H), 3.05 (t, J=9.0 Hz, 2H), 1.33 (d, J=22.1 Hz, 6H). | Rt = 2.50 min, m/z 437.0 $[M+H]^+$ (Method 4) |

(continued)

| Example | Structure | Amine | 1H NMR | LC-MS |
|---|---|---|---|---|
| 32 | 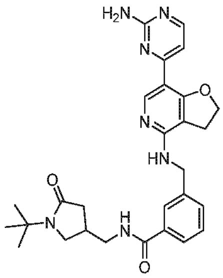 3-(((7-(2-Aminopyrimidin-4-yl)-2,3-dihydrofuro[3,2-c] pyridin-4-yl)amino) methyl)-N-((1-(tert-butyl)-5-oxopyrrolidin-3-yl)methyl) benzamide (Enantiomer 1) | 4-(Aminomethyl)-1-(tert-butyl)pyrrolidin-2-one | $^1$H NMR (400 MHz, d6-DMSO) δ 8.68 (s, 1H), 8.59 (t, J=5.6 Hz, 1H), 8.16 (d, J=5.3 Hz, 1H), 7.84 (s, 1H), 7.71 - 7.67 (m, 1H), 7.52 - 7.48 (m, 1H), 7.40 (t, J=7.7 Hz, 1H), 7.18 (t, J=5.9 Hz, 1H), 7.07 (d, J=5.3 Hz, 1H), 6.42 (s, 2H), 4.75 (t, J=8.9 Hz, 2H), 4.69 (d, J=6.0 Hz, 2H), 3.51 (dd, J=7.4, 9.8 Hz, 1H), 3.27 - 3.22 (m, 3H), 3.08 - 3.01 (m, 2H), 2.71 - 2.67 (m, 1H), 2.36 - 2.33 (m, 1H), 2.05 (dd, J=5.4, 16.3 Hz, 1H), 1.31 - 1.31 (m, 9H). | Rt = 3.86 min, m/z 516.5 $[M+H]^+$ (Method 5) |
| 33 | 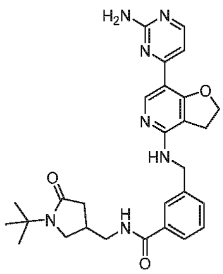 3-(((7-(2-Aminopyrimidin-4-yl)-2,3-dihydrofuro[3,2-c] pyridin-4-yl)amino) methyl)-N-((1-(tert-butyl)-5-oxopyrrolidin-3-yl)methyl) benzamide (Enantiomer 2) | 4-(Aminomethyl)-1-(tert-butyl)pyrrolidin-2-one | $^1$H NMR (400 MHz, d6-DMSO) δ 8.68 (s, 1H), 8.62 - 8.57 (m, 1H), 8.16 (d, J=5.3 Hz, 1H), 7.85 - 7.83 (m, 1H), 7.69 (d, J=7.7 Hz, 1H), 7.50 (d, J=7.7 Hz, 1H), 7.40 (t, J=7.6 Hz, 1H), 7.18 (t, J=6.0 Hz, 1H), 7.07 (d, J=5.3 Hz, 1H), 6.42 (s, 2H), 4.75 (t, J=8.9 Hz, 2H), 4.69 (d, J=5.9 Hz, 2H), 3.51 (dd, J=7.5, 9.7 Hz, 1H), 3.28 - 3.22 (m, 3H), 3.05 (t, J=9.0 Hz, 2H), 2.40 - 2.32 (m, 2H), 2.05 (dd, J=5.0, 16.3 Hz, 1H), 1.31 - 1.30 (m, 9H). | Rt = 3.86 min, m/z 516.3 $[M+H]^+$ (Method 5) |
| 34 | 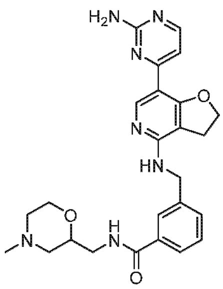 3-(((7-(2-Aminopyrimidin-4-yl)-2,3-dihydrofuro[3,2-c] pyridin-4-yl)amino) methyl)-N-((4-methylmorpholin-2-yl)methyl)benzamide (Enantiomer 1) | (4-Methylmorpholin-2-yl)methanamine | $^1$H NMR (400 MHz, d6-DMSO) δ 8.67 (s, 1H), 8.48 (t, J=5.8 Hz, 1H), 8.15 (d, J=5.3 Hz, 1H), 7.84 (s, 1H), 7.71 - 7.67 (m, 1H), 7.50 - 7.46 (m, 1H), 7.38 (t, J=7.6 Hz, 1H), 7.16 (t, J=6.1 Hz, 1H), 7.05 (d, J=5.3 Hz, 1H), 6.42 - 6.39 (m, 2H), 4.77 - 4.65 (m, 4H), 3.79 - 3.74 (m, 1H), 3.61 - 3.53 (m, 1H), 3.51 - 3.43 (m, 1H), 3.30 - 3.22 (m, 2H), 3.06 - 3.00 (m, 2H), 2.72 - 2.67 (m, 1H), 2.56 (d, J=11.6 Hz, 1H), 2.15 (s, 3H), 1.98 - 1.90 (m, 1H), 1.70 (dd, J=10.1, 11.1 Hz, 1H). | Rt = 2.89 min, m/z 476.1 $[M+H]^+$ (Method 7) |

(continued)

| Example | Structure | Amine | 1H NMR | LC-MS |
|---|---|---|---|---|
| 35 | 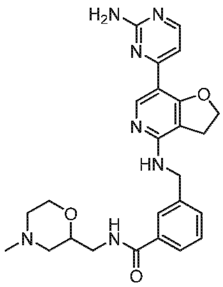 3-(((7-(2-Aminopyrimidin-4-yl)-2,3-dihydrofuro[3,2-c] pyridin-4-yl)amino) methyl)-N-((4-methylmorpholin-2-yl)methyl)benzamide (Enantiomer 2) | (4-Methylmorpholin-2-yl)methanamine | $^1$H NMR (400 MHz, d6-DMSO) δ 8.68 (s, 1H), 8.50 (t, J=5.8 Hz, 1H), 8.17 (d, J=5.3 Hz, 1H), 7.86 (s, 1H), 7.71 (d, J=7.8 Hz, 1H), 7.50 (d, J=7.8 Hz, 1H), 7.42 - 7.37 (m, 1H), 7.18 (t, J=6.1 Hz, 1H), 7.07 (d, J=5.3 Hz, 1H), 6.42 (s, 2H), 4.76 (t, J=8.8 Hz, 2H), 4.70 (d, J=6.0 Hz, 2H), 3.78 (qd, J=1.6, 11.1 Hz, 1H), 3.63 - 3.55 (m, 1H), 3.48 (dt, J=2.4, 11.2 Hz, 1H), 3.32 - 3.25 (m, 3H), 3.05 (t, J=8.9 Hz, 2H), 2.73 - 2.68 (m, 1H), 2.17 (s, 3H), 1.96 (dt, J=3.3, 11.3 Hz, 1H), 1.72 (dd, J=10.0, 11.1 Hz, 1H). | Rt = 1.79 min, m/z 476.2 [M+H]$^+$ (Method 4) |
| 36 | 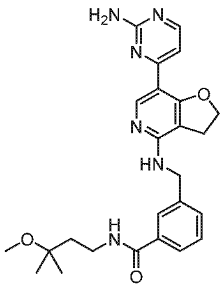 3-(((7-(2-Aminopyrimidin-4-yl)-2,3-dihydrofuro[3,2-c] pyridin-4-yl)amino) methyl)-N-(3-methoxy-3-methylbutyl)benzamide | 3-methoxy-3-methyl-butan-1-amine | $^1$H NMR (400 MHz, d6-DMSO) δ 8.68 (s, 1H), 8.39 (dd, J=5.5, 5.5 Hz, 1H), 8.16 (d, J=5.3 Hz, 1H), 7.82 (s, 1H), 7.67 (d, J=7.8 Hz, 1H), 7.48 (d, J=7.9 Hz, 1H), 7.41 -7.36 (m, 1H), 7.17 (t, J=6.0 Hz, 1H), 7.07 (d, J=5.3 Hz, 1H), 6.42 (s, 2H), 4.75 (t, J=9.1 Hz, 2H), 4.68 (d, J=6.2 Hz, 2H), 3.30 - 3.25 (m, 2H), 3.13 (s, 3H), 3.04 (t, J=9.0 Hz, 2H), 1.73 - 1.67 (m, 2H), 1.14 (s, 6H). | Rt = 2.58 min, m/z 463.0 [M+H]$^+$ (Method 4) |
| 37 | 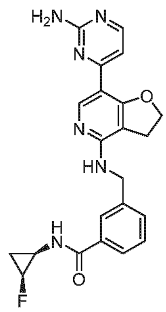 3-(((7-(2-Aminopyrimidin-4-yl)-2,3-dihydrofuro[3,2-c] pyridin-4-yl)amino) methyl)-N-((1R,2S)-2-fluorocyclopropyl)benzami de | (1R,2S)-2-Fluorocycloprop an-1-amine. TsOH | $^1$H NMR (400 MHz, DMSO) δ 8.68 (s, 1H), 8.54 (d, J=3.8 Hz, 1H), 8.16 (d, J=5.3 Hz, 1H), 7.86 (s, 1H), 7.71 (d, J=7.7 Hz, 1H), 7.51 (d, J=7.7 Hz, 1H), 7.40 (t, J=7.7, 1H), 7.18 (t, J=6.1Hz, 1H), 7.07 (d, J=5.3 Hz, 1H), 6.42 (s, 2H), 4.86 - 4.82 (m, 1H), 4.75 (t, J=8.6 Hz, 2H), 4.71 - 4.65 (m, 2H), 3.04 (t, J=8.8 Hz, 2H), 2.86 - 2.79 (m, 1H), 1.23 - 1.08 (m, 2H). | Rt = 2.26 min, m/z 421.0 [M+H]$^+$ (Method 4) |

(continued)

| Example | Structure | Amine | 1H NMR | LC-MS |
|---|---|---|---|---|
| 38 | 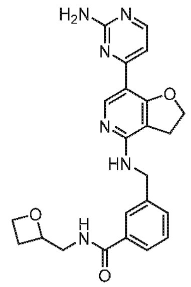 3-(((7-(2-Aminopyrimidin-4-yl)-2,3-dihydrofuro[3,2-c] pyridin-4-yl)amino) methyl)-N-(oxetan-2-yl-methyl)benzamide | Oxetan-2-yl-ylmetha-namine | $^1$H NMR (400 MHz, DMSO) δ 8.68 (s, 1H), 8.61 (t, J=5.7 Hz, 1H), 8.16 (d, J=5.3 Hz, 1H), 7.87 (s, 1H), 7.73 (d, J=7.7 Hz, 1H), 7.50 (d, J=7.9 Hz, 1H), 7.40 (t, J=7.7 Hz, 1H), 7.17 (t, J=6.1 Hz, 1H), 7.07 (d, J=5.3 Hz, 1H), 6.42 (s, 2H), 4.86 - 4.80 (m, 1H), 4.75 (t, J=9.1 Hz, 2H), 4.68 (d, J=6.3 Hz, 2H), 4.53 - 4.48 (m, 1H), 4.46 - 4.39 (m, 1H), 3.60 - 3.53 (m, 1H), 3.50 - 3.44 (m, 1H), 3.04 (t, J=8.8 Hz, 2H), 2.69 - 2.55 (m, 1H), 2.46 - 2.34 (m, 1H). | Rt = 2.17 min, m/z 433.0 $[M+H]^+$ (Method 4) |
| 39 | 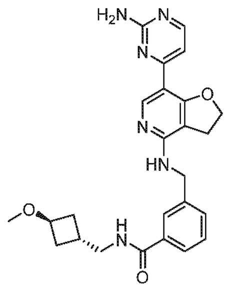 3-(((7-(2-Aminopyrimidin-4-yl)-2,3-dihydrofuro[3,2-c] pyridin-4-yl)amino) methyl)-N-(((1R,3R)-3-methoxycyclobutyl)methyl ) benzamide | [(1R, 3R)-3-Methoxy-cyclobu tyl]methana-mine .HCl | (400 MHz, DMSO) δ 8.68 (s, 1H), 8.49 (t, J = 5.6Hz, 1H), 8.16 (d, J = 5.5Hz, 1H), 7.84 (s, 1H), 7.71-7.67 (m, 1H), 7.51-7.46 (m, 1H), 7.39 (t, J = 7.4Hz, 1H), 7.17 (t, J = 6.0Hz, 1H), 7.07 (d, J = 5.7Hz, 1H), 6.42 (s, 2H), 4.75 (t, J = 8.9Hz, 2H), 4.69 (d, J = 6.0Hz, 2H), 3.99-3.90 (m, 1H), 3.36-3.29 (m, 2H), 3.11 (s, 3H), 3.04 (t, J = 9.0Hz, 2H), 2.46-2.32 (m, 1H), 2.08-1.99 (m, 2H), 1.98-1.88 (m, 2H). | Rt = 2.42 min, m/z 461.0 $[M+H]^+$ (Method 4) |
| 40 | 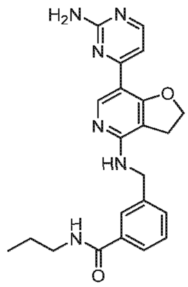 3-(((7-(2-Aminopyrimidin-4-yl)-2,3-dihydrofuro[3,2-c] pyridin-4-yl)amino) methyl)-N-propylbenzamide | Propylamine | $^1$H NMR (400 MHz, DMSO) δ 8.68 (s, 1H), 8.43 (t, J=5.6 Hz, 1H), 8.16 (d, J=5.3 Hz, 1H), 7.84 (s, 1H), 7.69 (d, J=7.8 Hz, 1H), 7.48 (d, J=7.8 Hz, 1H), 7.41 - 7.36 (m, 1H), 7.17 (t, J=6.1 Hz, 1H), 7.07 (d, J=5.3 Hz, 1H), 6.42 (s, 2H), 4.75 (t, J=8.9 Hz, 2H), 4.69 (d, J=5.7 Hz, 2H), 3.25 - 3.18 (m, 2H), 3.04 (t, J=9.0, 2H), 1.59 - 1.48 (m, 2H), 0.89 (t, J= 7.4 Hz, 3H). | Rt = 2.47 min, m/z 405.0 $[M+H]^+$ (Method 4) |

(continued)

| Example | Structure | Amine | 1H NMR | LC-MS |
|---|---|---|---|---|
| 41 | 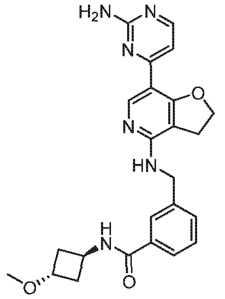 3-(((7-(2-Aminopyrimidin-4-yl)-2,3-dihydrofuro[3,2-c] pyridin-4-yl)amino) methyl)-N-((1R,3R)-3-methoxycyclobutyl)benza mide | trans-3-Methoxycyclobu tan-1-amine | $^1$H NMR (400 MHz, DMSO) δ 8.68 (s, 1H), 8.64 (d, J=6.9 Hz, 1H), 8.17 (d, J=5.3 Hz, 1H), 7.84 (s, 1H), 7.71 (d, J=7.8 Hz, 1H), 7.49 (d, J=7.7 Hz, 1H), 7.42 - 7.37 (m, 1H), 7.18 (dd, J=6.1, 6.1 Hz, 1H), 7.07 (d, J=5.3 Hz, 1H), 6.43 (s, 2H), 4.75 (t, J=9.0 Hz, 2H), 4.69 (d, J=6.1 Hz, 2H), 4.47 - 4.40 (m, 1H), 4.05 - 3.98 (m, 1H), 3.17 (s, 3H), 3.05 (t, J=8.9 Hz, 2H), 2.29 - 2.24 (m, 4H). | Rt = 2.36 min, m/z 447.0 $[M+H]^+$ (Method 4) |
| 42 | 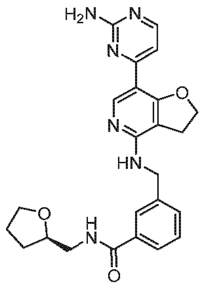 (R)-3-(((7-(2-Aminopyrimidin-4-yl)-2,3-dihydrofuro [3,2-c]pyridin-4-yl)amino) methyl)-N-((tetrahydrofuran-2-yl)methyl)benzamide | (R)-(-)-Tetrahydrofurfur ylamine | $^1$H NMR (400 MHz, DMSO) δ 8.68 (s, 1H), 8.49 (t, J=5.7 Hz, 1H), 8.17 (d, J=5.3 Hz, 1H), 7.85 (s, 1H), 7.71 (d, J=7.8 Hz, 1H), 7.49 (d, J=7.9 Hz, 1H), 7.42 - 7.37 (m, 1H), 7.18 (t, J=6.1 Hz, 1H), 7.07 (d, J=5.3 Hz, 1H), 6.42 (s, 2H), 4.76 (t, J=9.0 Hz, 2H), 4.69 (d, J=6.2 Hz, 2H), 4.02 - 3.94 (m, 1H), 3.81 - 3.75 (m, 1H), 3.69 - 3.60 (m, 1H), 3.33 - 3.25 (m, 2H), 3.05 (t, J=9.0 Hz, 2H), 1.93 - 1.77 (m, 3H), 1.64 - 1.55 (m, 1H). | Rt = 2.35 min, m/z 447.0 $[M+H]^+$ (Method 4) |
| 43 | 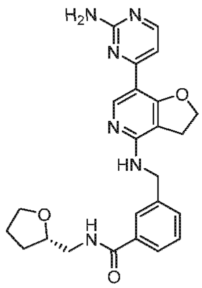 (S)-3-(((7-(2-Aminopyrimidin-4-yl)-2,3-dihydrofuro [3,2-c]pyridin-4-yl)amino) methyl)-N-((tetrahydrofuran-2-yl)methyl)benzamide | (S)-(-)-Tetrahydrofurfur ylamine | $^1$H NMR (400 MHz, DMSO) δ 8.68 (s, 1H), 8.49 (t, J=5.7 Hz, 1H), 8.17 (d, J=5.3 Hz, 1H), 7.85 (s, 1H), 7.71 (d, J=7.8 Hz, 1H), 7.49 (d, J=7.9 Hz, 1H), 7.42 - 7.37 (m, 1H), 7.18 (t, J=6.1 Hz, 1H), 7.07 (d, J=5.3 Hz, 1H), 6.42 (s, 2H), 4.76 (t, J=9.0 Hz, 2H), 4.69 (d, J=6.2 Hz, 2H), 4.02 - 3.94 (m, 1H), 3.81 - 3.75 (m, 1H), 3.69 - 3.60 (m, 1H), 3.33 - 3.25 (m, 2H), 3.05 (t, J=9.0 Hz, 2H), 1.93 - 1.77 (m, 3H), 1.64 - 1.55 (m, 1H). | Rt = 2.35 min, m/z 447.0 $[M+H]^+$ (Method 4) |

(continued)

| Example | Structure | Amine | 1H NMR | LC-MS |
|---|---|---|---|---|
| 44 | 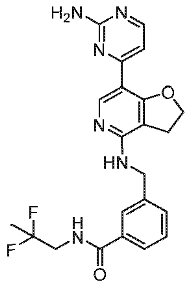 3-(((7-(2-Aminopyrimidin-4-yl)-2,3-dihydrofuro[3,2-c] pyridin-4-yl)amino) methyl)-N-(2,2-difluoropropyl)benzamide | 2,2-Difluoropropan-1-amine | (400 MHz, DMSO) δ 8.81 (t, J = 6.2Hz, 1H), 8.68 (s, 1H), 8.16 (d, J = 8.2Hz, 1H), 7.89 (s, 1H), 7.75 (d, J = 7.9Hz, 1H), 7.52 (d, J = 7.9Hz, 1H), 7.42 (t, J = 7.5Hz, 1H), 7.15 (t, J= 5.8Hz, 1H), 7.07 (d, J = 5.4Hz, 1H), 6.42 (s, 2H), 4.75 (t, J = 8.8Hz, 2H), 4.70 (d, J = 6.0Hz, 2H), 3.79-3.66 (m, 2H), 3.04 (t, J = 8.7Hz, 2H), 1.62 (t, J = 18.7Hz, 3H). | Rt = 2.51 min, m/z 441.0 $[M+H]^+$ (Method 4) |
| 45 | 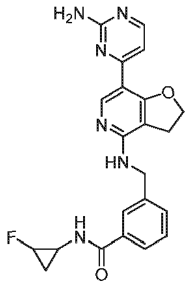 Trans 3-(((7-(2-Aminopyrimidin-4-yl)-2,3-dihydrofuro [3,2-c]pyridin-4-yl)amino) methyl)-N-(2-fluorocyclopropyl)benzami de (Enantiomer 1) | trans-2-Fluorocyclo-prop anamine 4-methylbenzenes ulfo-nate | $^1$H NMR (400 MHz, DMSO) δ 8.67 (s, 1H), 8.47 (s, 1H), 8.16 (d, J=5.3 Hz, 1H), 7.82 (s, 1H), 7.65 (d, J=7.8 Hz, 1H), 7.50 (d, J=7.9 Hz, 1H), 7.42 - 7.37 (m, 1H), 7.19 (t, J=6.0 Hz, 1H), 7.07 (d, J=5.3 Hz, 1H), 6.44 (s, 2H), 4.76 (t, J=8.8 Hz, 2H), 4.83 - 4.66 (m, 1H), 4.68 (d, J=5.7 Hz, 2H), 3.23 - 3.15 (m, 1H), 3.04 (t, J=8.9 Hz, 2H), 1.44 - 1.32 (m, 1H), 1.10 - 1.01 (m, 1H). | Rt = 2.34 min, m/z 421.0 $[M+H]^+$ (Method 4) first eluting |
| 46 | 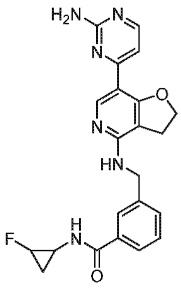 Trans 3-(((7-(2-Aminopyrimidin-4-yl)-2,3-dihydrofuro [3,2-c]pyridin-4-yl)amino) methyl)-N-(2-fluorocyclopropyl)benzami de (Enantiomer 2) | trans-2-Fluorocyclo-prop anamine 4-methylbenzenes ulfo-nate | $^1$H NMR (400 MHz, DMSO) δ 8.67 (s, 1H), 8.47 (s, 1H), 8.16 (d, J=5.3 Hz, 1H), 7.82 (s, 1H), 7.65 (d, J=7.8 Hz, 1H), 7.50 (d, J=7.9 Hz, 1H), 7.42 - 7.37 (m, 1H), 7.19 (t, J=6.0 Hz, 1H), 7.07 (d, J=5.3 Hz, 1H), 6.44 (s, 2H), 4.76 (t, J=8.8 Hz, 2H), 4.83 - 4.66 (m, 1H), 4.68 (d, J=5.7 Hz, 2H), 3.23 - 3.15 (m, 1H), 3.04 (t, J=8.9 Hz, 2H), 1.44 - 1.32 (m, 1H), 1.10 - 1.01 (m, 1H). | Rt = 2.35 min, m/z 421.0 $[M+H]^+$ (Method 4) Second eluting |

(continued)

| Example | Structure | Amine | 1H NMR | LC-MS |
|---|---|---|---|---|
| 47 | 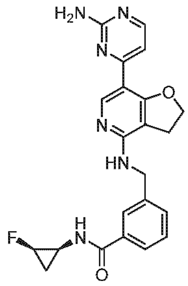 3-(((7-(2-Aminopyrimidin-4-yl)-2,3-dihydrofuro[3,2-c] pyridin-4-yl)amino) methyl)-N-((1S,2R)-2-fluorocyclopropyl)benzami de | (1S,2R)-2-Fluorocycloprop an-1-amine | $^1$H NMR (400 MHz, DMSO) δ 8.68 - 8.67 (m, 1H), 8.57 - 8.54 (m, 1H), 8.16 (d, J=5.3 Hz, 1H), 7.86 (s, 1H), 7.71 (d, J=7.7 Hz, 1H), 7.52 - 7.49 (m, 1H), 7.40 (t, J=7.7 Hz, 1H), 7.20 (t, J=6.1 Hz, 1H), 7.07 (d, J=5.3 Hz, 1H), 6.44 (s, 2H), 4.80 (m, 1H), 4.78 - 4.66 (m, 4H), 3.08 - 3.01 (m, 2H), 2.87 - 2.79 (m, 1H), 1.23 - 1.06 (m, 2H). | Rt = 2.27 min, m/z 421 $[M+H]^+$ (Method 4) |
| 48 | 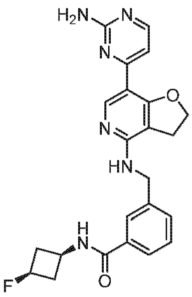 3-(((7-(2-Aminopyrimidin-4-yl)-2,3-dihydrofuro[3,2-c] pyridin-4-yl)amino) methyl)-N-((1S,3S)-3-fluorocyclobutyl)benzamid e | cis-Fluorocyclobutan-1-amine.HCl | $^1$H NMR (400 MHz, DMSO) δ 8.70 - 8.66 (m, 2H), 8.16 (d, J=5.3 Hz, 1H), 7.85 (s, 1H), 7.71 (d, J=7.8 Hz, 1H), 7.50 (d, J=7.8 Hz, 1H), 7.40 (t, J=7.7 Hz, 1H), 7.18 (t, J=6.1 Hz, 1H), 7.07 (d, J=5.3 Hz, 1H), 6.42 (s, 2H), 4.98- 4.79 (m, 1H), 4.74 (t, J=8.7 Hz, 2H), 4.69 (d, J=6.3 Hz, 2H), 4.03 - 3.96 (m, 1H), 3.04 (t, J=8.9 Hz, 2H), 2.78 - 2.68 (m, 2H), 2.36 - 2.24 (m, 2H). | Rt = 2.5 min, m/z 435 $[M+H]^+$ (Method 4) |
| 49 | 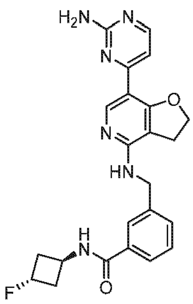 3-(((7-(2-Aminopyrimidin-4-yl)-2,3-dihydrofuro[3,2-c] pyridin-4-yl)amino) methyl)-N-((1r,3r)-3-fluorocyclobutyl)benzamid e | trans-Fluorocyclobutan-1-amine.HCl | $^1$H NMR (400 MHz, DMSO) δ 8.72 - 8.67 (m, 2H), 8.22 (d, J=5.8 Hz, 1H), 7.84 (s, 1H), 7.73 (d, J=7.7 Hz, 1H), 7.51 (d, J=7.8 Hz, 1H), 7.43 (t, J=7.7 Hz, 1H), 7.20 (d, J=5.8 Hz, 1H), 7.09 (s, 1H), 5.37 - 5.21 (m, 1H), 4.82 (t, J=8.8 Hz, 2H), 4.72 (d, J=5.9 Hz, 2H), 4.59 - 4.52 (m, 1H), 3.09 (t, J=8.9 Hz, 2H), 2.48 - 2.43 (m, 4H). | Rt = 2.48 min, m/z 435 $[M+H]^+$ (Method 4) |

(continued)

| Example | Structure | Amine | 1H NMR | LC-MS |
|---|---|---|---|---|
| 50 | 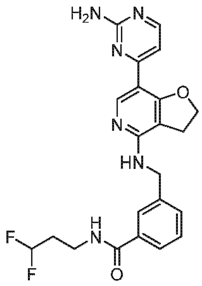 3-(((7-(2-Aminopyrimidin-4-yl)-2,3-dihydrofuro[3,2-c]pyridin-4-yl)amino)methyl)-N-(3,3-difluoropropyl)benzamide | 3,3-Difluoropropan-1-amine | $^1$H NMR (400 MHz, DMSO) δ 8.68 (s, 1H), 8.57 (t, J=5.6 Hz, 1H), 8.16 (d, J=5.3 Hz, 1H), 7.84 (s, 1H), 7.69 (d, J=7.7 Hz, 1H), 7.50 (d, J=7.9 Hz, 1H), 7.40 (t, J=7.7 Hz, 1H), 7.18 (t, J=6.1 Hz, 1H), 7.07 (d, J=5.3 Hz, 1H), 6.42 (s, 2H), 6.16 (tt, J=4.4, 56.4 Hz, 1H), 4.75 (t, J=8.4 Hz, 2H), 4.69 (d, J=6.9 Hz, 2H), 3.44 - 3.38 (m, 2H), 3.04 (t, J=8.9 Hz, 2H), 2.18 - 2.04 (m, 2H). | Rt = 2.47 min, m/z 441 $[M+H]^+$ (Method 4) |

The following example were prepared from their racemic mixtures by SFC using the methods described below.

| **Racemate** | **SFC conditions used for purification** | **Enantiomer 1** | **Enantiomer 2** |
|---|---|---|---|
| Example 32+33 | YMC Amylose-C 4.6x250mm, 5um 55/45 MeOH (0.1% DEA) / $CO_2$, 100mL/min, 120bar, 40°C | Example 32<br>Rt = 5.9 min (YMC AMYLOSE-C + 0.1% DEA iPrOH 55% MeOH) | Example 33<br>Rt = 10.8 min (YMC AMYLOSE-C + 0.1% DEA iPrOH 55% MeOH) |
| Example 34+35 | YMC Amylose-C 4.6x250mm, 5um 55/45 MeOH (0.1% DEA) / $CO_2$, 5.0mL/min, 120bar, 40°C | Example 34<br>Rt = 6.3 min (Cellulose-C Basic MeOH 20% +0.1% DEA 80% $CO_2$) | Example 35<br>Rt = 9.5 min (Cellulose-C Basic MeOH 20% +0.1% DEA 80% $CO_2$) |
| Example 45+46 | LUX Cellulose-4 10x250mm, 5um 45/55 MeOH (0.1% DEA) / $CO_2$, 15mL/min, 120bar, 40°C | Example 45<br>Rt = 4.62 min (LUX cellulose basic 0.1% DEA iPrOH 45% MeOH) | Example 46<br>Rt = 5.75 min (LUX cellulose basic 0.1% DEA iPrOH 45% MeOH) |

*Synthesis of the amine required for the preparation of Example 6.*

**Intermediate 6A**

***Step A***

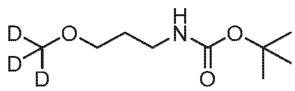

**tert-Butyl (3-(methoxy-d3)propyl)carbamate (Intermediate 6A)**

tert-Butyl (3-hydroxypropyl)carbamate (0.15 mL, 0856 mmol) was added to a stirred mixture of 0.1M NaOH (1 mL) and toluene (1.5 mL). Tetrabutylammonium bromide (138 mg, 0.428 mmol) was added and the reaction mixture was stirred until dissolution had occurred. Iodomethane-$d_3$ (0.11 mL, 1.71 mmol) was added and the reaction mixture was stirred at RT for 48 h. The reaction mixture was partitioned between water (10 mL) and diethyl ether (10 mL). The aqueous layer was extracted with diethyl ether (10 mL), the combined organic layer was dried over $Na_2SO_4$ and concentrated *in*

*vacuo.* The residue was purified by flash chromatography on silica eluting with 0-50% EtOAc in cyclohexane. The relevant fractions were evaporated to give the product (150 mg).

$^1$H NMR (400 MHz, d6-DMSO) δ 6.84 - 6.69 (m, 1H), 3.29 (t, J=6.3 Hz, 2H), 2.94 (q, J=6.6 Hz, 2H), 1.63 - 1.54 (m, 2H), 1.37 (s, 9H).

***Step B***

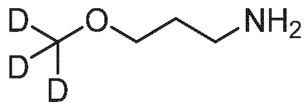

**3-(Methoxy-*d*$_3$)propan-1-amine (Intermediate 6B)**

To a stirred solution of Intermediate 6A (150 mg, 0.78 mmol) in DCM (3 mL) was added TFA (0.3 mL, 3.9 mmol) and the reaction mixture was stirred at RT for 18 h. The reaction mixture was concentrated *in vacuo,* the residue was diluted with water and was passed down an SCX-2 cartridge eluting with MeOH and then 2M methanolic ammonia. The relevant fractions were evaporated to give the desired product.

$^1$H NMR (400 MHz, d6-DMSO) δ (t, J=6.4 Hz, 2H), 3.17 (s, 2H), 2.56 (t, J=6.8 Hz, 2H), 1.58 - 1.51 (m, 2H).

**Example 51**

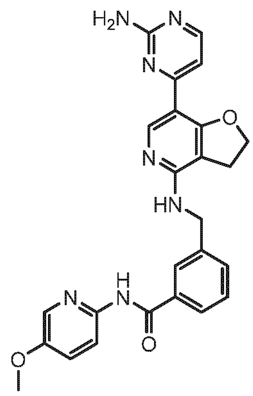

**3-(((7-(2-Aminopyrimidin-4-yl)-2,3-dihydrofuro[3,2-c]pyridin-4-yl)amino)methyl)-N-(5-methoxypyridin-2-yl)benzamide (Example 51)**

To a solution of Intermediate 1J (94 mg, 0.259 mmol) and 1-(methylsulfonyl)-1H-benzotriazole (204 mg, 1.03 mmol) dissolved in 2-methyltetrahydrofuran (4 mL) were added triethylamine (0.22 mL) and 2-amino-5-methoxypyridine (64 mg, 0.517 mmol) and the reaction mixture was flushed with argon. The reaction was heated to 150°C under microwave irradiation for 3 hours. Further triethylamine (0.11 mL) and 1-(methylsulfonyl)-1H-benzotriazole (105 mg, 0.570 mmol) were added and the reaction was heated to 150°C for a further 4 hours. The reaction was stirred at 175°C under microwave irradiation for a further 2 hours. The reaction mixture was purified using an SCX-2 column, eluting with methanol followed by 2N methanolic ammonia. The relevant fractions were combined and concentrated *in vacuo.* The resulting residue was dissolved in DCM and purified using flash chromatography on silica gel by eluting with 0-100% DCM in ethyl acetate, followed by 20% methanol in ethyl acetate. The relevant fractions were combined and concentrated *in vacuo.* The resulting residue was dissolved in 1:1 acetonitrile water and freeze dried. The resulting solid was purified by MDAP (Sunfire C18 19x150mm, 10um 5-60% acetonitrile/$H_2O$ (0.1% FA), 20 mL/min RT) to give the desired product (12.7 mg).

LCMS (Method 4): Rt = 2.61 min, m/z 470.0 $(M+H)^+$

$^1$H NMR (400 MHz, d6-DMSO) δ 10.62 (br s, 1H), 8.69 (br s, 1H), 8.16 (d, J=5.2 Hz, 1H), 8.12 (s, 1H), 8.10 (d, J=6.7 Hz, 1H), 8.01 (s, 1H), 7.89 (s, J=7.8 Hz, 1H), 7.55 (d, J=7.5 Hz, 1H), 7.49 (dd, J=3.2, 9.0 Hz, 1H), 7.44 (t, J=7.7 Hz, 1H), 7.19

(t, J=6.1 Hz, 1H), 7.07 (d, J=5.3, 1H), 6.43 (br s, 2H), 4.70-4.79 (m, 4H), 3.85 (s, 3H), 3.06 (t, J=8.8 Hz, 2H).

**Example 52 to 53**

The following examples were prepared from intermediate 1J and the given amine using a method similar to that used for *example 51.* For those examples that use an *amino heterocycle* containing a Boc protected amino moiety, the Boc group was removed after amide coupling using similar reaction conditions to that described in *step J* for the preparation of *intermediate 1J.*

| Example | Structure | Amino heterocycle | 1H NMR | LC-MS |
|---|---|---|---|---|
| 52 | 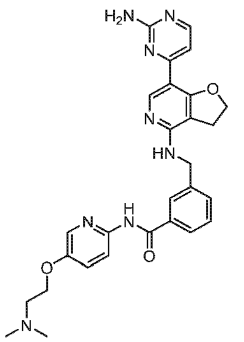 3-(((7-(2-Aminopyrimidin-4-yl)-2,3-dihydrofuro[3,2-c]pyridin-4-yl)amino) methyl)-N-(5-(2-(dimethylamino) ethoxy)py ridin-2-yl)benzamide | 5-(2-(Dimethylamin o)ethoxy)pyrid in-2-amine | (400 MHz, d6-DMSO) δ 10.61 (s, 1H), 8.69 (s, 1H), 8.16 (d, J=5.0 Hz, 1H), 8.12 - 8.07 (m, 2H), 8.01 (t, J=1.6 Hz, 1H), 7.89 (ddd, J=1.5, 1.5, 7.8 Hz, 1H), 7.55 (ddd, J=1.3, 1.3, 7.5 Hz, 1H), 7.51 (dd, J=3.2, 9.0 Hz, 1H), 7.44 (t, 7.5 Hz, 1H), 7.19 (t, J=6.2 Hz, 1H), 7.07 (d, J=5.4 Hz, 1H), 6.43 (s, 2H), 4.78 - 4.70 (m, 4H), 4.14 (t, J=5.6 Hz, 2H), 3.05 (t, J=8.6 Hz, 2H), 2.65 (t, J=5.8 Hz, 2H), 2.23 (s, 6H). | Rt = 1.97 min, m/z 527.0 $[M+H]^+$ (Method 4) |
| 53 | 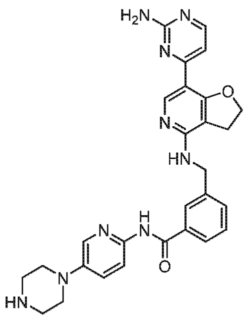 3-(((7-(2-aminopyrimidin-4-yl)-2,3-dihydrofuro[3,2-c]pyridin-4-yl)amino)methyl)-N-(5-(piperazin-1-yl)pyridin-2-yl)benzamide | tert-butyl 4-(6-aminopyridin-3-yl)piperazine-1-carboxylate | $^1$H NMR (400 MHz, d6-DMSO) d 10.50 - 10.48 (m, 1H), 8.69 (s, 1H), 8.16 (d, J=5.4 Hz, 1H), 8.06 (d, J=2.8 Hz, 1H), 8.01 (d, J=8.9 Hz, 2H), 7.90 - 7.87 (m, 1H), 7.54 (d, J=7.8 Hz, 1H), 7.46 - 7.41 (m, 2H), 7.19 (t, J=6.0 Hz, 1H), 7.07 (d, J=5.3 Hz, 1H), 6.43 (s, 2H), 4.78 - 4.70 (m, 4H), 3.10 - 3.02 (m, 6H), 2.88 - 2.84 (m, 4H). | Rt = 1.97 min, m/z 524.0 $[M+H]^+$ (Method 4) |

**Example 54**

***Step A***

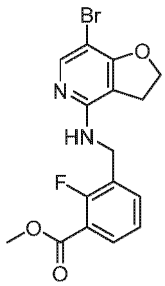

**Methyl 3-(((7-bromo-2,3-dihydrofuro[3,2-c]pyridin-4-yl)amino)methyl)-2-fluorobenzoate (Intermediate 54A)**

To a stirred solution of Intermediate 1D (0.40 g, 1.86 mmol), methyl 2-fluoro-3-formylbenzoate (0.58 g, 3.49 mmol) and molecular sieves 4Å in DCM (9 mL) was added chlorotriisopropoxytitanium(IV) (0.97 mg, 3.72 mmol) and the resulting suspension was allowed to stir at room temperature for 2 h. Then sodium triacetoxyborohydride (0.79 g, 3.72 mmol) and acetic acid (0.22 g, 0.21 mL, 3.72 mmol) were added and the resulting mixture was allowed to stir at RT for 24 h. The reaction was quenched with 1N NaOH (8 mL), diluted with DCM and filtered through diatomaceous earth. The filtrate was washed with water, dried with $Na_2SO_4$, filtered and concentrated *in vacuo.* Purification by flash chromatography on a silica gel cartridge eluting with 0-70% EtOAc in DCM gave the desired product (206 mg).
LCMS (Method 1): Rt = 1.24 min, m/z 380.9/382.9 $[M+H]^+$

***Step B***

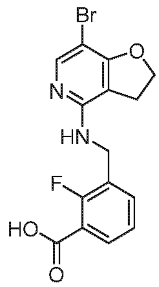

**3-(((7-Bromo-2,3-dihydrofuro[3,2-c]pyridin-4-yl)amino)methyl)-2-fluorobenzoic acid (Intermediate 54B)**

A solution of Intermediate 54A (206 mg, 0.54 mmol) and lithium hydroxide monohydrate (68 mg, 1.62 mmol) dissolved in MeOH (2 mL), THF (2 mL) and water (4 mL) was stirred at room temperature for 20 h. The solution was acidified to pH 5-6 using 1N HCl, then resulting mixture was extracted with 2-Me-THF (x4). The combined organic phases were dried ($Na_2SO_4$), filtered and concentrated *in vacuo* to give the title compound (198 mg).
LCMS (Method 3): Rt = 1.33 min, m/z 367/369 $[M+H]^+$

***Step C***

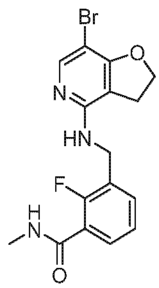

**3-(((7-Bromo-2,3-dihydrofuro[3,2-c]pyridin-4-yl)amino)methyl)-2-fluoro-N-methylbenzamide (Intermediate 54C)**

A solution of Intermediate 54B (198 mg, 0.54 mmol), TBTU (225 mg, 0.70 mmol) and DIPEA (0.56 mL, 3.24 mmol) in DCM (5 mL) was stirred at RT for 5 min. Methylamine hydrochloride (109 mg, 1.62 mmol) was added and the solution stirred at RT for 1 h. Water was added to the solution and the product was extracted three times with 2-Me-THF. The combined organic phase was dried with $Na_2SO_4$, filtered and concentrated *in vacuo.* The crude was purified by flash chromatography on a silica gel cartridge eluting with 0-10% MeOH in DCM to give the desired product (205 mg). LCMS (Method 1): Rt = 0.90 min, m/z 380/382 $[M+H]^+$

***Step D***

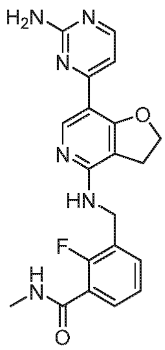

**3-(((7-(2-Aminopyrimidin-4-yl)-2,3-dihydrofuro[3,2-c]pyridin-4-yl)amino)methyl)-2-fluoro-N-methylbenzamide (Example 54)**

Intermediate 54C (205 mg, 0.54 mmol), Intermediate 1G (272 mg, 0.59 mmol), tetrakis(triphenylphosphine) palladium (31 mg, 0.027 mmol) and copper (I) thiophene-2-carboxylate (10 mg, 0.054 mmol) in dioxane (6 mL) was degassed then heated under microwave irradiation at 130°C for 1.5 h. The resulting mixture was diluted with EtOAc and filtered through diatomaceous earth, washed with saturated aqueous NaCl, dried with $Na_2SO_4$, filtered and concentrated *in vacuo.* The residue was dissolved in DCM (2 mL) and added TFA (2 mL), the solution stirred at RT for 3 h. The solution was concentrated *in vacuo* and the residue diluted with MeOH, was passed down on a SCX-2 cartridge eluting with MeOH and then 7N methanolic ammonia. The ammonia solution was concentrated *in vacuo* and product purified by MDAP (Sunfire C18 3x50mm, 3μm, 5-95% ACN / $H_2O$ (0.1% FA), 1.7mL/min, RT) to give the title product (26 mg).

LCMS (Method 4): Rt = 2.08 min, m/z 395.0 $[M+H]^+$
$^1H$ NMR (400 MHz, d6-DMSO) δ 8.66 (s, 1H), 8.28 (br s, 1H), 8.17 (d, J=5.4 Hz, 1H), 7.49 - 7.42 (m, 2H), 7.19 (dd, J=7.2, 7.2 Hz, 1H), 7.14 (t, J=5.8 Hz, 1H), 7.07 (d, J=5.4 Hz, 1H), 6.43 (s, 2H), 4.76 (t, J=8.5 Hz, 2H), 4.71 (d, J=6.4 Hz, 2H), 3.06 (t, J=8.8 Hz, 2H), 2.79 (d, J=4.6 Hz, 3H).

**Example 55**

***Step A***

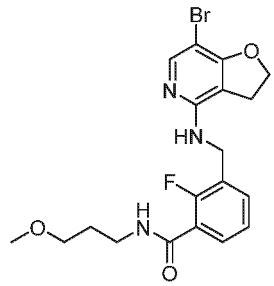

**3-(((7-bromo-2,3-dihydrofuro[3,2-c]pyridin-4-yl)amino)methyl)-2-fluoro-N-(3-methoxypropyl)benzamide (Intermediate 55A)**

To a stirred solution of Intermediate 54B (290 mg, 0.790 mmol), 3-methoxypropylamine (0.24 mL, 2.37 mmol) and TBTU (330 mg, 1.03 mmol) in DMF (6 mL) was added DIPEA (0.83 mL, 4.74 mmol) and the reaction mixture was stirred at RT overnight. Further amounts of 3-methoxypropylamine (0.08 mL, 0.79 mmol) and TBTU (254 mg, 0.79 mmol) were added and stirring continued for 12 h. The reaction mixture was diluted with EtOAc and washed with water then a 5% aqueous solution of LiCl. The organics were dried with $Na_2SO_4$, filtered and concentrated *in vacuo.* The crude material was purified by flash chromatography on a silica gel cartridge, eluting with 0-60% (10% 2M ammonia in MeOH in DCM) in DCM. The relevant fractions were combined and concentrated *in vacuo* to give the desired product (211 mg).
LCMS (Method 1): Rt = 1.06 min, m/z 438.1/440.1 $[M+H]^+$

***Step B***

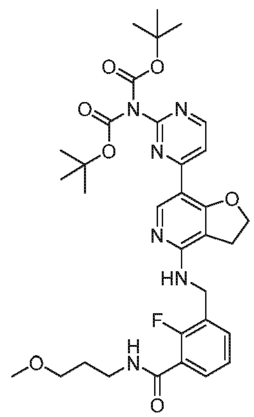

**tert-Butyl (tert-butoxycarbonyl)(4-(4-((2-fluoro-3-((3-methoxypropyl)-carbamoyl)benzyl)amino)-2,3-dihydrofuro[3,2-c]pyridin-7-yl)pyrimidin-2-yl)-carbamate (Intermediate 55B)**

To a degassed solution of Intermediate 55A (211 mg, 0.481 mmol) and Intermediate 1G (243 mg, 0.530 mmol) in dioxane (5 mL) were added copper (I) thiophene-2-carboxylate (9.2 mg, 0.048 mmol) and tetrakis (triphenylphosphine) palladium(0) (28 mg, 0.024 mmol) and the reaction was stirred at 130°C under microwave irradiation for 1.5 h. The reaction mixture was diluted with EtOAc and filtered through diatomaceous earth, washing with EtOAc. The filtrate was washed with water and brine then the organic layers were separated and dried with $Na_2SO_4$, filtered and concentrated *in vacuo.* The residue was purified by flash chromatography on a silica gel cartridge, eluting with 0-80% (10% 2M ammonia in MeOH in DCM) in DCM. The relevant fractions were combined and concentrated *in vacuo* to give the product (269 mg).
LCMS (Method 1): Rt = 1.28 min, m/z 653.3 $[M+H]^+$

***Step C***

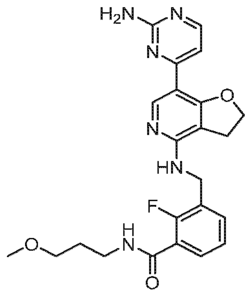

**3-(((7-(2-Aminopyrimidin-4-yl)-2,3-dihydrofuro[3,2-c]pyridin-4-yl)amino)methyl)-2-fluoro-N-(3-methoxypropyl) benzamide (Example 55)**

To a stirred solution of Intermediate 55B (269 mg, 0.0.247 mmol) in DCM (2.5 mL) was added TFA (2.5 mL) and the reaction mixture was stirred at RT for 2 h. The reaction mixture was concentrated *in vacuo,* the residue was diluted with MeOH and was passed down amSCX-2 cartridge eluting with MeOH and then 2M methanolic ammonia. The relevant fractions were evaporated and purified by MDAP (Luna Phenyl-Hexyl 21.2 x 150mm, 10μm 5-60% MeOH/$H_2O$ (0.1% FA), 20mL/min, RT) then purified again by MDAP (Sunfire C18 19 x 150mm, 10μm 5-95% ACN/$H_2O$ (0.1% FA), 20mL/min, RT) to give the product (20.1 mg).

LCMS (Method 4): Rt = 2.33 min, m/z 453.0 $[M+H]^+$
$^1H$ NMR (400 MHz, d6-DMSO) δ 8.66 (s, 1H), 8.35 (t, J=5.0 Hz, 1H), 8.17 (d, J=5.3 Hz, 1H), 7.47 - 7.42 (m, 2H), 7.21 - 7.12 (m, 2H), 7.07 (d, J=5.3 Hz, 1H), 6.43 (s, 2H), 4.76 (t, J=8.9 Hz, 2H), 4.71 (d, J=5.9 Hz, 2H), 3.40 (t, J=6.4 Hz, 2H), 3.32 - 3.28 (m, 2H), 3.26 (s, 3H), 3.07 (t, J=8.9 Hz, 2H), 1.80 - 1.72 (m, 2H).

**Example 56**

***Step A***

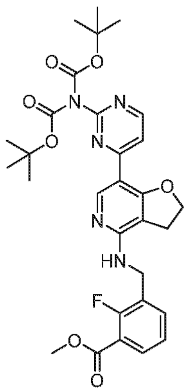

**Methyl 3-(((7-(2-(bis(tert-butoxycarbonyl)amino)pyrimidin-4-yl)-2,3-dihydrofuro[3,2-c]pyridin-4-yl)amino) methyl)-2-fluorobenzoate (Intermediate 56A)**

Intermediate 56A was prepared using a similar procedure to that used for *intermediate 1H* by replacing intermediate 1E with intermediate 54A.
LCMS (Method 1): Rt = 1.49 min, m/z 596.1 $[M+H]^+$

***Step B***

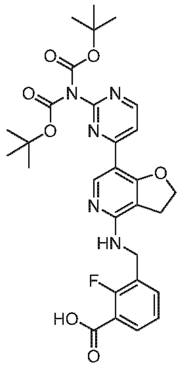

**3-(((7-(2-(Bis(tert-butoxycarbonyl)amino)pyrimidin-4-yl)-2,3-dihydrofuro[3,2-c]pyridin-4-yl)amino)methyl)-2-fluorobenzoic acid (Intermediate 56B)**

Intermediate 56B was prepared using a similar procedure to that used for *intermediate 1I* by replacing intermediate 1H with intermediate 56A.
LCMS (Method 1): Rt = 1.32 min, m/z 582.0 $[M+H]^+$

***Step C***

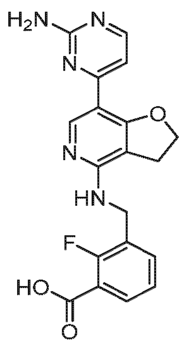

**3-(((7-(2-Aminopyrimidin-4-yl)-2,3-dihydrofuro[3,2-c]pyridin-4-yl)amino)methyl)-2-fluorobenzoic acid (Intermediate 56C)**

Intermediate 56C was prepared using a similar procedure to that used for *intermediate 1J* by replacing intermediate 1I with intermediate 56B.
LCMS (Method 1): Rt = 0.78 min, m/z 382.0 $[M+H]^+$

***Step D***

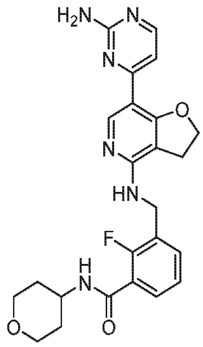

**3-(((7-(2-Aminopyrimidin-4-yl)-2,3-dihydrofuro[3,2-c]pyridin-4-yl)amino)methyl)-2-fluoro-N-(tetrahydro-2H-pyran-4-yl)benzamide (Example 56)**

Example 56 was prepared using a similar procedure to that used for *step K* of *Example 1* by replacing intermediate Intermediate 1J and cyclobutylamine respectively with Intermediate 56C and 4-aminotetrahydropyran.

LCMS (Method 4): Rt = 2.31 min, m/z 465.4 $[M+H]^+$
$^1H$ NMR (400 MHz, d6-DMSO) δ 8.66 (s, 1H), 8.34 (d, J=7.5 Hz, 1H), 8.17 (d, J=5.3 Hz, 1H), 7.46 - 7.38 (m, 2H), 7.20 - 7.13 (m, 2H), 7.07 (d, J=5.3 Hz, 1H), 6.42 (s, 2H), 4.77 (t, J=8.9 Hz, 2H), 4.71 (d, J=5.7 Hz, 2H), 4.05 - 3.95 (m, 1H), 3.90 - 3.85 (m, 2H), 3.44 - 3.37 (m, 2H), 3.07 (t, J=8.5 Hz, 2H), 1.81 - 1.76 (m, 2H), 1.60 - 1.49 (m, 2H).

**Example 57 to 63**

**Intermediate 57A to 63A**

The following intermediates were prepared similarly to Intermediate 54A from Intermediate 1D and the aldehyde indicated.

| Intermediate | Structure | Aldehyde | LCMS |
|---|---|---|---|
| 57A | 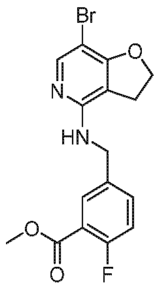 | Methyl 2-fluoro-5-formylbenzoate | Rt = 1.55 min, m/z 381.2/ 383.2 $[M+H]^+$ (Method 2) |
| 59A | 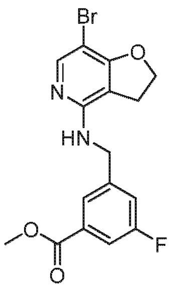 | Methyl 3-fluoro-5-formylbenzoate | Rt = 0.78 min, m/z 380.9/382.9 $[M+H]^+$ (Method 1) |

(continued)

| Intermediate | Structure | Aldehyde | LCMS |
|---|---|---|---|
| 61A | 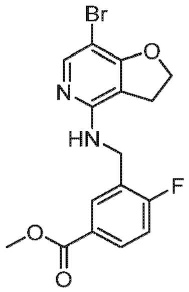 | Methyl 4-fluoro-3-formylbenzoate | Rt = 1.59 min, m/z 381.1/ 383.2 $[M+H]^+$ (Method 2) |
| 63A | 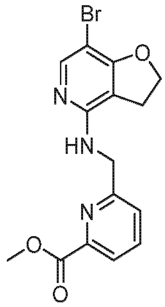 | Methyl 6-formyl-2-pyridine carboxylate | Rt = 0.99 min, m/z 363.9/365.8 $[M+H]^+$ (Method 1) |

## Intermediate 57B to 63B

The following intermediates were prepared using a similar procedure to that used for intermediate 1H by replacing intermediate 1E with indicated starting material.

| Intermediate | Structure | Starting material | LCMS |
|---|---|---|---|
| 57B | 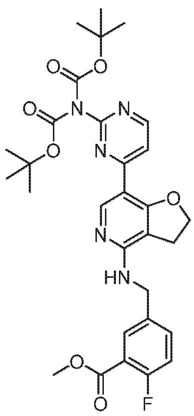 | Intermediate 57A | Rt = 1.72 min, m/z 596.5 $[M+H]^+$ (Method 2) |

(continued)

| Intermediate | Structure | Starting material | LCMS |
|---|---|---|---|
| 59B | 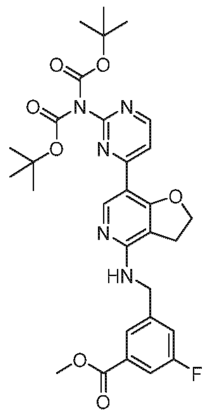 | Intermediate 59A | Rt = 1.76 mins, m/z 596.5 $[M+H]^+$ (Method 2) |
| 61B | 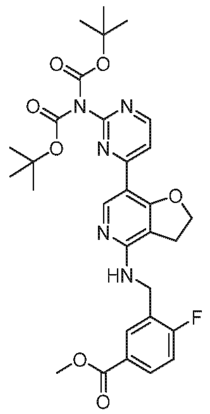 | Intermediate 61A | Rt = 1.77 min, m/z 596.5 $[M+H]^+$ (Method 2) |
| 63B | 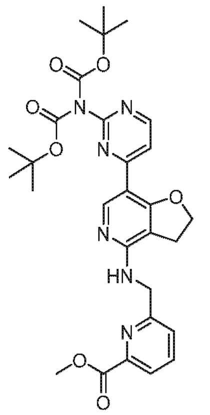 | Intermediate 63A | Rt = 1.27 min, m/z 579.1 $[M+H]^+$ (Method 1) |

**Intermediate 57C to 63C**

The following intermediates were prepared using a similar procedure to that used for intermediate 1I by replacing intermediate 1H with indicated starting material.

| Intermediate | Structure | Starting material | LCMS |
|---|---|---|---|
| 57C | 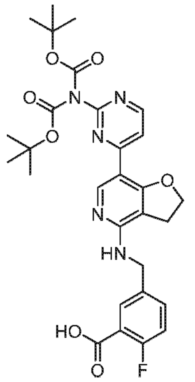 | Intermediate 57B | Rt = 1.05 min, m/z 582.1 $[M+H]^+$ (Method 2) |
| 59C | 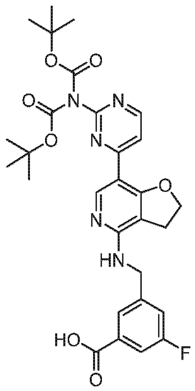 | Intermediate 59B | Rt = 1.10 mins, m/z 582.5 $[M+H]^+$ (Method 2) |
| 61C | 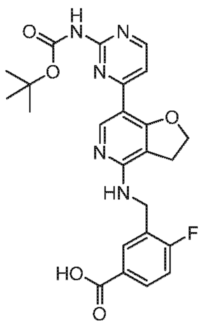 | Intermediate 61B | Rt = 1.07 min, m/z 482.5 $[M+H]^+$ (Method 1) |
| 63C | 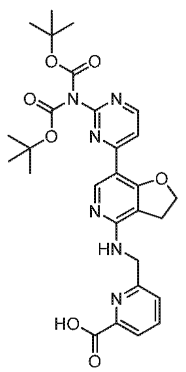 | Intermediate 63B | Rt = 1.21 min, m/z 565.1 $[M+H]^+$ (Method 1) |

**Intermediate 57D to 63D**

The following intermediates were prepared using a similar procedure to that used for intermediate 1J by replacing intermediate 1I with indicated starting material.

| Intermediate | Structure | Starting material | LCMS |
|---|---|---|---|
| 57D | 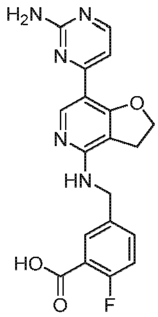 | Intermediate 57C | Rt = 0.79 min, m/z 382.3 $[M+H]^+$ (Method 2) |
| 59D | 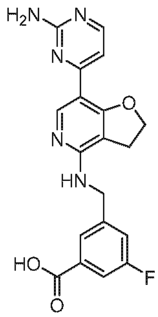 | Intermediate 59C | Rt = 0.82 mins, m/z 382.3 $[M+H]^+$ (Method 2) |
| 61D | 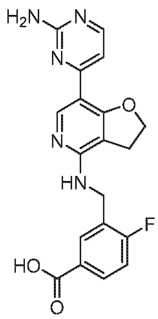 | Intermediate 61C | Rt = 0.89 min, m/z 382.3 $[M+H]^+$ (Method 2) |
| 63D | 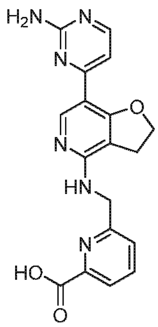 | Intermediate 63D | Rt = 0.73 min, m/z 365 $[M+H]^+$ (Method 1) |

**Example 57 to 63**

The following examples were made using a similar procedure to that used for *step K* of *Example 1* by replacing intermediate Intermediate 1J and cyclobutylamine respectively with the acid intermediate and amine indicated in the table below.

| Example | Structure | Acid Intermediate / amine | 1H NMR | LC-MS |
|---|---|---|---|---|
| 57 | 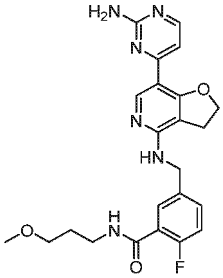 5-(((7-(2-Aminopyrimidin-4-yl)-2,3-dihydrofuro[3,2-c]pyridin-4-yl)amino)methyl)-2-fluoro-N-(3-methoxypropyl)benzamide | Intermediate 57D and 3-methoxy-propan -1-amine | $^1$H NMR (400 MHz, DMSO) δ 8.68 (s, 1H), 8.32 - 8.23 (m, 1H), 8.17 - 8.15 (m, 1H), 7.60 (dd, J=2.3, 7.0 Hz, 1H), 7.50 - 7.42 (m, 1H), 7.24 - 7.16 (m, 2H), 7.07 (d, J=5.3 Hz, 1H), 6.43 (s, 2H), 4.75 (t, J=8.9 Hz, 2H), 4.63 (d, J=5.9 Hz, 2H), 3.40 - 3.36 (m, 2H), 3.32 - 3.26 (m, 2H), 3.24 (s, 3H), 3.03 (t, J=9.0 Hz, 2H), 1.77 - 1.70 (m, 2H). | Rt = 2.49 min, m/z 453.0 $[M+H]^+$ (Method 4) |
| 58 | 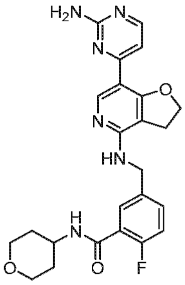 5-(((7-(2-Aminopyrimidin-4-yl)-2,3-dihydrofuro[3,2-c]pyridin-4-yl)amino)methyl)-2-fluoro-N-(tetrahydro-2H-pyran-4-yl)benzamide | Intermediate 57D and 4-aminotetra-hydr opyran | $^1$H NMR (400 MHz, DMSO) δ 8.68 (s, 1H), 8.27 (d, J=7.7 Hz, 1H), 8.16 (d, J=5.3 Hz, 1H), 7.55 (dd, J=2.3, 6.9 Hz, 1H), 7.49 - 7.44 (m, 1H), 7.24 - 7.16 (m, 2H), 7.07 (d, J=5.3 Hz, 1H), 6.43 (s, 2H), 4.75 (t, J=8.9 Hz, 2H), 4.63 (d, J=5.9 Hz, 2H), 4.01 - 3.92 (m, 1H), 3.86 (td, J=3.2, 11.4 Hz, 2H), 3.39 (dt, J=2.1, 11.6 Hz, 2H), 3.03 (t, J=8.9 Hz, 2H), 1.76 (dd, J=2.3, 12.3 Hz, 2H), 1.59 - 1.46 (m, 2H). | Rt = 2.35 min, m/z 465.4 $[M+H]^+$ (Method 4) |
| 59 | 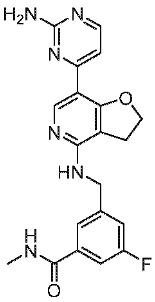 3-(((7-(2-Aminopyrimidin-4-yl)-2,3-dihydrofuro[3,2-c]pyridin-4-yl)amino)methyl)-5-fluoro-N-methylbenzamide | Intermediate 59D and methylamine hydrochloride | $^1$H NMR (400 MHz, DMSO) δ 8.67 (s, 1H), 8.54 - 8.51 (m, 1H), 8.17 (d, J=5.3 Hz, 1H), 7.70 (s, 1H), 7.49 - 7.47 (m, 1H), 7.30 (d, J=9.4 Hz, 1H), 7.22 (t, J=6.1 Hz, 1H), 7.07 (d, J=5.3 Hz, 1H), 6.45 (s, 2H), 4.76 (t, J=8.9 Hz, 2H), 4.69 (d, J=5.9 Hz, 2H), 3.06 (t, J=9.0 Hz, 2H), 2.78 (d, J=4.6 Hz, 3H). | Rt = 2.24min, m/z 395 $[M+H]^+$ (Method 4) |

(continued)

| Example | Structure | Acid Intermediate / amine | 1H NMR | LC-MS |
|---|---|---|---|---|
| 60 | 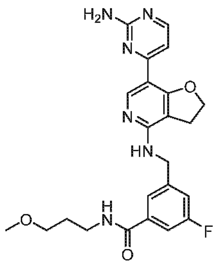 3-(((7-(2-Aminopyrimidin-4-yl)-2,3-dihydrofuro[3,2-c]pyridin-4-yl)amino)methyl)-5-fluoro-N-(3-methoxypropyl)benzamide | Intermediate 59D and 3-methoxy-propyl amine | $^1$H NMR (400 MHz, DMSO) δ 8.69 (s, 1H), 8.55 (t, J=5.6 Hz, 1H), 8.24 (d, J=6.0 Hz, 1H), 7.71 (s, 1H), 7.54 (d, J=9.4 Hz, 1H), 7.47-7.30 (m, 2H), 7.26 (d, J=5.9 Hz, 1H), 4.85 (t, J=8.8 Hz, 2H), 4.73 (d, J=6.0 Hz, 2H), 3.37 (t, J=5.8 Hz, 2H), 3.33 - 3.28 (m, 2H), 3.24 (s, 3H), 3.11 (t, J=8.9 Hz, 2H), 1.79 - 1.72 (m, 2H). | Rt = 2.51 min, m/z 453 [M+H]+ (Method 4) |
| 61 | 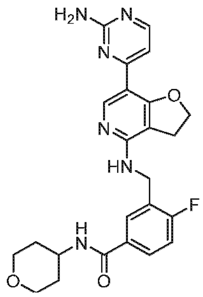 3-(((7-(2-Aminopyrimidin-4-yl)-2,3-dihydrofuro[3,2-c]pyridin-4-yl)amino)methyl)-4-fluoro-N-(tetrahydro-2H-pyran-4-yl)benzamide | Intermediate 61D and 4-aminotetrahydr opyran | $^1$H NMR (400 MHz, DMSO) δ 8.68 (s, 1H), 8.30 (d, J=7.7 Hz, 1H), 8.17 (d, J=5.3 Hz, 1H), 7.94 (dd, J=2.2, 7.3 Hz, 1H), 7.82 - 7.77 (m, 1H), 7.30 - 7.25 (m, 1H), 7.14 (t, J=5.9 Hz, 1H), 7.07 (d, J=5.3 Hz, 1H), 6.43 (s, 2H), 4.76 (t, J=9.0 Hz, 2H), 4.70 (d, J=5.1 Hz, 2H), 4.00 - 3.84 (m, 3H), 3.41 - 3.36 (m, 1H), 3.05 (t, J=9.0 Hz, 2H), 2.36 - 2.34 (m, 1H), 1.76 - 1.71 (m, 2H), 1.62 - 1.50 (m, 2H). | Rt = 2.38 min, m/z 465 [M+H]+ (Method 4) |
| 62 | 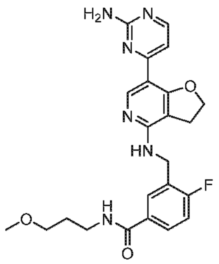 3-(((7-(2-Aminopyrimidin-4-yl)-2,3-dihydrofuro[3,2-c]pyridin-4-yl)amino)methyl)-4-fluoro-N-(3-methoxypropyl)benzamide | Intermediate 61D and 3-methoxy-propyl amine | $^1$H NMR (400 MHz, DMSO) δ 8.68 (s, 1H), 8.44 (t, J=5.6 Hz, 1H), 8.17 (d, J=5.3 Hz, 1H), 7.93 (dd, J=2.3, 7.4 Hz, 1H), 7.80 - 7.74 (m, 1H), 7.30 - 7.24 (m, 1H), 7.14 (t, J=6.0 Hz, 1H), 7.07 (d, J=5.3 Hz, 1H), 6.43 (s, 2H), 4.76 (t, J=9.1 Hz, 2H), 4.70 (d, J=5.7 Hz, 2H), 3.38-3.33 (m, 2H), 3.30 - 3.24 (m, 2H), 3.22 (s, 3H), 3.06 (t, J=8.9 Hz, 2H), 1.77 - 1.69 (m, 2H). | Rt = 2.44 min, m/z 453 [M+H]+ (Method 4) |

(continued)

| Example | Structure | Acid Intermediate / amine | 1H NMR | LC-MS |
|---|---|---|---|---|
| 63 | 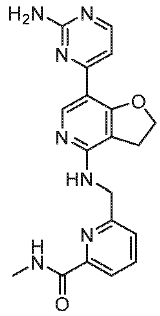 6-(((7-(2-Aminopyrimidin-4-yl)-2,3-dihydrofuro[3,2-c]pyridin-4-yl)amino)methyl)-N-methylpicolinamide | Intermediate 63D and methylamine hydrochloride | $^1$H NMR (400 MHz, DMSO) δ 8.71 (q, J=5.1 Hz, 1H), 8.65 - 8.64 (m, 1H), 8.17 (d, J=5.3 Hz, 1H), 7.93 - 7.86 (m, 2H), 7.49 (dd, J=1.6, 7.3 Hz, 1H), 7.25 (t, J=6.1 Hz, 1H), 7.08 (d, J=5.3 Hz, 1H), 6.44 - 6.41 (m, 2H), 4.82 - 4.75 (m, 4H), 3.12 (t, J=8.9 Hz, 2H), 2.87 (d, J=4.9 Hz, 3H). | Rt = 2.06 min, m/z 378 [M+H]+ (Method 4) |

**Example 64**

***Step A***

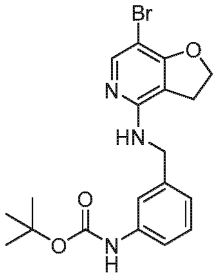

**tert-Butyl (3-(((7-bromo-2,3-dihydrofuro[3,2-c]pyridin-4-yl)amino)methyl)-phenyl)carbamate (Intermediate 64A)**

To a solution of Intermediate 1D (300 mg, 1.40 mmol) and tert-butyl (3-formylphenyl)carbamate (463 mg, 2.09 mmol) dissolved in DCM (10 mL) was added molecular sieves (4 Å), chlorotriisopropoxytitanium(IV) (0.67 mL, 2.79 mmol) was added and the mixture was stirred for 10 mins before the addition of sodium triacethoxyborohydride (591 mg, 2.79 mmol) and acetic acid (0.16 mL, 2.79 mmol). The reaction was stirred at RT under argon for 18 h. The reaction mixture was quenched using 1 M NaOH (20 mL) and the resulting suspension was filtered through diatomaceous earth,washing with water and DCM. The phases of the filtrate were separated, and the organic phase was dried with$Na_2SO_4$, filtered and concentrated *in vacuo.* The product was purified by flash chromatography on a silica gel cartridge, eluting with 0-50% EtOAc in cyclohexane. The relevant fractions were combined and concentrated *in vacuo* to give the desired product (487 mg).
LCMS (Method 2): Rt = 1.64 min, m/z 420.3/422.3 $[M+H]^+$

***Step B***

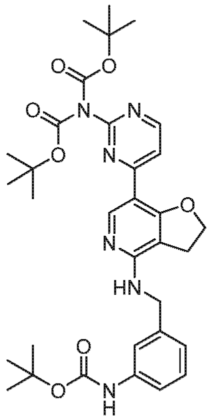

**tert-Butyl (tert-butoxycarbonyl)(4-(4-((3-((tert-butoxycarbonyl)amino)-benzyl)amino)-2,3-dihydrofuro[3,2-c] pyridin-7-yl)pyrimidin-2-yl)carbamate (Intermediate 64B)**

To a degassed solution of Intermediate 64A (350 mg, 0.833 mmol), Intermediate 1G (420 mg, 0.916 mmol) and copper (I) thiophene-2-carboxylate (16 mg, 0.0833 mmol) dissolved in dioxane (10 mL) was added tetrakis (triphenylphosphine)palladium(0) (48 mg, 0.0416 mmol) and the reaction was stirred at 130°C under microwave irradiation for 1.5 h. The reaction mixture was diluted with EtOAc and filtered through Celite®. The resulting filtrate was washed with water and brine then the organic layers were separated and dried with $Na_2SO_4$, filtered and concentrated *in vacuo.* The residue was dissolved in DCM and purified by flash chromatography on a silica gel cartridge, eluting with 0-40% EtOAc in DCM. The relevant fractions were combined and concentrated *in vacuo* to give the title product (181 mg).
LCMS (Method 2): Rt = 1.78 min, m/z 635.6 $[M+H]^+$

***Step C***

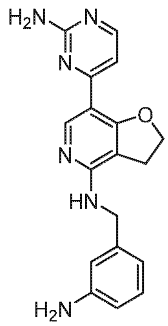

**N-(3-Aminobenzyl)-7-(2-aminopyrimidin-4-yl)-2,3-dihydrofuro[3,2-clpyridin-4-amine (Intermediate** 64C)

To a solution of Intermediate 64B (181 mg, 0.285 mmol) in DCM (8 mL) was added TFA (0.87 mL, 11.41 mmol) and the reaction mixture was stirred overnight. The reaction mixture was concentrated *in vacuo* and the resulting material was dissolved in MeOH and applied to an SCX-2 column, eluting with MeOH then methanolic ammonia. The relevant fractions were combined and concentrated *in vacuo* to afford the title compound (95 mg).
LCMS (Method 2): Rt = 1.08 min, m/z 335.3 $[M+H]^+$

***Step D***

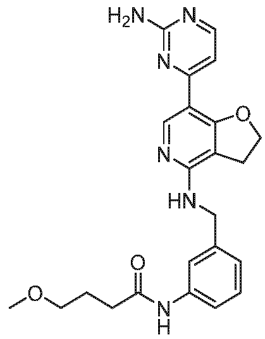

**N-(3-(((7-(2-aminopyrimidin-4-yl)-2,3-dihydrofuro[3,2-c]pyridin-4-yl)amino)methyl)phenyl)-4-methoxybutanamide (Example 64)**

To a stirred solution of Intermediate 64C (56 mg, 0.167 mmol), 4-methoxybutanoic acid (22 mg, 0.184 mmol) and TBTU (70mg, 0.184 mmol) in DMF (1.5 mL) was added DIPEA (0.088 mL, 0.502 mmol) and the reaction mixture was stirred at RTovernight. The reaction mixture was diluted with MeOH and then passed down anSCX-2 cartridge eluting with MeOH and then 2M methanolic ammonia. The relevant fractions were evaporated and further purified by MDAP (Luna Phenyl-Hexyl 21.2 x 150mm, 10μm 20-80% MeOH/$H_2O$ (0.1% FA), 20mL/min, RT) to give the title product (28 mg).

LCMS (Method 4): Rt = 2.33 min, m/z 435.0 $[M+H]^+$

$^1H$ NMR (400 MHz, d6-DMSO) δ 9.85 (s, 1H), 8.68 (s, 1H), 8.2, (s, 0.3 H), 8.16 (d, J=5.3 Hz, 1H), 7.51 (d, J=8.4 Hz, 2H), 7.22 (t, J=7.8 Hz, 1H), 7.12 (t, J=6.1 Hz, 1H), 7.07 (d, J=5.3 Hz, 1H), 7.01 (d, J=7.8 Hz, 1H), 6.42 (s, 2H), 4.75 (t, J=8.9 Hz, 2H), 4.62 (d, J=6.0 Hz, 2H), 3.23 (s, 3H), 3.04 (t, J=8.9 Hz, 2H), 2.34 (t, J=7.5 Hz, 2H), 1.84 - 1.76 (m, 2H).

**Example A (comparative)**

*Scheme*

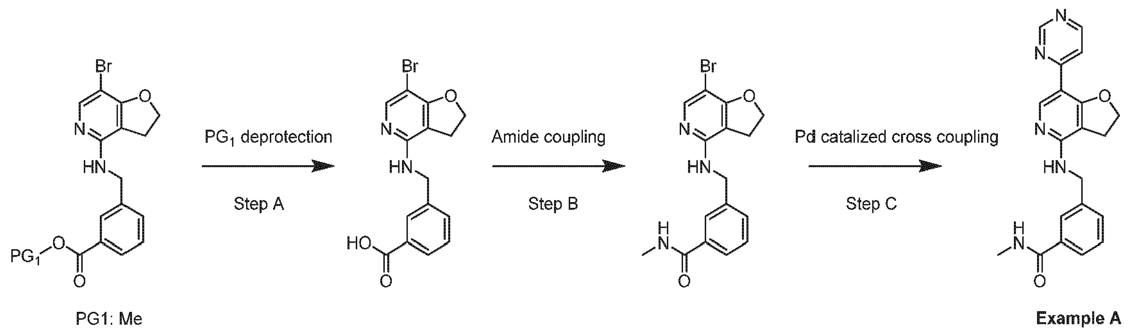


***Step A***

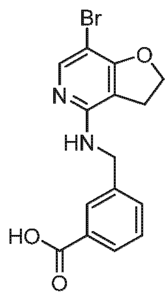

**3-(((7-Bromo-2,3-dihydrofuro[3,2-c]pyridin-4-yl)amino)methyl)benzoic acid (Intermediate AA)**

A solution of Intermediate 1E (100 mg, 0.27 mmol), lithium hydroxide monohydrate (0.035 g, 0.83 mmol) in THF (1 mL), MeOH (1 mL) and water (2 mL) was stirred at ambient temperature for 1.5 h. The resulting mixture was diluted with water and extracted with EtOAc. The pH of the aqueous phase was adjusted to pH ~2-3 with aqueous 1M HCl. The organic layer was dried over sodium sulphate and evaporated *in vacuo* to give the title product (89 mg).
LCMS (Method 1): Rt = 0.81 min, m/z 348.9/350.9 $[M+H]^+$

***Step B***

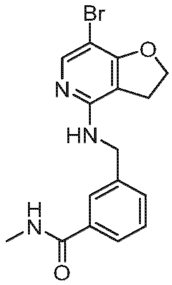

**3-(((7-Bromo-2,3-dihydrofuro[3,2-c]pyridin-4-yl)amino)methyl)-N-methylbenzamide (Intermediate AB)**

A mixture of Intermediate AA (40 mg, 0.12 mmol), methylamine hydrochloride (23 mg, 0.35 mmol), TBTU (150 mg, 0.46 mmol) and N,N-diisopropylethylamine (0.12 mL, 0.69 mmol) in DCM (4 mL) was stirred at ambient temperature for 18 h. The resulting mixture was diluted with water and extracted with DCM. The organic layer was dried over sodium sulphate and evaporated *in vacuo.* The residue, diluted with methanol, was passed down an SCX-2 cartridge eluting with methanol and then 2M methanolic ammonia. The relevant fractions were poole d and concentrated to dryness to give the title product (29 mg).
LCMS (Method 1): Rt = 0.73 min, m/z 362.0/364.0 $[M+H]^+$

***Step C***

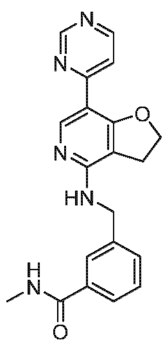

**N-Methyl-3-(((7-(pyrimidin-4-yl)-2,3-dihydrofuro[3,2-c]pyridin-4-yl)amino)methyl)benzamide (Example A)**

A degassed mixture of Intermediate AB (100 mg, 0.28 mmol), 4-(tributylstannyl)pyrimidine (110 mg, 0.304 mmol), tetrakis(triphenylphosphine)-palladium(0) (16 mg, 0.014 mmol) and copper(I) thiophene-2-carboxylate (5.3 mg, 0.028 mmol) in dioxane (3 mL) was heated at 150 °C under microwave irradiation for 1 h. The reaction mixture, diluted with MeOH, was passed down an SCX-2 cartridge eluting with MeOH and then 2M methanolic ammonia. The solution was concentrated *in vacuo* and the residue was purified by flash chromatography on silica gel by eluting with 0-10% MeOH in EtOAc. The relevant fractions were combined and concentrated. The residue was purified by MDAP (Luna Phenyl-Hexyl MeOH Acidic 5-60, Luna Phenyl-Hexyl 21.2x150mm, 10μm 5-60% MeOH / $H_2O$ (0.1% FA), 20 mL/min, RT) to give the

product (21 mg).

LCMS (Method 4): Rt = 2.18 min, m/z 362.2 $[M+H]^+$
$^1$H NMR (400 MHz, d6-DMSO) δ d 9.08 (d, J=1.1 Hz, 1H), 8.80 (s, 1H), 8.69 (d, J=5.5 Hz, 1H), 8.46-8.37 (m, 1H), 7.90 (dd, J=1.4, 5.5 Hz, 1H), 7.83 (s, 1H), 7.70-7.67 (m, 1H), 7.50-7.47 (m, 1H), 7.42-7.33 (m, 2H), 4.81 (t, J=9.0 Hz, 2H), 4.72 (d, J=6.0 Hz, 2H), 3.09 (t, J=9.0 Hz, 2H), 2.78 (d, J=4.6 Hz, 3H).

**Example B**

*Scheme*

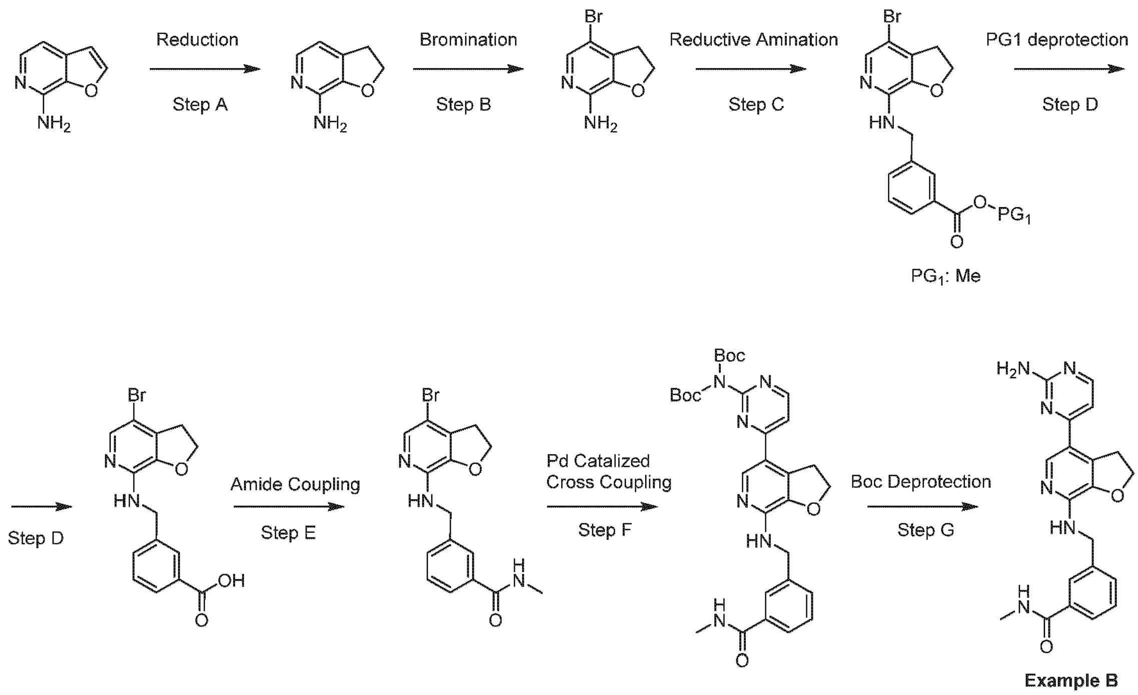


**Example B**

***Step A***

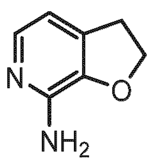

**2,3-Dihydrofuro[2,3-c]pyridin-7-amine (Intermediate BA)**

Intermediate BA was prepared using a similar procedure to that described for Intermediate 1C in *Step C* by replacing Intermediate 1B with furo[2,3-c]pyridin-7-amine.
LCMS (Method 10): Rt = 0.75 min, m/z 137.1 $[M+H]^+$

***Step B***

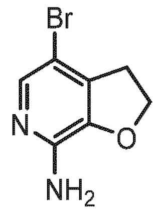

4-Bromo-2,3-dihydrofuro[2,3-c]pyridin-7-amine (Intermediate BB)

Intermediate BB was prepared similarly to Intermediate 1D by replacing Intermediate 1C of *step* C with Intermediate BA.
LCMS (Method 12): Rt = 1.72 min, m/z 214.8 an 216.8 $[M+H]^+$

***Step C***

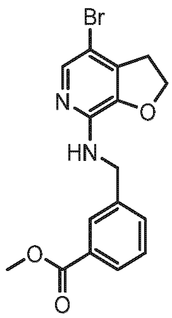

Methyl 3-(((4-bromo-2,3-dihydrofuro[2,3-c]pyridin-7-yl)amino)methyl)-benzoate (Intermediate BC)

A solution of Intermediate BB (500 mg, 2.33 mmol), methyl 3-formylbenzoate (573 mg, 3.49 mmol) and chlorotriisopropoxytitanium(IV) (1212 mg, 4.65 mmol) dissolved in DCM (15 mL) was stirred at room temperature for 18 h with molecular sieves. To this solution, acetic acid (0.27 mL) and sodium triacetoxyborohydride (1478 mg, 6.98 mmol) were added and the reaction was left to stir at room temperature for 48 h. The reaction mixture was quenched with 1N NaOH (120 mL), filtered through a pad of diatomaceous earth, and washed with DCM. The organic phase was washed with water, dried with $Na_2SO_4$ and concentrated *in vacuo.* The resulting residue was purified by flash chromatography on silica gel by eluting with 0-75% ethyl acetate in cyclohexane. The relevant fractions were combined and concentrated *in vacuo* to give the desired product (718 mg).
LCMS (Method 3): Rt = 2.90 min, m/z 363.2/365.2 $[M+H]^+$

***Step D***

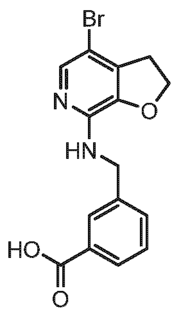

**3-(((4-Bromo-2,3-dihydrofuro[2,3-c]pyridin-7-yl)amino)methyl)benzoic acid (Intermediate BD)**

A solution of Intermediate BC (720 mg, 1.98 mmol) and lithium hydroxide monohydrate (250 mg, 5.95 mmol) dissolved in MeOH (3 mL), THF (3 mL) and water (6 mL) was stirred at room temperature for 6 h. The organic phase was evaporated and the aqueous phase was acidified to pH 2~3 using 1M HCl. The resulting mixture was diluted with water and extracted with Me-THF. The organic layer was dried with $Na_2SO_4$ and concentrated *in vacuo.* The residue was dissolved in water (8 mL), THF (7 mL) and MeOH (3 mL). More lithium hydroxide monohydrate (125 g, 2.98 mmol) was added and the reaction was left to stir at room temperature for a further 3 h. The organic phase was evaporated and the aqueous phase was acidified to pH 2~3 using 1M HCl. The resulting mixture was diluted with water and extracted with Me-THF. The organic layer was dried (with $Na_2SO_4$) and concentrated in vacuo to give the desired product (697 mg).
LCMS (Method 2): Rt = 0.92 min, m/z 349.2/351.2 $[M+H]^+$

***Step E***

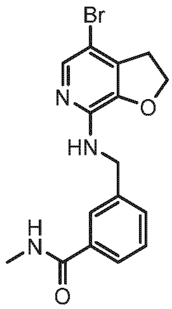

**3-(((4-Bromo-2,3-dihydrofuro[2,3-c]pyridin-7-yl)amino)methyl)-N-methylbenzamide (Intermediate BE)**

To a solution of Intermediate BD (250 mg, 0.716 mmol) and TBTU (300 mg, 0.931 mmol) dissolved in DCM (5 mL) were added DIPEA (075 mL) and methylamine hydrochloride (150 mg, 2.15 mmol) and the reaction mixture was left to stir at room temperature for 6 h. The reaction mixture was diluted with DCM and washed with water. The organic phase was dried with $Na_2SO_4$ and concentrated *in vacuo.* The resulting residue was dissolved in MeOH and passed through an SCX column eluting with MeOH followed by methanolic ammonia (2M). The relevant fractions were combined and concentrated *in vacuo* to give the desired product.
LCMS (Method 2): Rt = 1.32 min, m/z 362.3/364.2 $[M+H]^+$

***Step F***

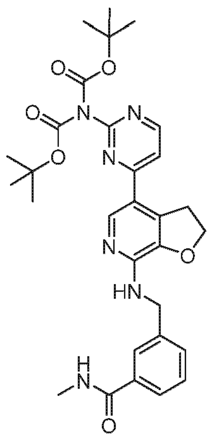

**tert-Butyl (tert-butoxycarbonyl)(4-(7-((3-(methylcarbamoyl)benzyl)amino)-2,3-dihydrofuro[2,3-c]pyridin-4-yl) pyrimidin-2-yl)carbamate (Intermediate BF)**

To a degassed solution of Intermediate BE (122 mg, 0.338 mmol), tetrakis (triphenylphosphine)palladium(0) (20 mg, 0.0169 mmol), copper(I) thiophene-2-carboxylate (6.4 mg, 0.0338 mmol) dissolved in dioxane (3 mL) was added Intermediate 1G (170 mg, 0.371 mmol) and the reaction was stirred at 130°C under microwave irradiation for 1.5 h. The reaction mixture was diluted with ethyl acetate and filtered through a pad of diatomaceous earth. The solution was washed with water and brine and the organic phase was combined, dried with $Na_2SO_4$, filtered and concentrated *in vacuo.* The resulting crude was dissolved in DCM and methanol and purified using flash chromatography on silica gel by eluting with 0-5% methanol in DCM. The residue was concentrated *in vacuo,* dissolved in DCM and purified using flash chromatography on silica eluting with 0-5% methanol in DCM. The relevant fractions from both purifications were combined and concentrated *in vacuo* to give the product (79 mg).
LCMS (Method 1): Rt = 1.45 min, m/z 577.1 $[M+H]^+$

***Step G***

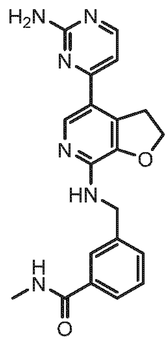

**3-(((4-(2-aminopyrimidin-4-yl)-2,3-dihydrofuro[2,3-c]pyridin-7-yl)amino)methyl)-N-methylbenzamide (Example B)**

To a solution of Intermediate BF (79 mg,0.137 mmol) dissolved in DCM (4 mL) was added TFA (1 mL) and the reaction was stirred at room temperature for 18 h. The reaction mixture was passed down an SCX-2 column eluting with methanol followed by 2M methanolic ammonia. The relevant fractions were combined, concentrated *in vacuo,* and the residue was purified by MDAP (Luna Phenyl-Hexyl 21.2x150mm, 10μm 5-60% MeOH/$H_2O$ (0.1% FA), 20 mL/min, RT) to give the desired product (33.8 mg).

LCMS (Method 4): Rt = 3.21 min, m/z 377.0 $[M+H]^+$
$^1$H NMR (400 MHz, d6-DMSO) δ 8.40 (m, 1H), 8.16-8.20 (m, 2H), 7.81 (s, 1H), 7.65 (d, J=7.7 Hz, 1H), 7.46 (d, J=7.9 Hz, 1H), 7.37 (t, J=7.8 Hz, 1H), 7.10 (t, J=6.4 Hz, 1H), 6.92 (d, J=5.3 Hz, 1H), 6.48 (br s, 2H), 4.61-4.68 (m, 4H), 3.68 (t, J=8.8 Hz, 2H), 2.78 (d, J=4.6 Hz, 3H).

**Example C**

*Scheme*

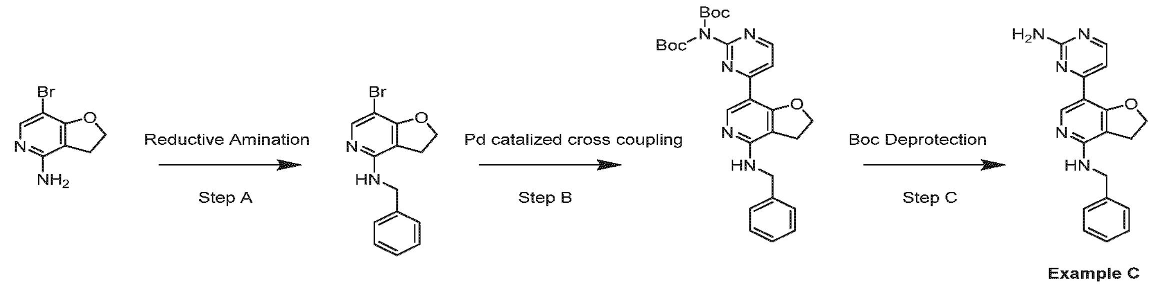


***Step A***

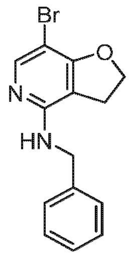

**N-Benzyl-7-bromo-2,3-dihydrofuro[3,2-c]pyridin-4-amine (Intermediate CA)**

Intermediate CA was prepared similarly to *Intermediate BC* by replacing methyl 3-formylbenzoate with benzaldehyde.
LCMS (Method 2): Rt = 1.57 min, 303.0-305.0 m/z $[M+H]^+$

***Step B***

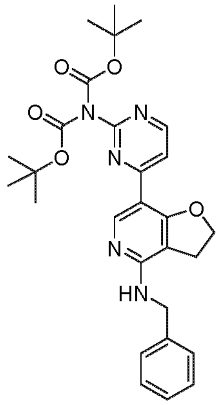

**tert-Butyl (4-(4-(benzylamino)-2,3-dihydrofuro[3,2-c]pyridin-7-yl)pyrimidin-2-yl)(tert-butoxycarbonyl)carbamate (Intermediate CB)**

Intermediate CB was prepared similarly to *Intermediate BF* by replacing Intermediate BE with Intermediate CA.
LCMS (Method 2): Rt = 1.73 min, m/z 520.4 $[M+H]^+$

***Step C***

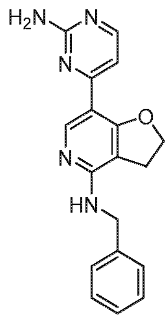

**7-(2-Aminopyrimidin-4-yl)-N-benzyl-2,3-dihydrofuro[3,2-c]pyridin-4-amine**

Example C was prepared similarly to *Example B* by replacing Intermediate BF of *step G* with Intermediate CB.

LCMS (Method 4): Rt = 2.5 min, m/z 320 $[M+H]^+$
$^1H$ NMR (400 MHz, d6-DMSO) δ 8.68 (s, 1H), 8.16 (d, J=5.3 Hz, 1H), 7.36 - 7.28 (m, 4H), 7.25 - 7.20 (m, 1H), 7.13 (t, J=5.5 Hz, 1H), 7.07 (d, J=5.0 Hz, 1H), 6.41 (s, 2H), 4.75 (t, J=9.0 Hz, 2H), 4.66 (d, J=6.0 Hz, 2H), 3.04 (t, J=8.9 Hz, 2H).

**PHARMACOLOGICAL ACTIVITY OF THE COMPOUNDS OF THE INVENTION.**

**In vitro inhibitory activity assay description ROCK1 and ROCK2**

The effectiveness of compounds of the present invention to inhibit Rho kinase activity can be determined in a 10μl assay containing 40mM Tris pH7.5, 20mM $MgCl_2$ 0.1mg/mL BSA, 50μM DTT and 2.5μM peptide substrate (Myelin Basic Protein) using an ADP-Glo kit (Promega). Compounds were dissolved in DMSO such that the final concentration of DMSO was 1% in the assay. All reactions/incubations are performed at 25oC. Compound (2ul) and either Rho kinase 1 or 2 (4μl) were mixed and incubated for 30 mins. Reactions were initiated by addition of ATP (4μl) such that the final concentration of ATP in the assay was 200μM. After a 1 hour incubation 10μl of ADP-Glo Reagent was added and after a further 1 hour incubation 20ul of Kinase Detection Buffer was added and the mixture incubated for a further 45 minutes. The luminescent signal was measured on a luminometer. Controls consisted of assay wells that did not contain compound with background determined using assay wells with no enzyme added. Compounds were tested in dose-response format and the inhibition of kinase activity was calculated at each concentration of compound. To determine the IC50 (concentration of compound required to inhibit 50% of the enzyme activity) data were fit to a plot of % inhibition vs Log10 compound concentration using a sigmoidal fit with a variable slope and fixing the maximum to 100% and the minimum to 0%. To determine the Ki values the Cheng-Prusoff equation was utilized ($Ki=IC_{50}/(1+[S]/Km)$.

Compounds according to the invention showed Ki values lower than 500 nM on both isoforms.

The results for individual compounds are provided below in following table and are expressed as range of activity.

**In vitro inhibitory activity assay description for PKA**

The effectiveness of compounds of the present invention to inhibit PKA activity can be determined in a 10μl assay containing 40mM Tris pH7.5, 20mM MgCl2 0.1mg/ml BSA, 50μM DTT and 260μM peptide substrate (kemptide) using an ADP-Glo kit (Promega). Compounds were dissolved in DMSO such that the final concentration of DMSO was 1% in the assay. All reactions/incubations are performed at 25oC. Compound and PKA enzyme (6μl) were mixed and incubated for 30 mins. Reactions were initiated by addition of ATP (4μl) such that the final concentration of ATP in the assay was 10μM. After a 30 minute incubation 10μl of ADP-Glo Reagent was added and after a further 1 hour incubation 20μl of Kinase Detection Buffer was added and the mixture incubated for a further 45 minutes. The luminescent signal was measured on a luminometer. Controls consisted of assay wells that did not contain compound with background determined using assay wells with no enzyme added. Compounds were tested in dose-response format and the inhibition of kinase activity was calculated at each concentration of compound. To determine the $IC_{50}$ (concentration of compound required to inhibit 50% of the enzyme activity) data were fit to a plot of % inhibition vs $Log_{10}$ compound concentration using a sigmoidal fit with a variable slope and fixing the maximum to 100% and the minimum to 0%. To determine the Ki values the Cheng-Prusoff equation was utilized ($Ki=IC_{50}/(1+[S]/Km)$.

In vitro inhibitory activities for PKA were reported as selectivity ratio vs. ROCK-2. Selectivity ratio PKA/ROCK2 was calculated by dividing the Ki value for PKA by Ki value of ROCK2 and reported in the following table.

Table 1

| Ex No. | ROCK 1 | ROCK2 | Ratio (PKA/ROCK2) |
|---|---|---|---|
| 1 | +++ | +++ | ** |
| 2 | +++ | +++ | *** |
| 3 | +++ | +++ | *** |
| 4 | +++ | +++ | *** |
| 5 | +++ | +++ | *** |
| 6 | +++ | +++ | *** |
| 7 | +++ | +++ | *** |
| 8 | +++ | +++ | *** |
| 9 | +++ | +++ | *** |
| 10 | +++ | +++ | ** |
| 11 | ++ | ++ | *** |
| 12 | ++ | +++ | *** |
| 13 | +++ | +++ | *** |
| 14 | +++ | +++ | *** |
| 15 | +++ | +++ | *** |
| 16 | +++ | +++ | *** |
| 17 | +++ | +++ | *** |
| 18 | +++ | +++ | *** |
| 19 | +++ | +++ | *** |
| 20 | +++ | +++ | *** |
| 21 | ++ | +++ | *** |
| 22 | ++ | ++ | ** |
| 23 | +++ | +++ | *** |
| 24 | +++ | +++ | *** |
| 25 | +++ | +++ | *** |
| 26 | +++ | +++ | *** |
| 27 | +++ | +++ | *** |
| 28 | ++ | +++ | *** |
| 29 | +++ | +++ | *** |
| 30 | +++ | +++ | ** |
| 31 | +++ | +++ | *** |
| 32 | +++ | +++ | *** |
| 33 | +++ | +++ | *** |
| 34 | +++ | +++ | *** |
| 35 | +++ | +++ | *** |
| 36 | +++ | +++ | *** |
| 37 | +++ | +++ | *** |

(continued)

| Ex No. | ROCK 1 | ROCK2 | Ratio (PKA/ROCK2) |
|---|---|---|---|
| 38 | +++ | +++ | *** |
| 39 | +++ | +++ | *** |
| 40 | +++ | +++ | *** |
| 41 | +++ | +++ | *** |
| 42 | +++ | +++ | *** |
| 43 | +++ | +++ | *** |
| 44 | +++ | +++ | *** |
| 45 | +++ | +++ | *** |
| 46 | +++ | +++ | *** |
| 47 | +++ | +++ | *** |
| 48 | +++ | +++ | ** |
| 49 | +++ | +++ | *** |
| 50 | +++ | +++ | *** |
| 51 | +++ | +++ | *** |
| 52 | +++ | +++ | *** |
| 53 | +++ | +++ | *** |
| 54 | + | ++ | *** |
| 55 | ++ | +++ | *** |
| 56 | ++ | ++ | *** |
| 57 | ++ | +++ | *** |
| 58 | ++ | +++ | *** |
| 59 | +++ | +++ | *** |
| 60 | +++ | +++ | *** |
| 61 | + | ++ | *** |
| 62 | + | ++ | *** |
| 63 | + | ++ | *** |
| 64 | ++ | +++ | *** |
| A | + | + | ** |
| B | + | + | *** |
| C | + | ++ | * |

wherein the compounds are classified in term of potency with respect to their inhibitory activity on ROCK1 and ROCK2 isoforms according to the following classification criterion:

+++ :

$$Ki \leq 3 \text{ nM}$$

++ :

$$3 < Ki \leq 30 \text{ nM}$$

+ :

$$Ki > 30 \text{ nM}$$

The Compounds according to the invention showed advantageously Ki values equal to or lower than 30 nM, preferably even equal to or lower that 3 nM, at least on ROCK2; further preferably equal to or lower than 30 nM, preferably even equal to or lower that 3 nM on both isoforms. The compounds according to the invention are more potent than the *comparative example A and B.*

Moreover, preferred compounds according to the invention exhibit marked selectivity versus PKA. The compounds according to the invention are at least 5 fold, preferably equal to or more than 10 fold, selective in terms of ROCK2 selectivity vs PKA. Overall the compounds of the invention are more selective than the *comparative example C*.

In the table the compounds are classified in term of selectivity with respect to their ratio of inhibitory activity (Ki) of PKA on ROCK2 isoform according to the following classification criterion:

*** :

$$\text{ratio} \geq 10$$

** :

$$5 \leq \text{ratio} < 10$$

* :

$$\text{ratio} < 5$$

## Claims

**1.** A compound of formula (I)

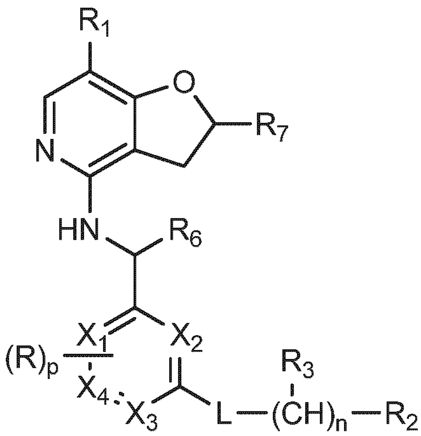

(I)

wherein

**$X_1$, $X_2$, $X_3$** and **$X_4$** are all CH or one of $X_1$, $X_2$, $X_3$ and $X_4$ is N and the others are CH;
p is zero or an integer from 1 to 4;
each R, when present, is in each occurrence independently selected from ($C_1$-$C_6$)alkyl and halogen selected from F, Cl, Br and I; wherein preferably R is F, Cl or methyl;
$R_1$ is pyrimidinyl, preferably pyrimidin-4yl, substituted by one or more group selected from $-(CH_2)_mNH_2$; particularly preferably $R_1$ is 2-aminopyrimidin-4-yl;

L is -C(O)NH- or -NHC(O)- ;
n is in each occurrence independently 0 or an integer selected from 1, 2 or 3;
$R_2$ and $R_3$ are in each occurrence independently selected from the group consisting of
-H,
halogen,
-OH,
$-(CH_2)_mNR_4R_5$,
$(C_1-C_6)$alkyl,
$(C_1-C_6)$hydroxyalkyl,
$(C_1-C_6)$ alkoxy,
$(C_1-C_6)$ alkoxy $(C_1-C_6)$alkyl,
$(C_1-C_6)$haloalkyl,
$(C_1-C_6)$haloalkoxy,
$(C_1-C_6)$haloalkoxy $(C_1-C_6)$alkyl,
$(C_3-C_{10})$cycloalkyl,
aryl and $(C_3-C_6)$heterocycloalkyl,
each of which cycloalkyl, aryl and heterocycloalkyl
is in its turn optionally and independently substituted with one or more groups selected from
halogen,
-OH,
$(C_1-C_6)$alkyl,
$(C_1-C_6)$hydroxyalkyl,
$(C_1-C_6)$ alkoxy,
$(C_1-C_6)$ alkoxy $(C_1-C_6)$alkyl,
$(C_1-C_6)$haloalkyl,
$(C_1-C_6)$haloalkoxy,
$-(CH_2)_mNR_4R_5$,
$-O-(CH_2)_mNR_4R_5$,
alkanoyl,
aryl, heteroaryl, cycloalkyl,
aryl-$(C_1-C_6)$alkyl,
heteroaryl-$(C_1-C_6)$alkyl ,
$(C_3-C_8)$heterocycloalkyl,
$(C_3-C_8)$heterocycloalkyl-$(C_1-C_6)$alkyl,
$(C_3-C_8)$cycloalkyl-$(C_1-C_6)$alkyl,
each of said aryl, heteroaryl, cycloalkyl, heterocycloalkyl is still further optionally substituted by one or more group selected independently from halogen selected from F, Cl, Br and I, preferably F, -OH, $(C_1-C_8)$alkyl, $(C_1-C_6)$ haloalkyl, $(C_1-C_6)$hydroxyalkyl,
m is in each occurrence independently 0 or an integer selected from 1, 2 or 3; $R_4$ and $R_5$, the same or different, are selected from the group consisting of
-H,
$(C_1-C_6)$alkyl,
$(C_1-C_6)$haloalkyl,
$(C_1-C_6)$hydroxyalkyl,
$(C_3-C_6)$heterocycloalkyl;
$R_6$ and $R_7$ are independently selected from the group consisting of -H, $(C_1-C_6)$alkyl;
wherein, where not already specified, aryl refers to mono, bi- or tri-cyclic ring systems which have 6 to 20 ring atoms, in which at least one ring is aromatic; heteroaryl refers to mono-, bi- or tri-cyclic ring systems with 5 to 20 ring atoms, in which at least one ring is aromatic and in which at least one ring atom is a heteroatom selected from N, S or O; $(C_3-C_6)$heterocycloalkyl refers to saturated or partially unsaturated monocyclic $(C_3-C_6)$cycloalkyl groups in which at least one ring carbon atom is replaced by at least one heteroatom selected from N, NH, S or O and/or may bear an oxo substituent group;
single enantiomers, diastereoisomers and mixtures thereof in any proportion and pharmaceutically acceptable salts and solvates thereof.

**2.** A compound according to Claim 1,

wherein $X_1$, $X_3$ and $X_4$ are all CH groups and $X_2$ is a CH group or a nitrogen atom; $R_1$ is 2-aminopyrimidin-4-yl;

all the other variables being as defined in claim 1.

**3.** A compound according to Claim 1,

wherein $X_1$ , $X_2$, $X_3$, $X_4$ are all CH group;
each R, when present, is in each occurrence independently selected from F, Cl, Br and I;
$R_1$ is pyrimidinyl substituted by $-NH_2$;
L is -C(O)NH-;
n is 0;
$R_2$ is selected from the group consisting of
$(C_1-C_6)$alkyl,
$(C_1-C_6)$hydroxyalkyl,
$(C_1-C_6)$haloalkyl,
$(C_1-C_6)$ alkoxy $(C_1-C_6)$alkyl,
$(C_1-C_6)$haloalkoxy $(C_1-C_6)$alkyl,
all the other variables being as defined in claim 1,
single enantiomers, diastereoisomers and mixtures thereof in any proportion
and/or or pharmaceutically acceptable salts and solvates thereof.

**4.** A compound according to Claim 3,

wherein $R_2$ is selected from methyl, ethyl, propyl, 2-hydroxy-2-methylpropyl, 3-methoxypropyl, 2-methoxyethyl, 2-ethoxyethyl, 3-isopropoxypropyl, 3-methoxy-3-methylbutyl, 2-methoxy-2-methylpropyl, 3-fluoropropyl, 2-fluoro-2-methylpropyl, 2,2-difluoropropyl, 3,3-difluoropropyl,
single enantiomers, diastereoisomers and mixtures thereof in any proportion
and/or pharmaceutically acceptable salts and solvates thereof

**5.** A compound according to Claim 1,

wherein $\mathbf{X_1}$**,** $\mathbf{X_2}$**,** $\mathbf{X_3}$ and $\mathbf{X_4}$ are all CH;
p is zero or an integer from 1 to 4;
each R, when present, is halogen in each occurrence independently selected from F, Cl, Br and I, wherein preferably R is F;
$R_1$ is pyrimidinyl substituted by $-NH_2$; particularly preferably $R_1$ is 2-aminopyrimidin-4-yl;
L is -C(O)NH-;
n is in each occurrence independently 0 or an integer selected from 1, 2 or 3; $R_3$, when present, is H,
and
$R_2$ is $(C_3-C_6)$heterocycloalkyl,
which
is in its turn optionally substituted with one or more groups selected from $(C_1-C_6)$alkyl,
$(C_1-C_6)$hydroxyalkyl,
$(C_1-C_6)$ alkoxy,
$(C_1-C_6)$ alkoxy $(C_1-C_6)$alkyl,
heteroaryl-$(C_1-C_6)$alkyl,
$(C_3-C_8)$cycloalkyl-$(C_1-C_6)$alkyl,
$-O-(CH_2)_mNR_4R_5$,
$(C_3-C_6)$heterocycloalkyl m is in each occurrence independently 0 or an integer selected from 1, 2 or 3;
$R_4$ and $R_5$, the same or different, are selected from the group consisting of
-H,
$(C_1-C_6)$alkyl,
$(C_1-C_6)$haloalkyl,
$(C_1-C_6)$hydroxyalkyl,
all the other variables being as defined in claim 1,
single enantiomers, diastereoisomers and mixtures thereof in any proportion and/or pharmaceutically acceptable salts and solvates thereof.

**6.** A compound according to Claim 5, wherein $R_2$ is selected from tetrahydrofuranyl, dimethyltetrahydrofuranyl, oxetan-2-yl.

**7.** A compound of formula (I) according to Claim 1, wherein

**$X_1$**, **$X_2$, $X_3$** and **$X_4$** are all CH or $X_2$, is N and the others are CH;
p is zero or 1;
each R, when present, is F;
$R_1$ is **2-aminopyrimidin-4-yl;**
L is -C(O)NH- or -NHC(O)- ;
n is in each occurrence independently 0 or an integer selected from 1, 2 or 3;
$R_2$ and $R_3$ are in each occurrence independently selected from the group consisting of
-H,
$(C_1-C_6)$alkyl which is methyl, propyl, isopropyl, tert-butyl,
$(C_1-C_6)$hydroxyalkyl which is 2-hydroxy-2-methylpropyl,
$(C_1-C_6)$ alkoxy $(C_1-C_6)$alkyl which is 3-methoxypropyl, 2-ethoxyethyl, 3-isopropoxypropyl, 3-methoxy-3-methylbutyl, 2-methoxyethyl, 2-methoxy-2-methylpropyl,
$(C_1-C_6)$haloalkyl which is 2,2-difluoroethyl, 2,2,2-trifluoroethyl, 2,2,2-trifluoromethyl, 2-fluoro-2-methylpropyl, 3-fluoropropyl, 2,2-difluoropropyl, 3,3-difluoropropyl,
$(C_1-C_6)$haloalkoxy $(C_1-C_6)$alkyl which is 3-(difluoromethoxy)propyl,
$(C_3-C_{10})$cycloalkyl which is cyclopropyl, cyclobutyl,
Heteroaryl which is pyridinyl, and
and
$(C_3-C_6)$heterocycloalkyl which is azetidin-3-yl, piperidinyl, morpholinyl, 5-oxopyrrolidin-3-yl, (1R,5S,6r)-3-oxabicyclo[3.1.0]hexan-6-yl, tetrahydrofuranyl, tetrahydro-2H-pyran-4-yl,
each of which cycloalkyl, heteroaryl and heterocycloalkyl
is in its turn optionally and independently substituted with one or more groups selected from
halogen which is selected from F, Cl, Br, I,
$(C_1-C_6)$alkyl which is methyl, tert-butyl,
$(C_1-C_6)$ alkoxy which is methoxy,
$(C_1-C_6)$ alkoxy $(C_1-C_6)$alkyl which is methoxymethyl,
$R_6$ and $R_7$ are -H,
single enantiomers, diastereoisomers and mixtures thereof in any proportion,
or pharmaceutically acceptable salts and solvates thereof.

**8.** A compound according to claim 1 selected from:

3-(((7-(2-aminopyrimidin-4-yl)-2,3-dihydrofuro[3,2-c]pyridin-4-yl)amino)methyl)-N-cyclobutylbenzamide;
3-(((7-(2-aminopyrimidin-4-yl)-2,3-dihydrofuro[3,2-c]pyridin-4-yl)amino)methyl)-N-((tetrahydro-2H-pyran-4-yl)methyl)benzamide;
3-(((7-(2-aminopyrimidin-4-yl)-2,3-dihydrofuro[3,2-c]pyridin-4-yl)amino)methyl)-N-(5,5-dimethyltetrahydrofuran-3-yl)benzamide;
3-(((7-(2-aminopyrimidin-4-yl)-2,3-dihydrofuro[3,2-c]pyridin-4-yl)amino)methyl)-N-cyclopropylbenzamide;
3-(((7-(2-aminopyrimidin-4-yl)-2,3-dihydrofuro[3,2-c]pyridin-4-yl)amino)methyl)-N-(2-(piperidin-1-yl)ethyl)benzamide;
3-(((7-(2-aminopyrimidin-4-yl)-2,3-dihydrofuro[3,2-c]pyridin-4-yl)amino)methyl)-N-(3-(methoxy-d3)propyl)benzamide;
3-(((7-(2-aminopyrimidin-4-yl)-2,3-dihydrofuro[3,2-c]pyridin-4-yl)amino)methyl)-N-(tetrahydro-2H-pyran-4-yl)benzamide;
3-(((7-(2-aminopyrimidin-4-yl)-2,3-dihydrofuro[3,2-c]pyridin-4-yl)amino)methyl)-N-(3-methoxypropyl)benzamide;
3-(((7-(2-aminopyrimidin-4-yl)-2,3-dihydrofuro[3,2-c]pyridin-4-yl)amino)methyl)-N-(2-methoxyethyl)benzamide;
3-(((7-(2-aminopyrimidin-4-yl)-2,3-dihydrofuro[3,2-c]pyridin-4-yl)amino)methyl)-N-(3,3-difluorocyclobutyl)benzamide;
3-(((7-(2-aminopyrimidin-4-yl)-2,3-dihydrofuro[3,2-c]pyridin-4-yl)amino)methyl)-N-isopropylbenzamide;
3-(((7-(2-aminopyrimidin-4-yl)-2,3-dihydrofuro[3,2-c]pyridin-4-yl)amino)methyl)-N-(2,2,2-trifluoroethyl)benzamide;
3-(((7-(2-aminopyrimidin-4-yl)-2,3-dihydrofuro[3,2-c]pyridin-4-yl)amino)methyl)-N-(1-methylpiperidin-4-yl)benzamide;

3-(((7-(2-aminopyrimidin-4-yl)-2,3-dihydrofuro[3,2-c]pyridin-4-yl)amino)methyl)-N-(3-(difluoromethoxy)propyl) benzamide;
3-(((7-(2-aminopyrimidin-4-yl)-2,3-dihydrofuro[3,2-c]pyridin-4-yl)amino)methyl)-N-(3-isopropoxypropyl)benzamide;
3-(((7-(2-aminopyrimidin-4-yl)-2,3-dihydrofuro[3,2-c]pyridin-4-yl)amino)methyl)-N-(3-morpholinopropyl)benzamide;
3-(((7-(2-aminopyrimidin-4-yl)-2,3-dihydrofuro[3,2-c]pyridin-4-yl)amino)methyl)-N-(2-hydroxy-2-methylpropyl) benzamide;
3-(((7-(2-aminopyrimidin-4-yl)-2,3-dihydrofuro[3,2-c]pyridin-4-yl)amino)methyl)-N-((1s,3s)-3-methoxycyclobutyl)benzamide;
3-(((7-(2-aminopyrimidin-4-yl)-2,3-dihydrofuro[3,2-c]pyridin-4-yl)amino)methyl)-N-(2-((2R,6S)-2,6-dimethylpiperidin-1-yl)ethyl)benzamide;
3-(((7-(2-aminopyrimidin-4-yl)-2,3-dihydrofuro[3,2-c]pyridin-4-yl)amino)methyl)-N-(2-ethoxyethyl)benzamide;
3-(((7-(2-aminopyrimidin-4-yl)-2,3-dihydrofuro[3,2-c]pyridin-4-yl)amino)methyl)-N-methylbenzamide;
3-(((7-(2-aminopyrimidin-4-yl)-2,3-dihydrofuro[3,2-c]pyridin-4-yl)amino)methyl)-N-(1-methylcyclopropyl)benzamide;
3-(((7-(2-aminopyrimidin-4-yl)-2,3-dihydrofuro[3,2-c]pyridin-4-yl)amino)methyl)-N-((1R,5S,6r)-3-oxabicyclo [3.1.0]hexan-6-yl)benzamide;
3-(((7-(2-aminopyrimidin-4-yl)-2,3-dihydrofuro[3,2-c]pyridin-4-yl)amino)methyl)-N-(1-(oxetan-3-yl)azetidin-3-yl)benzamide;
3-(((7-(2-aminopyrimidin-4-yl)-2,3-dihydrofuro[3,2-c]pyridin-4-yl)amino)methyl)-N-(cyclopropylmethyl)benzamide;
3-(((7-(2-aminopyrimidin-4-yl)-2,3-dihydrofuro[3,2-c]pyridin-4-yl)amino)methyl)-N-((1s,3s)-3-(difluoromethoxy) cyclobutyl)benzamide;
3-(((7-(2-aminopyrimidin-4-yl)-2,3-dihydrofuro[3,2-c]pyridin-4-yl)amino)methyl)-N-(2-methoxy-2-methylpropyl) benzamide;
3-(((7-(2-aminopyrimidin-4-yl)-2,3-dihydrofuro[3,2-c]pyridin-4-yl)amino)methyl)-N-((1-(methoxymethyl)cyclopropyl)methyl)benzamide;
3-(((7-(2-aminopyrimidin-4-yl)-2,3-dihydrofuro[3,2-c]pyridin-4-yl)amino)methyl)-N-(3-fluoropropyl)benzamide;
3-(((7-(2-aminopyrimidin-4-yl)-2,3-dihydrofuro[3,2-c]pyridin-4-yl)amino)methyl)-N-((1-methylcyclopropyl) methyl)benzamide;
3-(((7-(2-aminopyrimidin-4-yl)-2,3-dihydrofuro[3,2-c]pyridin-4-yl)amino)methyl)-N-(2-fluoro-2-methylpropyl) benzamide;
3-(((7-(2-aminopyrimidin-4-yl)-2,3-dihydrofuro[3,2-c]pyridin-4-yl)amino)methyl)-N-((1-(tert-butyl)-5-oxopyrrolidin-3-yl)methyl)benzamide (Enantiomer 1);
3-(((7-(2-aminopyrimidin-4-yl)-2,3-dihydrofuro[3,2-c]pyridin-4-yl)amino)methyl)-N-((1-(tert-butyl)-5-oxopyrrolidin-3-yl)methyl)benzamide (Enantiomer 2);
3-(((7-(2-aminopyrimidin-4-yl)-2,3-dihydrofuro[3,2-c]pyridin-4-yl)amino)methyl)-N-((4-methylmorpholin-2-yl) methyl)benzamide (Enantiomer 1);
3-(((7-(2-aminopyrimidin-4-yl)-2,3-dihydrofuro[3,2-c]pyridin-4-yl)amino)methyl)-N-((4-methylmorpholin-2-yl) methyl)benzamide (Enantiomer 2);
3-(((7-(2-aminopyrimidin-4-yl)-2,3-dihydrofuro[3,2-c]pyridin-4-yl)amino)methyl)-N-(3-methoxy-3-methylbutyl) benzamide;
3-(((7-(2-aminopyrimidin-4-yl)-2,3-dihydrofuro[3,2-c]pyridin-4-yl)amino)methyl)-N-((1R,2S)-2-fluorocyclopropyl)benzamide;
3-(((7-(2-aminopyrimidin-4-yl)-2,3-dihydrofuro[3,2-c]pyridin-4-yl)amino)methyl)-N-(oxetan-2-ylmethyl)benzamide;
3-(((7-(2-aminopyrimidin-4-yl)-2,3-dihydrofuro[3,2-c]pyridin-4-yl)amino)methyl)-N-(((1r,3r)-3-methoxycyclobutyl)methyl)benzamide;
3-(((7-(2-aminopyrimidin-4-yl)-2,3-dihydrofuro[3,2-c]pyridin-4-yl)amino)methyl)-N-propylbenzamide;
3-(((7-(2-aminopyrimidin-4-yl)-2,3-dihydrofuro[3,2-c]pyridin-4-yl)amino)methyl)-N-((1r,3r)-3-methoxycyclobutyl)benzamide;
(R)-3-(((7-(2-aminopyrimidin-4-yl)-2,3-dihydrofuro[3,2-c]pyridin-4-yl)amino)methyl)-N-((tetrahydrofuran-2-yl) methyl)benzamide;
(S)-3-(((7-(2-aminopyrimidin-4-yl)-2,3-dihydrofuro[3,2-c]pyridin-4-yl)amino)methyl)-N-((tetrahydrofuran-2-yl) methyl)benzamide;
3-(((7-(2-aminopyrimidin-4-yl)-2,3-dihydrofuro[3,2-c]pyridin-4-yl)amino)methyl)-N-(2,2-difluoropropyl)benzamide;

Trans 3-(((7-(2-aminopyrimidin-4-yl)-2,3-dihydrofuro[3,2-c]pyridin-4-yl)amino)methyl)-N-(2-fluorocyclopropyl) benzamide (Enantiomer 1);
Trans 3-(((7-(2-aminopyrimidin-4-yl)-2,3-dihydrofuro[3,2-c]pyridin-4-yl)amino)methyl)-N-(2-fluorocyclopropyl) benzamide (Enantiomer 2);
3-(((7-(2-aminopyrimidin-4-yl)-2,3-dihydrofuro[3,2-c]pyridin-4-yl)amino)methyl)-N-((1S,2R)-2-fluorocyclopropyl)benzamide;
3-(((7-(2-aminopyrimidin-4-yl)-2,3-dihydrofuro[3,2-c]pyridin-4-yl)amino)methyl)-N-((1s,3 s)-3-fluorocyclobutyl) benzamide;
3-(((7-(2-aminopyrimidin-4-yl)-2,3-dihydrofuro[3,2-c]pyridin-4-yl)amino)methyl)-N-((1r,3r)-3-fluorocyclobutyl) benzamide;
3-(((7-(2-aminopyrimidin-4-yl)-2,3-dihydrofuro[3,2-c]pyridin-4-yl)amino)methyl)-N-(3,3-difluoropropyl)benzamide;
3-(((7-(2-aminopyrimidin-4-yl)-2,3-dihydrofuro[3,2-c]pyridin-4-yl)amino)methyl)-N-(5-methoxypyridin-2-yl)benzamide;
3-(((7-(2-aminopyrimidin-4-yl)-2,3-dihydrofuro[3,2-c]pyridin-4-yl)amino)methyl)-N-(5-(2-(dimethylamino) ethoxy)pyridin-2-yl)benzamide;
3-(((7-(2-aminopyrimidin-4-yl)-2,3-dihydrofuro[3,2-c]pyridin-4-yl)amino)methyl)-N-(5-(piperazin-1-yl)pyridin-2-yl)benzamide;
3-(((7-(2-aminopyrimidin-4-yl)-2,3-dihydrofuro[3,2-c]pyridin-4-yl)amino)methyl)-2-fluoro-N-methylbenzamide;
3-(((7-(2-aminopyrimidin-4-yl)-2,3-dihydrofuro[3,2-c]pyridin-4-yl)amino)methyl)-2-fluoro-N-(3-methoxypropyl) benzamide;
3-(((7-(2-aminopyrimidin-4-yl)-2,3-dihydrofuro[3,2-c]pyridin-4-yl)amino)methyl)-2-fluoro-N-(tetrahydro-2H-pyran-4-yl)benzamide;
5-(((7-(2-aminopyrimidin-4-yl)-2,3-dihydrofuro[3,2-c]pyridin-4-yl)amino)methyl)-2-fluoro-N-(3-methoxypropyl) benzamide;
5-(((7-(2-aminopyrimidin-4-yl)-2,3-dihydrofuro[3,2-c]pyridin-4-yl)amino)methyl)-2-fluoro-N-(tetrahydro-2H-pyran-4-yl)benzamide;
3-(((7-(2-aminopyrimidin-4-yl)-2,3-dihydrofuro[3,2-c]pyridin-4-yl)amino)methyl)-5-fluoro-N-methylbenzamide;
3-(((7-(2-aminopyrimidin-4-yl)-2,3-dihydrofuro[3,2-c]pyridin-4-yl)amino)methyl)-5-fluoro-N-(3-methoxypropyl) benzamide;
3-(((7-(2-aminopyrimidin-4-yl)-2,3-dihydrofuro[3,2-c]pyridin-4-yl)amino)methyl)-4-fluoro-N-(tetrahydro-2H-pyran-4-yl)benzamide;
3-(((7-(2-aminopyrimidin-4-yl)-2,3-dihydrofuro[3,2-c]pyridin-4-yl)amino)methyl)-4-fluoro-N-(3-methoxypropyl) benzamide;
6-(((7-(2-aminopyrimidin-4-yl)-2,3-dihydrofuro[3,2-c]pyridin-4-yl)amino)methyl)-N-methylpicolinamide;
N-(3-(((7-(2-aminopyrimidin-4-yl)-2,3-dihydrofuro[3,2-c]pyridin-4-yl)amino)methyl)phenyl)-4-methoxybutanamide;
single enantiomers, diastereoisomers and mixtures thereof in any proportion,
or pharmaceutically acceptable salts and solvates thereof.

**9.** A pharmaceutical composition comprising a compound as defined in any one of claims 1 to 8, or a pharmaceutically acceptable salt thereof, in admixture with one or more pharmaceutically acceptable carrier or excipient.

**10.** A pharmaceutical composition according to claim 9 suitable to be administered by inhalation, selected from inhalable powders, propellant-containing metering aerosols or propellant-free inhalable formulations.

**11.** A device comprising the pharmaceutical composition according to claim 10, which may be a single- or multi-dose dry powder inhaler, a metered dose inhaler or a soft mist nebulizer.

**12.** A pharmaceutical composition according to claim 9 suitable to be administered by oral route, selected from, gelcaps, capsules, caplets, granules, lozenges and bulk powders or aqueous and non-aqueous solutions, emulsions, suspensions, syrups, or elixirs formulations.

**13.** A compound or a pharmaceutical composition according to any one of claims 1 to 8 or 9 for use as a medicament.

**14.** A compound or a pharmaceutical composition for use according to claim 13, in the prevention and /or treatment of immune system disorders including Graft-versus-host disease (GVHD), and pulmonary diseases selected from the group consisting of asthma, chronic obstructive pulmonary disease COPD, idiopathic pulmonary fibrosis (IPF),

pulmonary hypertension (PH) and specifically Pulmonary Arterial Hypertension (PAH).

**15.** A compound or a pharmaceutical composition for use according to claim 14 for use via oral route of administration particularly in the prevention and /or treatment of asthma, chronic obstructive pulmonary disease COPD, idiopathic pulmonary fibrosis (IPF), pulmonary hypertension (PH) and specifically Pulmonary Arterial Hypertension (PAH).

**16.** A combination of a compound as defined in any one of the claims 1 to 8 with one or more active ingredients selected from the classes consisting of organic nitrates and NO donors; inhaled NO; stimulator of soluble guanylate cyclase (sGC); prostaciclin analogue PGI2 and agonist of prostacyclin receptors; compounds that inhibit the degradation of cyclic guanosine monophosphate (cGMP) and/or cyclic adenosine monophosphate (cAMP); human neutrophilic elastase inhibitors; compounds inhibiting the signal transduction cascade; active substances for lowering blood pressure; neutral endopeptidase inhibitor; osmotic agents; ENaC blockers; antiinflammatories including corticosteroids and antagonists of chemokine receptors; antihistamine drugs; anti-tussive drugs; antibiotics and DNase drug substance and selective cleavage agents; agents that inhibit ALK5 and/or ALK4 phosphorylation of Smad2 and Smad3; tryptophan hydroylase 1 (TPH1) inhibitors and multi-kinase inhibitors, beta2-agonists, corticosteroids, anticholinergic or antimuscarinic agents, mitogen-activated protein kinases (P38 MAP kinase) inhibitors, nuclear factor kappa-B kinase subunit beta (IKK2) inhibitors, leukotriene modulators, non-steroidal anti-inflammatory agents (NSAIDs), mucus regulators, mucolytics, expectorant/mucokinetic modulators, peptide mucolytics, inhibitors of JAK, SYK inhibitors, inhibitors of PI3Kdelta or PI3Kgamma and combinations thereof.

## Patentansprüche

**1.** Verbindung der Formel (I),

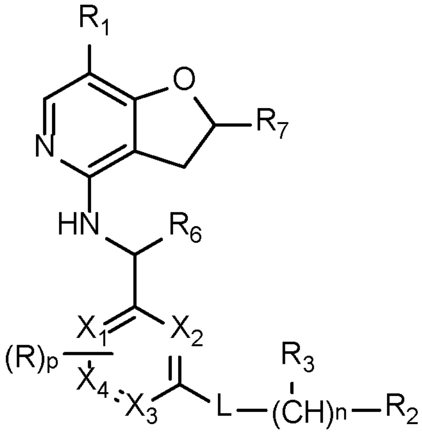

(I)

worin

**$X_1$, $X_2$, $X_3$** und **$X_4$** alle CH oder eines von $X_1$, $X_2$, $X_3$ sind und $X_4$ N ist und die anderen CH sind;
p Null oder eine ganze Zahl von 1 bis 4 ist;
jeder R, wenn vorhanden, bei jedem Vorkommen unabhängig ausgewählt ist aus ($C_1$-$C_6$)-Alkyl und Halogen, ausgewählt aus F, Cl, Br und I; wobei R vorzugsweise F, Cl oder Methyl ist;
$R_1$ Pyrimidinyl ist, vorzugsweise Pyrimidin-4yl, substituiert durch eine oder mehrere Gruppe ausgewählt aus $-(CH_2)_mNH_2$; wobei besonders bevorzugt $R_1$ 2-Aminopyrimidin-4-yl ist;
L -C(O)NH- oder -NHC(O)- ist;
n bei jedem Vorkommen unabhängig 0 oder eine ganze Zahl ausgewählt aus 1, 2 oder 3 ist;
$R_2$ und $R_3$ bei jedem Vorkommen unabhängig ausgewählt sind aus der Gruppe bestehend aus
-H,
Halogen,
-OH,
$-(CH2)_mNR_4R_5$,

$(C_1-C_6)$-Alkyl,
$(C_1-C_6)$-Hydroxyalkyl,
$(C_1-C_6)$-Alkoxy,
$(C_1-C_6)$-Alkoxy-$(C_1-C_6)$-alkyl,
$(C_1-C_6)$-Halogenalkyl,
$(C_1-C_6)$-Halogenalkoxy,
$(C_1-C_6)$-Halogenalkoxy-$(C_1-C_6)$-alkyl,
$(C_3-C_{10})$-Cycloalkyl,
Aryl und $(C_3-C_6)$-Heterocycloalkyl,
wobei jedes Cycloalkyl, Aryl und Heterocycloalkyl
wiederum optional und unabhängig substituiert ist mit einer oder mehreren Gruppen ausgewählt aus
Halogen,
-OH,
$(C_1-C_6)$-Alkyl,
$(C_1-C_6)$-Hydroxyalkyl,
$(C_1-C_6)$-Alkoxy,
$(C_1-C_6)$-Alkoxy-$(C_1-C_6)$-alkyl,
$(C_1-C_6)$-Halogenalkyl,
$(C_1-C_6)$-Halogenalkoxy,
$-(CH2)_mNR_4R_5$,
$-O-(CH_2)_mNR_4R_5$,
Alkanoyl,
Aryl, Heteroaryl, Cycloalkyl,
Aryl-$(C_1-C_6)$-alkyl,
Heteroaryl-$(C_1-C_6)$-alkyl,
$(C_3-C_8)$-Heterocycloalkyl,
$(C_3-C_8)$-Heterocycloalkyl-$(C_1-C_6)$-alkyl,
$(C_3-C_8)$-Cycloalkyl-$(C_1-C_6)$-alkyl,
wobei jedes von dem Aryl, Heteroaryl, Cycloalkyl und Heterocycloalkyl optional noch weiter durch eine oder mehrere Gruppen substituiert ist, unabhängig ausgewählt aus Halogen, ausgewählt aus F, Cl, Br und I, vorzugsweise F, -OH, $(C_1-C_8)$-Alkyl, $(C_1-C_6)$-Halogenalkyl, $(C_1-C_6)$-Hydroxyalkyl,
m bei jedem Vorkommen unabhängig 0 oder eine ganze Zahl ausgewählt aus 1, 2 oder 3 ist;
$R_4$ und $R_5$, gleich oder verschieden, aus der Gruppe ausgewählt sind, bestehend aus:
-H,
$(C_1-C_6)$-Alkyl,
$(C_1-C_6)$-Halogenalkyl,
$(C_1-C_6)$-Hydroxyalkyl,
$(C_3-C_6)$-Heterocycloalkyl;
$R_6$ und $R_7$ unabhängig ausgewählt sind aus der Gruppe bestehend aus -H, $(C_1-C_6)$-Alkyl;
wobei, wo nicht bereits spezifiziert, Aryl sich auf mono-, bi- oder tricyclische Ringsysteme bezieht, die 6 bis 20 Ringatome aufweisen, in denen mindestens ein Ring aromatisch ist; Heteroaryl sich auf mono-, bi- oder tricyclische Ringsysteme mit 5 bis 20 Ringatomen bezieht, in denen mindestens ein Ring aromatisch ist und in denen mindestens ein Ringatom ein Heteroatom ausgewählt aus N, S oder O ist; $(C_3-C_6)$-Heterocycloalkyl sich auf gesättigte oder teilweise ungesättigte monocyclische $(C_3-C_6)$-Cycloalkylgruppen bezieht, in denen mindestens ein Ringkohlenstoffatom durch mindestens ein Heteroatom ausgewählt aus N, NH, S oder O ersetzt ist und/oder eine Oxo-Substituentengruppe tragen kann;
einzelnen Enantiomeren, Diastereoisomeren und Mischungen davon in beliebigem Verhältnis und pharmazeutisch verträglichen Salzen und Solvaten davon.

**2.** Verbindung nach Anspruch 1,

wobei $X_1$, $X_3$ und $X_4$ alle CH-Gruppen sind und $X_2$ eine CH-Gruppe oder ein Stickstoffatom ist;
$R_1$ 2-Aminopyrimidin-4-yl ist;
alle der anderen Variablen wie in Anspruch 1 definiert sind.

**3.** Verbindung nach Anspruch 1,

worin $X_1$, $X_2$, $X_3$, $X_4$ alle CH-Gruppe sind;

jeder R, wenn vorhanden, bei jedem Vorkommen unabhängig ausgewählt ist aus F, Cl, Br und I;
$R_1$ Pyrimidinyl ist, substituiert durch $-NH_2$;
L -C(O)NH- ist;
n 0 ist;
$R_2$ ausgewählt ist aus der Gruppe bestehend aus
$(C_1-C_6)$-Alkyl,
$(C_1-C_6)$-Hydroxyalkyl,
$(C_1-C_6)$-Halogenalkyl,
$(C_1-C_6)$-Alkoxy-$(C_1-C_6)$-alkyl,
$(C_1-C_6)$-Halogenalkoxy-$(C_1-C_6)$-alkyl,
alle der anderen Variablen wie in Anspruch 1 definiert sind,
einzelnen Enantiomeren, Diastereoisomeren und Mischungen davon in beliebigem Verhältnis und/oder oder pharmazeutisch verträglichen Salzen und Solvaten davon.

**4.** Verbindung nach Anspruch 3,

worin $R_2$ ausgewählt ist aus Methyl, Ethyl, Propyl, 2-Hydroxy-2-methylpropyl, 3-Methoxypropyl, 2-Methoxyethyl, 2-Ethoxyethyl, 3-Isopropoxypropyl, 3-Methoxy-3-methylbutyl, 2-Methoxy-2-methylpropyl, 3-Fluorpropyl, 2-Fluor-2-methylpropyl, 2,2-Difluorpropyl, 3,3-Difluorpropyl,
einzelnen Enantiomeren, Diastereoisomeren und Mischungen davon in beliebigem Verhältnis und/oder pharmazeutisch verträglichen Salzen und Solvaten davon

**5.** Verbindung nach Anspruch 1,

worin $\mathbf{X_1}$, $\mathbf{X_2}$, $\mathbf{X_3}$ und $\mathbf{X_4}$ alle CH sind;
p Null oder eine ganze Zahl von 1 bis 4 ist;
jeder R, wenn vorhanden, bei jedem Vorkommen Halogen ist, unabhängig ausgewählt aus F, Cl, Br und I, wobei R vorzugsweise F ist;
$R_1$ Pyrimidinyl ist, substituiert durch $-NH_2$; wobei besonders bevorzugt $R_1$ 2-Aminopyrimidin-4-yl ist;
L -C(O)NH- ist;
n bei jedem Vorkommen unabhängig 0 oder eine ganze Zahl ausgewählt aus 1, 2 oder 3 ist;
$R_3$, wenn vorhanden, H ist,
und
$R_2$ $(C_3-C_6)$-Heterocycloalkyl ist,
das
wiederum optional mit einer oder mehreren Gruppen substituiert ist, ausgewählt aus
$(C_1-C_6)$-Alkyl,
$(C_1-C_6)$-Hydroxyalkyl,
$(C_1-C_6)$-Alkoxy,
$(C_1-C_6)$-Alkoxy-$(C_1-C_6)$-alkyl,
Heteroaryl-$(C_1-C_6)$-alkyl,
$(C_3-C_8)$-Cycloalkyl-$(C_1-C_6)$-alkyl,
$-O-(CH_2)_mNR_4R_5$,
$(C_3-C_6)$-Heterocycloalkyl m bei jedem Vorkommen unabhängig 0 oder eine ganze Zahl ausgewählt aus 1, 2 oder 3 ist;
$R_4$ und $R_5$, gleich oder verschieden, aus der Gruppe ausgewählt werden, bestehend aus:
-H,
$(C_1-C_6)$-Alkyl,
$(C_1-C_6)$-Halogenalkyl,
$(C_1-C_6)$-Hydroxyalkyl,
alle der anderen Variablen wie in Anspruch 1 definiert sind,
einzelnen Enantiomeren, Diastereoisomeren und Mischungen davon in beliebigem Verhältnis und/oder pharmazeutisch verträglichen Salzen und Solvaten davon.

**6.** Verbindung gemäß Anspruch 5, wobei $R_2$ ausgewählt ist aus Tetrahydrofuranyl, Dimethyltetrahydrofuranyl, Oxetan-2-yl.

**7.** Verbindung der Formel (I) nach Anspruch 1, worin

**$X_1$, $X_2$, $X_3$** und **$X_4$** alle CH oder $X_2$ sind, ist N und die anderen sind CH;
p Null oder 1 ist;
jeder R, wenn vorhanden, F ist;
$R_1$ **2-Aminopyrimidin-4-yl** ist;
L -C(O)NH- oder -NHC(O)- ist;
n bei jedem Vorkommen unabhängig 0 oder eine ganze Zahl ausgewählt aus 1, 2 oder 3 ist;
$R_2$ und $R_3$ bei jedem Vorkommen unabhängig ausgewählt sind aus der Gruppe bestehend aus
-H,
($C_1$-$C_6$)-Alkyl, das Methyl, Propyl, Isopropyl, tert-Butyl ist,
($C_1$-$C_6$)-Hydroxyalkyl, das 2-Hydroxy-2-methylpropyl ist,
($C_1$-$C_6$)-Alkoxy-($C_1$-$C_6$)-alkyl, das 3-Methoxypropyl, 2-Ethoxyethyl, 3-Isopropoxypropyl, 3-Methoxy-3-methylbutyl, 2-Methoxyethyl, 2-Methoxy-2-methylpropyl ist,
($C_1$-$C_6$)-Halogenalkyl, das 2,2-Difluorethyl, 2,2,2-Trifluorethyl, 2,2,2-Trifluormethyl, 2-Fluor-2-methylpropyl, 3-Fluorpropyl, 2,2-Difluorpropyl, 3,3-Difluorpropyl ist,
($C_1$-$C_6$)-Halogenalkoxy-($C_1$-$C_6$)-alkyl, das 3-(Difluormethoxy)propyl ist,
($C_3$-$C_{10}$)-Cycloalkyl, das Cyclopropyl, Cyclobutyl ist,
Heteroaryl, das Pyridinyl ist, und
und
($C_3$-$C_6$)-Heterocycloalkyl, das Azetidin-3-yl, Piperidinyl, Morpholinyl, 5-Oxopyrrolidin-3-yl, (1R,5S,6r)-3-Oxabicyclo[3.1.0]hexan-6-yl, Tetrahydrofuranyl, Tetrahydro-2H-pyran-4-yl ist,
wobei jedes von dem Cycloalkyl, Heteroaryl und Heterocycloalkyl
wiederum optional und unabhängig substituiert ist mit einer oder mehreren Gruppen ausgewählt aus
Halogen, das ausgewählt ist aus F, Cl, Br, I,
($C_1$-$C_6$)-Alkyl, das Methyl, tert-Butyl ist,
($C_1$-$C_6$)-Alkoxy, das Methoxy ist,
($C_1$-$C_6$)-Alkoxy-($C_1$-$C_6$)-alkyl, das Methoxymethyl ist,
$R_6$ und $R_7$ -H sind,
einzelnen Enantiomeren, Diastereoisomeren und Mischungen davon in beliebigem Verhältnis,
oder pharmazeutisch verträglichen Salzen und Solvaten davon.

**8.** Verbindung nach Anspruch 1, ausgewählt aus:

3-(((7-(2-Aminopyrimidin-4-yl)-2,3-dihydrofuro[3,2-c]pyridin-4-yl)amino)methyl)-N-cyclobutylbenzamid;
3-(((7-(2-Aminopyrimidin-4-yl)-2,3-dihydrofuro[3,2-c]pyridin-4-yl)amino)methyl)-N-((tetrahydro-2H-pyran-4-yl)methyl)benzamid;
3-(((7-(2-Aminopyrimidin-4-yl)-2,3-dihydrofuro[3,2-c]pyridin-4-yl)amino)methyl)-N-(5,5-dimethyltetrahydrofuran-3-yl)benzamid;
3-(((7-(2-Aminopyrimidin-4-yl)-2,3-dihydrofuro[3,2-c]pyridin-4-yl)amino)methyl)-N-cyclopropylbenzamid;
3-(((7-(2-Aminopyrimidin-4-yl)-2,3-dihydrofuro[3,2-c]pyridin-4-yl)amino)methyl)-N-(2-(piperidin-1-yl)ethyl)benzamid;
3-(((7-(2-Aminopyrimidin-4-yl)-2,3-dihydrofuro[3,2-c]pyridin-4-yl)amino)methyl)-N-(3-(methoxy-d3)propyl)benzamid;
3-(((7-(2-Aminopyrimidin-4-yl)-2,3-dihydrofuro[3,2-c]pyridin-4-yl)amino)methyl)-N-(tetrahydro-2H-pyran-4-yl)benzamid;
3-(((7-(2-Aminopyrimidin-4-yl)-2,3-dihydrofuro[3,2-c]pyridin-4-yl)amino)methyl)-N-(3-methoxypropyl)benzamid;
3-(((7-(2-Aminopyrimidin-4-yl)-2,3-dihydrofuro[3,2-c]pyridin-4-yl)amino)methyl)-N-(2-methoxyethyl)benzamid;
3-(((7-(2-Aminopyrimidin-4-yl)-2,3-dihydrofuro[3,2-c]pyridin-4-yl)amino)methyl)-N-(3,3-difluorcyclobutyl)benzamid;
3-(((7-(2-Aminopyrimidin-4-yl)-2,3-dihydrofuro[3,2-c]pyridin-4-yl)amino)methyl)-N-isopropylbenzamid;
3-(((7-(2-Aminopyrimidin-4-yl)-2,3-dihydrofuro[3,2-c]pyridin-4-yl)amino)methyl)-N-(2,2,2-trifluorethyl)benzamid;
3-(((7-(2-Aminopyrimidin-4-yl)-2,3-dihydrofuro[3,2-c]pyridin-4-yl)amino)methyl)-N-(1-methylpiperidin-4-yl)benzamid;
3-(((7-(2-Aminopyrimidin-4-yl)-2,3-dihydrofuro[3,2-c]pyridin-4-yl)amino)methyl)-N-(3-(difluormethoxy)propyl)benzamid;
3-(((7-(2-Aminopyrimidin-4-yl)-2,3-dihydrofuro[3,2-c]pyridin-4-yl)amino)methyl)-N-(3-isopropoxypropyl)benzamid;

3-(((7-(2-Aminopyrimidin-4-yl)-2,3-dihydrofuro[3,2-c]pyridin-4-yl)amino)methyl)-N-(3-morpholinopropyl)benzamid;
3-(((7-(2-Aminopyrimidin-4-yl)-2,3-dihydrofuro[3,2-c]pyridin-4-yl)amino)methyl)-N-(2-hydroxy-2-methylpropyl) benzamid;
3-(((7-(2-Aminopyrimidin-4-yl)-2,3-dihydrofuro[3,2-c]pyridin-4-yl)amino)methyl)-N-((1s,3s)-3-methoxycyclobutyl)benzamid;
3-(((7-(2-Aminopyrimidin-4-yl)-2,3-dihydrofuro[3,2-c]pyridin-4-yl)amino)methyl)-N-(2-((2R,6S)-2,6-dimethylpiperidin-1-yl)ethyl)benzamid;
3-(((7-(2-Aminopyrimidin-4-yl)-2,3-dihydrofuro[3,2-c]pyridin-4-yl)amino)methyl)-N-(2-ethoxyethyl)benzamid;
3-(((7-(2-Aminopyrimidin-4-yl)-2,3-dihydrofuro[3,2-c]pyridin-4-yl)amino)methyl)-N-methylbenzamid;
3-(((7-(2-Aminopyrimidin-4-yl)-2,3-dihydrofuro[3,2-c]pyridin-4-yl)amino)methyl)-N-(1-methylcyclopropyl)benzamid;
3-(((7-(2-Aminopyrimidin-4-yl)-2,3-dihydrofuro[3,2-c]pyridin-4-yl)amino)methyl)-N-((1R,5S,6r)-3-oxabicyclo [3.1.0]hexan-6-yl)benzamid;
3-(((7-(2-Aminopyrimidin-4-yl)-2,3-dihydrofuro[3,2-c]pyridin-4-yl)amino)methyl)-N-(1-(oxetan-3-yl)azetidin-3-yl)benzamid;
3-(((7-(2-Aminopyrimidin-4-yl)-2,3-dihydrofuro[3,2-c]pyridin-4-yl)amino)methyl)-N-(cyclopropylmethyl)benzamid;
3-(((7-(2-Aminopyrimidin-4-yl)-2,3-dihydrofuro[3,2-c]pyridin-4-yl)amino)methyl)-N-((1s,3s)-3-(difluormethoxy) cyclobutyl)benzamid;
3-(((7-(2-Aminopyrimidin-4-yl)-2,3-dihydrofuro[3,2-c]pyridin-4-yl)amino)methyl)-N-(2-methoxy-2-methylpropyl) benzamid;
3-(((7-(2-Aminopyrimidin-4-yl)-2,3-dihydrofuro[3,2-c]pyridin-4-yl)amino)methyl)-N-((1-(methoxymethyl)cyclopropyl)methyl)benzamid;
3-(((7-(2-Aminopyrimidin-4-yl)-2,3-dihydrofuro[3,2-c]pyridin-4-yl)amino)methyl)-N-(3-fluorpropyl)benzamid;
3-(((7-(2-Aminopyrimidin-4-yl)-2,3-dihydrofuro[3,2-c]pyridin-4-yl)amino)methyl)-N-((1-methylcyclopropyl)methyl)benzamid;
3-(((7-(2-Aminopyrimidin-4-yl)-2,3-dihydrofuro[3,2-c]pyridin-4-yl)amino)methyl)-N-(2-fluor-2-methylpropyl) benzamid;
3-(((7-(2-Aminopyrimidin-4-yl)-2,3-dihydrofuro[3,2-c]pyridin-4-yl)amino)methyl)-N-((1-(tert-butyl)-5-oxopyrrolidin-3-yl)methyl)benzamid (Enantiomer 1);
3-(((7-(2-Aminopyrimidin-4-yl)-2,3-dihydrofuro[3,2-c]pyridin-4-yl)amino)methyl)-N-((1-(tert-butyl)-5-oxopyrrolidin-3-yl)methyl)benzamid (Enantiomer 2);
3-(((7-(2-Aminopyrimidin-4-yl)-2,3-dihydrofuro[3,2-c]pyridin-4-yl)amino)methyl)-N-((4-methylmorpholin-2-yl) methyl)benzamid (Enantiomer 1);
3-(((7-(2-Aminopyrimidin-4-yl)-2,3-dihydrofuro[3,2-c]pyridin-4-yl)amino)methyl)-N-((4-methylmorpholin-2-yl) methyl)benzamid (Enantiomer 2);
3-(((7-(2-Aminopyrimidin-4-yl)-2,3-dihydrofuro[3,2-c]pyridin-4-yl)amino)methyl)-N-(3-methoxy-3-methylbutyl) benzamid;
3-(((7-(2-Aminopyrimidin-4-yl)-2,3-dihydrofuro[3,2-c]pyridin-4-yl)amino)methyl)-N-((1R,2S)-2-fluorcyclopropyl) benzamid;
3-(((7-(2-Aminopyrimidin-4-yl)-2,3-dihydrofuro[3,2-c]pyridin-4-yl)amino)methyl)-N-(oxetan-2-ylmethyl)benzamid;
3-(((7-(2-Aminopyrimidin-4-yl)-2,3-dihydrofuro[3,2-c]pyridin-4-yl)amino)methyl)-N-(((1r,3r)-3-methoxycyclobutyl)methyl)benzamid;
3-(((7-(2-Aminopyrimidin-4-yl)-2,3-dihydrofuro[3,2-c]pyridin-4-yl)amino)methyl)-N-propylbenzamid;
3-(((7-(2-Aminopyrimidin-4-yl)-2,3-dihydrofuro[3,2-c]pyridin-4-yl)amino)methyl)-N-((1r,3r)-3-methoxycyclobutyl)benzamid;
(R)-3-(((7-(2-Aminopyrimidin-4-yl)-2,3-dihydrofuro[3,2-c]pyridin-4-yl)amino)methyl)-N-((tetrahydrofuran-2-yl) methyl)benzamid;
(S)-3-(((7-(2-Aminopyrimidin-4-yl)-2,3-dihydrofuro[3,2-c]pyridin-4-yl)amino)methyl)-N-((tetrahydrofuran-2-yl) methyl)benzamid;
3-(((7-(2-Aminopyrimidin-4-yl)-2,3-dihydrofuro[3,2-c]pyridin-4-yl)amino)methyl)-N-(2,2-difluorpropyl)benzamid;
trans-3-(((7-(2-Aminopyrimidin-4-yl)-2,3-dihydrofuro[3,2-c]pyridin-4-yl)amino)methyl)-N-(2-fluorcyclopropyl) benzamid (Enantiomer 1);
trans-3-(((7-(2-Aminopyrimidin-4-yl)-2,3-dihydrofuro[3,2-c]pyridin-4-yl)amino)methyl)-N-(2-fluorcyclopropyl) benzamid (Enantiomer 2);

3-(((7-(2-Aminopyrimidin-4-yl)-2,3-dihydrofuro[3,2-c]pyridin-4-yl)amino)methyl)-N-((1S,2R)-2-fluorcyclopropyl) benzamid;
3-(((7-(2-Aminopyrimidin-4-yl)-2,3-dihydrofuro[3,2-c]pyridin-4-yl)amino)methyl)-N-((1s,3s)-3-fluorcyclobutyl) benzamid;
3-(((7-(2-Aminopyrimidin-4-yl)-2,3-dihydrofuro[3,2-c]pyridin-4-yl)amino)methyl)-N-((1r,3r)-3-fluorcyclobutyl) benzamid;
3-(((7-(2-Aminopyrimidin-4-yl)-2,3-dihydrofuro[3,2-c]pyridin-4-yl)amino)methyl)-N-(3,3-difluorpropyl)benzamid;
3-(((7-(2-Aminopyrimidin-4-yl)-2,3-dihydrofuro[3,2-c]pyridin-4-yl)amino)methyl)-N-(5-methoxypyridin-2-yl) benzamid;
3-(((7-(2-Aminopyrimidin-4-yl)-2,3-dihydrofuro[3,2-c]pyridin-4-yl)amino)methyl)-N-(5-(2-(dimethylamino)ethoxy)pyridin-2-yl)benzamid;
3-(((7-(2-Aminopyrimidin-4-yl)-2,3-dihydrofuro[3,2-c]pyridin-4-yl)amino)methyl)-N-(5-(piperazin-1-yl)pyridin-2-yl)benzamid;
3-(((7-(2-Aminopyrimidin-4-yl)-2,3-dihydrofuro[3,2-c]pyridin-4-yl)amino)methyl)-2-fluor-N-methylbenzamid;
3-(((7-(2-Aminopyrimidin-4-yl)-2,3-dihydrofuro[3,2-c]pyridin-4-yl)amino)methyl)-2-fluor-N-(3-methoxypropyl) benzamid;
3-(((7-(2-Aminopyrimidin-4-yl)-2,3-dihydrofuro[3,2-c]pyridin-4-yl)amino)methyl)-2-fluor-N-(tetrahydro-2H-pyran-4-yl)benzamid;
5-(((7-(2-Aminopyrimidin-4-yl)-2,3-dihydrofuro[3,2-c]pyridin-4-yl)amino)methyl)-2-fluor-N-(3-methoxypropyl) benzamid;
5-(((7-(2-Aminopyrimidin-4-yl)-2,3-dihydrofuro[3,2-c]pyridin-4-yl)amino)methyl)-2-fluor-N-(tetrahydro-2H-pyran-4-yl)benzamid;
3-(((7-(2-Aminopyrimidin-4-yl)-2,3-dihydrofuro[3,2-c]pyridin-4-yl)amino)methyl)-5-fluor-N-methylbenzamid;
3-(((7-(2-Aminopyrimidin-4-yl)-2,3-dihydrofuro[3,2-c]pyridin-4-yl)amino)methyl)-5-fluor-N-(3-methoxypropyl) benzamid;
3-(((7-(2-Aminopyrimidin-4-yl)-2,3-dihydrofuro[3,2-c]pyridin-4-yl)amino)methyl)-4-fluor-N-(tetrahydro-2H-pyran-4-yl)benzamid;
3-(((7-(2-Aminopyrimidin-4-yl)-2,3-dihydrofuro[3,2-c]pyridin-4-yl)amino)methyl)-4-fluor-N-(3-methoxypropyl) benzamid;
6-(((7-(2-Aminopyrimidin-4-yl)-2,3-dihydrofuro[3,2-c]pyridin-4-yl)amino)methyl)-N-methylpicolinamid;
N-(3-(((7-(2-Aminopyrimidin-4-yl)-2,3-dihydrofuro[3,2-c]pyridin-4-yl)amino)methyl)phenyl)-4-methoxybutanamid;
einzelnen Enantiomeren, Diastereoisomeren und Mischungen davon in beliebigem Verhältnis oder pharmazeutisch verträglichen Salzen und Solvaten davon.

**9.** Pharmazeutische Zusammensetzung, umfassend eine Verbindung wie nach einem der Ansprüche 1 bis 8 definiert oder ein pharmazeutisch verträgliches Salz davon, in Beimischung mit einem oder mehreren pharmazeutisch verträglichen Trägern oder Hilfsstoffen.

**10.** Zur Verabreichung durch Inhalation geeignete pharmazeutische Zusammensetzung nach Anspruch 9, ausgewählt aus inhalierbaren Pulvern, treibgashaltigen Dosieraerosolen oder treibgasfreien inhalierbaren Formulierungen.

**11.** Vorrichtung, umfassend die pharmazeutische Zusammensetzung nach Anspruch 10, die ein Trockenpulverinhalator für Einzel- oder Mehrfachdosen, ein Dosierinhalator oder ein Zerstäuber für sanftem Nebel sein kann.

**12.** Pharmazeutische Zusammensetzung nach Anspruch 9, die geeignet ist, um durch oralen Weg verabreicht zu werden und aus Gelkapseln, Kapseln, Dragees, Granulat, Lutschtabletten und losen Pulvern oder wässrigen und nichtwässrigen Lösungen, Emulsionen, Suspensionen, Sirupen oder Elixirformulierungen ausgewählt ist.

**13.** Verbindung oder eine pharmazeutische Zusammensetzung nach einem der Ansprüche 1 bis 8 oder 9 zur Verwendung als ein Medikament.

**14.** Verbindung oder eine pharmazeutische Zusammensetzung zur Verwendung nach Anspruch 13 bei der Vorbeugung und/oder Behandlung von Störungen des Immunsystems, einschließlich der Graft-versus-Host-Krankheit (GVHD) und Lungenkrankheiten, ausgewählt aus der Gruppe bestehend aus Asthma, chronisch obstruktiver Lungenkrankheit COPD, idiopathischer Lungenfibrose (IPF), Lungenhochdruck (PH) und spezifisch arteriellem Lungenhochdruck (PAH).

**15.** Verbindung oder eine pharmazeutische Zusammensetzung zur Verwendung nach Anspruch 14 zur Verwendung mittels oralem Verabreichungsweg, besonders bei der Vorbeugung und/oder Behandlung von Asthma, chronisch obstruktiver Lungenkrankheit COPD, idiopathischer Lungenfibrose (IPF), Lungenhochdruck (PH) und insbesondere arteriellem Lungenhochdruck (PAH).

**16.** Kombination einer Verbindung wie nach einem der Ansprüche 1 bis 8 definiert mit einem oder mehreren aktiven Inhaltsstoffen ausgewählt aus den Klassen bestehend aus organischen Nitraten und NO-Donatoren; inhaliertem NO; Stimulator der löslichen Guanylatcyclase (sGC); Prostacyclinanalogon PGI2 und Agonist von Prostacyclinrezeptoren; Verbindungen, die den Abbau von cyclischem Guanosinmonophosphat (cGMP) und/oder cyclischem Adenosinmonophosphat (cAMP) hemmen; Inhibitoren der humanen neutrophilen Elastase; Verbindungen, die die Signaltransduktionskaskade hemmen; Wirkstoffen zum Senken des Blutdrucks; neutralem Endopeptidase-Inhibitor; osmotischen Mitteln; ENaC-Blockern; Entzündungshemmern einschließlich Kortikosteroiden und Antagonisten von Chemokinrezeptoren; Antihistaminika; hustenstillenden Arzneimitteln; Antibiotika und DNase-Arzneimittelstoff und selektiven Spaltmitteln; Mitteln, die die ALK5- und/oder ALK4-Phosphorylierung von Smad2 und Smad3 hemmen; Tryptophanhydroylase-1-Hemmer (TPH1-Hemmer) und Multikinase-Hemmer, Beta2-Agonisten, Kortikosteroide, Anticholinergika oder Antimuskarinika, Inhibitoren mitogenaktivierter Proteinkinasen (P38-MAP-Kinase-Inhibitoren), Inhibitoren der nukleären Faktor-Kappa-B-Kinase-Untereinheit Beta (IKK2-Inhibitoren), Leukotrienmodulatoren, nichtsteroidalen entzündungshemmenden Mitteln (NSAIDs), Schleimregulatoren, Mukolytika, Expektorantien/mukokinetische Modulatoren, Peptid-Mukolytika, Inhibitoren von JAK, SYK -Inhibitoren, Inhibitoren von PI3Kdelta oder PI3Kgamma und Kombinationen davon.

**Revendications**

**1.** Composé de formule (I)

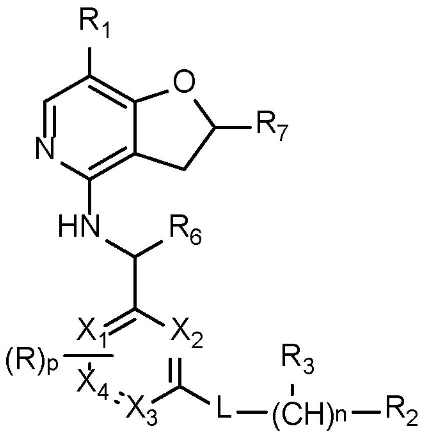

(I)

dans lequel

**$X_1$, $X_2$, $X_3$** et **$X_4$** sont tous CH ou l'un de $X_1$, $X_2$, $X_3$ et $X_4$ est N et les autres sont CH ;
p vaut zéro ou est un nombre entier allant de 1 à 4 ;
chaque R, lorsqu'il est présent, est choisi dans chaque cas indépendamment parmi alkyle en $C_1$-$C_6$ et halogène choisi parmi F, Cl, Br et I ; dans lequel, de préférence, R est F, Cl ou méthyle ;
$R_1$ est pyrimidinyle, de préférence pyrimidin-4-yle, substitué par un ou plusieurs groupes choisis parmi $-(CH_2)_mNH_2$ ; en particulier, de préférence, $R_1$ est 2-aminopyrimidin-4-yle ;
L est -C(O)NH- ou -NHC(O)- ;
n, dans chaque cas, vaut indépendamment 0 ou est un nombre entier choisi parmi 1, 2 ou 3 ;
$R_2$ et $R_3$, dans chaque cas, sont choisis indépendamment dans le groupe constitué de
-H,
halogène,
-OH,

$-(CH_2)_mNR_4R_5$,
alkyle en $C_1$-$C_6$,
hydroxyalkyle en $C_1$-$C_6$,
alcoxy en $C_1$-$C_6$,
alcoxy en $C_1$-$C_6$-alkyle en $C_1$-$C_6$,
haloalkyle en $C_1$-$C_6$,
haloalcoxy en $C_1$-$C_6$,
haloalcoxy en $C_1$-$C_6$-alkyle en $C_1$-$C_6$,
cycloalkyle en $C_3$-$C_{10}$,
aryle et hétérocycloalkyle en $C_3$-$C_6$,
dont chaque cycloalkyle, aryle ou hétérocycloalkyle
est à son tour facultativement et indépendamment substitué par un ou plusieurs groupes choisis parmi
halogène,
-OH,
alkyle en $C_1$-$C_6$,
hydroxyalkyle en $C_1$-$C_6$,
alcoxy en $C_1$-$C_6$,
alcoxy en $C_1$-$C_6$-alkyle en $C_1$-$C_6$,
haloalkyle en $C_1$-$C_6$,
haloalcoxy en $C_1$-$C_6$,
$-(CH_2)_mNR_4R_5$,
$-O-(CH_2)_mNR_4R_5$,
alcanoyle,
aryle, hétéroaryle, cycloalkyle,
aryl-alkyle en $C_1$-$C_6$,
hétéroaryl-alkyle en $C_1$-$C_6$,
hétérocycloalkyle en $C_3$-$C_8$,
hétérocycloalkyl en $C_3$-$C_8$-alkyle en $C_1$-$C_6$,
cycloalkyl en $C_3$-$C_8$-alkyle en $C_1$-$C_6$,
chacun desdits aryle, hétéroaryle, cycloalkyle, hétérocycloalkyle est en outre facultativement substitué par un ou plusieurs groupes choisis indépendamment parmi halogène choisi parmi F, Cl, Br et I, de préférence F, - OH, alkyle en $C_1$-$C_8$, haloalkyle en $C_1$-$C_6$, hydroxyalkyle en $C_1$-$C_6$,
m, dans chaque cas, vaut indépendamment 0 ou est un nombre entier choisi parmi 1, 2 ou 3 ;
$R_4$ et $R_5$, identiques ou différents, sont choisis dans le groupe constitué de
-H,
alkyle en $C_1$-$C_6$,
haloalkyle en $C_1$-$C_6$,
hydroxyalkyle en $C_1$-$C_6$,
hétérocycloalkyle en $C_3$-$C_6$ ;
$R_6$ et $R_7$ sont choisis indépendamment dans le groupe constitué de -H, alkyle en $C_1$-$C_6$ ;
dans lequel, lorsque ce n'est pas déjà spécifié, aryle se réfère à des systèmes cycliques mono-, bi- ou tri-cycliques qui ont 6 à 20 atomes de cycle, dans lesquels au moins un cycle est aromatique ; hétéroaryle se réfère à des systèmes cycliques mono-, bi- ou tri-cycliques avec 5 à 20 atomes de cycle, dans lesquels au moins un cycle est aromatique et dans lesquels au moins un atome de cycle est un hétéroatome choisi parmi N, S ou O ; hétérocycloalkyle en $C_3$-$C_6$ se réfère à des groupes cycloalkyle en $C_3$-$C_6$ monocycliques, saturés ou partiellement insaturés, dans lesquels au moins un atome de carbone de cycle est remplacé par au moins un hétéroatome choisi parmi N, NH, S ou O et/ou peut porter un groupe substituant oxo ;
énantiomères seuls, diastéréo-isomères et mélanges de ceux-ci dans n'importe quelle proportion et sels et solvates pharmaceutiquement acceptables de ceux-ci.

**2.** Composé selon la revendication 1,

dans lequel $X_1$, $X_3$ et $X_4$ sont tous des groupes CH et $X_2$ est un groupe CH ou un atome d'azote ;
$R_1$ est 2-aminopyrimidin-4-yle ;
toutes les autres variables étant telles que définies dans la revendication 1.

**3.** Composé selon la revendication 1,

dans lequel $X_1$, $X_2$, $X_3$, $X_4$ sont tous des groupes CH ;
chaque R, lorsqu'il est présent, est dans chaque cas choisi indépendamment parmi F, Cl, Br et I ;
$R_1$ est pyrimidinyle substitué par $-NH_2$ ;
L est -C(O)NH- ;
n vaut 0 ;
$R_2$ est choisi dans le groupe constitué de
alkyle en $C_1$-$C_6$,
hydroxyalkyle en $C_1$-$C_6$,
haloalkyle en $C_1$-$C_6$,
alcoxy en $C_1$-$C_6$-alkyle en $C_1$-$C_6$,
haloalcoxy en $C_1$-$C_6$-alkyle en $C_1$-$C_6$,
toutes les autres variables étant telles que définies dans la revendication 1,
énantiomères seuls, diastéréo-isomères et mélanges de ceux-ci dans n'importe quelle proportion et/ou sels et solvates pharmaceutiquement acceptables de ceux-ci.

**4.** Composé selon la revendication 3,

dans lequel $R_2$ est choisi parmi méthyle, éthyle, propyle, 2-hydroxy-2-méthylpropyle, 3-méthoxypropyle, 2-méthoxyéthyle, 2-éthoxyéthyle, 3-isopropoxypropyle, 3-méthoxy-3-méthylbutyle, 2-méthoxy-2-méthylpropyle, 3-fluoropropyle, 2-fluoro-2-méthylpropyle, 2,2-difluoropropyle, 3,3-difluoropropyle,
énantiomères seuls, diastéréo-isomères et mélanges de ceux-ci dans n'importe quelle proportion et/ou sels et solvates pharmaceutiquement acceptables de ceux-ci

**5.** Composé selon la revendication 1,

dans lequel $\mathbf{X_1}$, $\mathbf{X_2}$, $\mathbf{X_3}$ et $\mathbf{X_4}$ sont tous CH ;
p vaut zéro ou est un nombre entier allant de 1 à 4 ;
chaque R, lorsqu'il est présent, est halogène dans chaque cas choisi indépendamment parmi F, Cl, Br et I, dans lequel, de préférence, R est F ;
$R_1$ est pyrimidinyle substitué par $-NH_2$ ; en particulier, de préférence, $R_1$ est 2-aminopyrimidin-4-yle ;
L est -C(O)NH- ;
n, dans chaque cas, vaut indépendamment 0 ou est un nombre entier choisi parmi 1, 2 ou 3 ;
$R_3$, lorsqu'il est présent, est H,
et
$R_2$ est hétérocycloalkyle en $C_3$-$C_6$,
qui
est à son tour facultativement substitué par un ou plusieurs groupes choisis parmi
alkyle en $C_1$-$C_6$,
hydroxyalkyle en $C_1$-$C_6$,
alcoxy en $C_1$-$C_6$,
alcoxy en $C_1$-$C_6$-alkyle en $C_1$-$C_6$,
hétéroaryl-alkyle en $C_1$-$C_6$,
cycloalkyl en $C_3$-$C_8$-alkyle en $C_1$-$C_6$,
$-O-(CH_2)_mNR_4R_5$,
hétérocycloalkyle en $C_3$-$C_6$ m, dans chaque cas, vaut indépendamment 0 ou est un nombre entier choisi parmi 1, 2 ou 3 ;
$R_4$ et $R_5$, identiques ou différents, sont choisis dans le groupe constitué de
-H,
alkyle en $C_1$-$C_6$,
haloalkyle en $C_1$-$C_6$,
hydroxyalkyle en $C_1$-$C_6$,
toutes les autres variables étant telles que définies dans la revendication 1,
énantiomères seuls, diastéréo-isomères et mélanges de ceux-ci dans n'importe quelle proportion et/ou sels et solvates pharmaceutiquement acceptables de ceux-ci.

**6.** Composé selon la revendication 5, dans lequel $R_2$ est choisi parmi tétrahydrofuranyle, diméthyltétrahydrofuranyle, oxétan-2-yle.

**7.** Composé de formule (I) selon la revendication 1, dans lequel

**$X_1$, $X_2$, $X_3$** et **$X_4$** sont tous CH ou $X_2$ est N et les autres sont CH ;
p vaut zéro ou 1 ;
chaque R, lorsqu'il est présent, est F ;
$R_1$ est 2-aminopyrimidin-4-yle ;
L est -C(O)NH- ou -NHC(O)- ;
n, dans chaque cas, vaut indépendamment 0 ou est un nombre entier choisi parmi 1, 2 ou 3 ;
$R_2$ et $R_3$, dans chaque cas, sont choisis indépendamment dans le groupe constitué de
-H,
alkyle en $C_1$-$C_6$ qui est méthyle, propyle, isopropyle, tert-butyle,
hydroxyalkyle en $C_1$-$C_6$ qui est 2-hydroxy-2-méthylpropyle,
alcoxy en $C_1$-$C_6$-alkyle en $C_1$-$C_6$ qui est 3-méthoxypropyle, 2-éthoxyéthyle, 3-isopropoxypropyle, 3-méthoxy-3-méthylbutyle, 2-méthoxyéthyle, 2-méthoxy-2-méthylpropyle,
haloalkyle en $C_1$-$C_6$ qui est 2,2-difluoroéthyle, 2,2,2-trifluoroéthyle, 2,2,2-trifluorométhyle,2-fluoro-2-méthylpropyle, 3-fluoropropyle, 2,2-difluoropropyle, 3,3-difluoropropyle,
haloalcoxy en $C_1$-$C_6$-alkyle en $C_1$-$C_6$ qui est 3-(difluorométhoxy)propyle,
cycloalkyle en $C_3$-$C_{10}$ qui est cyclopropyle, cyclobutyle,
hétéroaryle qui est pyridinyle, et
et
hétérocycloalkyle en $C_3$-$C_6$ qui est azétidin-3-yle, pipéridinyle, morpholinyle, 5-oxopyrrolidin-3-yle, (1R,5S,6r)-3-oxabicyclo[3.1.0]hexan-6-yle, tétrahydrofuranyle, tétrahydro-2H-pyran-4-yle,
dont chaque cycloalkyle, hétéroaryle ou hétérocycloalkyle
est à son tour facultativement et indépendamment substitué par un ou plusieurs groupes choisis parmi
halogène qui est choisi parmi F, Cl, Br, I,
alkyle en $C_1$-$C_6$ qui est méthyle, tert-butyle,
alcoxy en $C_1$-$C_6$ qui est méthoxy,
alcoxy en $C_1$-$C_6$-alkyle en $C_1$-$C_6$ qui est méthoxyméthyle,
$R_6$ et $R_7$ sont -H,
énantiomères simples, diastéréo-isomères et mélanges de ceux-ci dans n'importe quelle proportion,
ou sels et solvates pharmaceutiquement acceptables de ceux-ci.

**8.** Composé selon la revendication 1 choisi parmi :

3-(((7-(2-aminopyrimidin-4-yl)-2,3-dihydrofuro[3,2-c]pyridin-4-yl)amino)méthyl)-N-cyclobutylbenzamide ;
3-(((7-(2-aminopyrimidin-4-yl)-2,3-dihydrofuro[3,2-c]pyridin-4-yl)amino)méthyl)-N-((tétrahydro-2H-pyran-4-yl)méthyl)benzamide ;
3-(((7-(2-aminopyrimidin-4-yl)-2,3-dihydrofuro[3,2-c]pyridin-4-yl)amino)méthyl)-N-(5,5-diméthyltétrahydrofuran-3-yl)benzamide ;
3-(((7-(2-aminopyrimidin-4-yl)-2,3-dihydrofuro[3,2-c]pyridin-4-yl)amino)méthyl)-N-cyclopropylbenzamide ;
3-(((7-(2-aminopyrimidin-4-yl)-2,3-dihydrofuro[3,2-c]pyridin-4-yl)amino)méthyl)-N-(2-(pipéridin-1-yl)éthyl)benzamide ;
3-(((7-(2-aminopyrimidin-4-yl)-2,3-dihydrofuro[3,2-c]pyridin-4-yl)amino)méthyl)-N-(3-(méthoxy-d3)propyl)benzamide ;
3-(((7-(2-aminopyrimidin-4-yl)-2,3-dihydrofuro[3,2-c]pyridin-4-yl)amino)méthyl)-N-(tétrahydro-2H-pyran-4-yl)benzamide ;
3-(((7-(2-aminopyrimidin-4-yl)-2,3-dihydrofuro[3,2-c]pyridin-4-yl)amino)méthyl)-N-(3-méthoxypropyl)benzamide ;
3-(((7-(2-aminopyrimidin-4-yl)-2,3-dihydrofuro[3,2-c]pyridin-4-yl)amino)méthyl)-N-(2-méthoxyéthyl)benzamide ;
3-(((7-(2-aminopyrimidin-4-yl)-2,3-dihydrofuro[3,2-c]pyridin-4-yl)amino)méthyl)-N-(3,3-difluorocyclobutyl)benzamide ;
3-(((7-(2-aminopyrimidin-4-yl)-2,3-dihydrofuro[3,2-c]pyridin-4-yl)amino)méthyl)-N-isopropylbenzamide ;
3-(((7-(2-aminopyrimidin-4-yl)-2,3-dihydrofuro[3,2-c]pyridin-4-yl)amino)méthyl)-N-(2,2,2-trifluoroéthyl)benzamide ;
3-(((7-(2-aminopyrimidin-4-yl)-2,3-dihydrofuro[3,2-c]pyridin-4-yl)amino)méthyl)-N-(1-méthylpipéridin-4-yl)benzamide ;
3-(((7-(2-aminopyrimidin-4-yl)-2,3-dihydrofuro[3,2-c]pyridin-4-yl)amino)méthyl)-N-(3-(difluorométhoxy)propyl)benzamide ;
3-(((7-(2-aminopyrimidin-4-yl)-2,3-dihydrofuro[3,2-c]pyridin-4-yl)amino)méthyl)-N-(3-isopropoxypropyl)benzamide ;
3-(((7-(2-aminopyrimidin-4-yl)-2,3-dihydrofuro[3,2-c]pyridin-4-yl)amino)méthyl)-N-(3-morpholinopropyl)benzamide ;
3-(((7-(2-aminopyrimidin-4-yl)-2,3-dihydrofuro[3,2-c]pyridin-4-yl)amino)méthyl)-N-(2-hydroxy-2-méthylpropyl) benzamide ;
3-(((7-(2-aminopyrimidin-4-yl)-2,3-dihydrofuro[3,2-c]pyridin-4-yl)amino)méthyl)-N-((1s,3s)-3-méthoxycyclobutyl)benzamide ;
3-(((7-(2-aminopyrimidin-4-yl)-2,3-dihydrofuro[3,2-c]pyridin-4-yl)amino)méthyl)-N-(2-((2R,6S)-2,6-diméthylpipéridin-1-yl)éthyl)benzamide ;
3-(((7-(2-aminopyrimidin-4-yl)-2,3-dihydrofuro[3,2-c]pyridin-4-yl)amino)méthyl)-N-(2-éthoxyéthyl)benzamide ;
3-(((7-(2-aminopyrimidin-4-yl)-2,3-dihydrofuro[3,2-c]pyridin-4-yl)amino)méthyl)-N-méthylbenzamide ;
3-(((7-(2-aminopyrimidin-4-yl)-2,3-dihydrofuro[3,2-c]pyridin-4-yl)amino)méthyl)-N-(1-méthylcyclopropyl)benzamide ;
3-(((7-(2-aminopyrimidin-4-yl)-2,3-dihydrofuro[3,2-c]pyridin-4-yl)amino)méthyl)-N-((1R,5S,6r)-3-oxabicyclo [3.1.0]hexan-6-yl)benzamide ;
3-(((7-(2-aminopyrimidin-4-yl)-2,3-dihydrofuro[3,2-c]pyridin-4-yl)amino)méthyl)-N-(1-(oxétan-3-yl)azétidin-3-yl)benzamide ;
3-(((7-(2-aminopyrimidin-4-yl)-2,3-dihydrofuro[3,2-c]pyridin-4-yl)amino)méthyl)-N-(cyclopropylméthyl)benzamide ;
3-(((7-(2-aminopyrimidin-4-yl)-2,3-dihydrofuro[3,2-c]pyridin-4-yl)amino)méthyl)-N-((1s,3s)-3-(difluorométhoxy) cyclobutyl)benzamide ;
3-(((7-(2-aminopyrimidin-4-yl)-2,3-dihydrofuro[3,2-c]pyridin-4-yl)amino)méthyl)-N-(2-méthoxy-2-méthylpropyl) benzamide ;
3-(((7-(2-aminopyrimidin-4-yl)-2,3-dihydrofuro[3,2-c]pyridin-4-yl)amino)méthyl)-N-((1-(méthoxyméthyl)cyclopropyl)méthyl)benzamide ;
3-(((7-(2-aminopyrimidin-4-yl)-2,3-dihydrofuro[3,2-c]pyridin-4-yl)amino)méthyl)-N-(3-fluoropropyl)benzamide ;
3-(((7-(2-aminopyrimidin-4-yl)-2,3-dihydrofuro[3,2-c]pyridin-4-yl)amino)méthyl)-N-((1-méthylcyclopropyl)méthyl)benzamide ;
3-(((7-(2-aminopyrimidin-4-yl)-2,3-dihydrofuro[3,2-c]pyridin-4-yl)amino)méthyl)-N-(2-fluoro-2-méthylpropyl) benzamide ;
3-(((7-(2-aminopyrimidin-4-yl)-2,3-dihydrofuro[3,2-c]pyridin-4-yl)amino)méthyl)-N-((1-(tert-butyl)-5-oxopyrrolidin-3-yl)méthyl)benzamide (énantiomère 1) ;
3-(((7-(2-aminopyrimidin-4-yl)-2,3-dihydrofuro[3,2-c]pyridin-4-yl)amino)méthyl)-N-((1-(tert-butyl)-5-oxopyrrolidin-3-yl)méthyl)benzamide (énantiomère 2) ;
3-(((7-(2-aminopyrimidin-4-yl)-2,3-dihydrofuro[3,2-c]pyridin-4-yl)amino)méthyl)-N-((4-méthylmorpholin-2-yl) méthyl)benzamide (énantiomère 1) ;
3-(((7-(2-aminopyrimidin-4-yl)-2,3-dihydrofuro[3,2-c]pyridin-4-yl)amino)méthyl)-N-((4-méthylmorpholin-2-yl) méthyl)benzamide (énantiomère 2) ;
3-(((7-(2-aminopyrimidin-4-yl)-2,3-dihydrofuro[3,2-c]pyridin-4-yl)amino)méthyl)-N-(3-méthoxy-3-méthylbutyl) benzamide ;
3-(((7-(2-aminopyrimidin-4-yl)-2,3-dihydrofuro[3,2-c]pyridin-4-yl)amino)méthyl)-N-((1R,2S)-2-fluorocyclopropyl)benzamide ;
3-(((7-(2-aminopyrimidin-4-yl)-2,3-dihydrofuro[3,2-c]pyridin-4-yl)amino)méthyl)-N-(oxétan-2-ylméthyl)benzamide ;
3-(((7-(2-aminopyrimidin-4-yl)-2,3-dihydrofuro[3,2-c]pyridin-4-yl)amino)méthyl)-N-(((1r,3r)-3-méthoxycyclobutyl)méthyl)benzamide ;
3-(((7-(2-aminopyrimidin-4-yl)-2,3-dihydrofuro[3,2-c]pyridin-4-yl)amino)méthyl)-N-propylbenzamide ;
3-(((7-(2-aminopyrimidin-4-yl)-2,3-dihydrofuro[3,2-c]pyridin-4-yl)amino)méthyl)-N-((1r,3r)-3-méthoxycyclobutyl)benzamide ;
(R)-3-(((7-(2-aminopyrimidin-4-yl)-2,3-dihydrofuro[3,2-c]pyridin-4-yl)amino)méthyl)-N-((tétrahydrofuran-2-yl) méthyl)benzamide ;
(S)-3-(((7-(2-aminopyrimidin-4-yl)-2,3-dihydrofuro[3,2-c]pyridin-4-yl)amino)méthyl)-N-((tétrahydrofuran-2-yl) méthyl)benzamide ;
3-(((7-(2-aminopyrimidin-4-yl)-2,3-dihydrofuro[3,2-c]pyridin-4-yl)amino)méthyl)-N-(2,2-difluoropropyl)benzamide ;
trans 3-(((7-(2-aminopyrimidin-4-yl)-2,3-dihydrofuro[3,2-c]pyridin-4-yl)amino)méthyl)-N-(2-fluorocyclopropyl)benzamide (énantiomère 1) ;
trans 3-(((7-(2-aminopyrimidin-4-yl)-2,3-dihydrofuro[3,2-c]pyridin-4-yl)amino)méthyl)-N-(2-fluorocyclopropyl) benzamide (énantiomère 2) ;
3-(((7-(2-aminopyrimidin-4-yl)-2,3-dihydrofuro[3,2-c]pyridin-4-yl)amino)méthyl)-N-((1S,2R)-2-fluorocyclopropyl)benzamide ;
3-(((7-(2-aminopyrimidin-4-yl)-2,3-dihydrofuro[3,2-c]pyridin-4-yl)amino)méthyl)-N-((1s,3s)-3-fluorocyclobutyl) benzamide ;
3-(((7-(2-aminopyrimidin-4-yl)-2,3-dihydrofuro[3,2-c]pyridin-4-yl)amino)méthyl)-N-((1r,3r)-3-fluorocyclobutyl) benzamide ;
3-(((7-(2-aminopyrimidin-4-yl)-2,3-dihydrofuro[3,2-c]pyridin-4-yl)amino)méthyl)-N-(3,3-difluoropropyl)benzamide ;
3-(((7-(2-aminopyrimidin-4-yl)-2,3-dihydrofuro[3,2-c]pyridin-4-yl)amino)méthyl)-N-(5-méthoxypyridin-2-yl)benzamide ;
3-(((7-(2-aminopyrimidin-4-yl)-2,3-dihydrofuro[3,2-c]pyridin-4-yl)amino)méthyl)-N-(5-(2-(diméthylamino)éthoxy)pyridin-2-yl)benzamide ;
3-(((7-(2-aminopyrimidin-4-yl)-2,3-dihydrofuro[3,2-c]pyridin-4-yl)amino)méthyl)-N-(5-(pipérazin-1-yl)pyridin-2-yl)benzamide ;
3-(((7-(2-aminopyrimidin-4-yl)-2,3-dihydrofuro[3,2-c]pyridin-4-yl)amino)méthyl)-2-fluoro-N-méthylbenzamide ;
3-(((7-(2-aminopyrimidin-4-yl)-2,3-dihydrofuro[3,2-c]pyridin-4-yl)amino)méthyl)-2-fluoro-N-(3-méthoxypropyl) benzamide ;
3-(((7-(2-aminopyrimidin-4-yl)-2,3-dihydrofuro[3,2-c]pyridin-4-yl)amino)méthyl)-2-fluoro-N-(tétrahydro-2H-pyran-4-yl)benzamide ;
5-(((7-(2-aminopyrimidin-4-yl)-2,3-dihydrofuro[3,2-c]pyridin-4-yl)amino)méthyl)-2-fluoro-N-(3-méthoxypropyl) benzamide ;
5-(((7-(2-aminopyrimidin-4-yl)-2,3-dihydrofuro[3,2-c]pyridin-4-yl)amino)méthyl)-2-fluoro-N-(tétrahydro-2H-pyran-4-yl)benzamide ;
3-(((7-(2-aminopyrimidin-4-yl)-2,3-dihydrofuro[3,2-c]pyridin-4-yl)amino)méthyl)-5-fluoro-N-méthylbenzamide ;
3-(((7-(2-aminopyrimidin-4-yl)-2,3-dihydrofuro[3,2-c]pyridin-4-yl)amino)méthyl)-5-fluoro-N-(3-méthoxypropyl) benzamide ;
3-(((7-(2-aminopyrimidin-4-yl)-2,3-dihydrofuro[3,2-c]pyridin-4-yl)amino)méthyl)-4-fluoro-N-(tétrahydro-2H-pyran-4-yl)benzamide ;
3-(((7-(2-aminopyrimidin-4-yl)-2,3-dihydrofuro[3,2-c]pyridin-4-yl)amino)méthyl)-4-fluoro-N-(3-méthoxypropyl) benzamide ;
6-(((7-(2-aminopyrimidin-4-yl)-2,3-dihydrofuro[3,2-c]pyridin-4-yl)amino)méthyl)-N-méthylpicolinamide ;
N-(3-(((7-(2-aminopyrimidin-4-yl)-2,3-dihydrofuro[3,2-c]pyridin-4-yl)amino)méthyl)phényl)-4-méthoxybutanamide ;
énantiomères seuls, diastéréo-isomères et mélanges de ceux-ci dans n'importe quelle proportion,
ou sels et solvates pharmaceutiquement acceptables de ceux-ci.

**9.** Composition pharmaceutique comprenant un composé selon l'une quelconque des revendications 1 à 8, ou un sel pharmaceutiquement acceptable de celui-ci, en mélange avec un ou plusieurs supports ou excipients pharmaceutiquement acceptables.

**10.** Composition pharmaceutique selon la revendication 9, apte à être administrée par inhalation, choisie parmi des poudres inhalables, aérosols-doseurs contenant un agent propulseur ou formulations inhalables sans agent propulseur.

**11.** Dispositif comprenant la composition pharmaceutique selon la revendication 10, qui peut être un inhalateur de poudre sèche à dose unique ou à doses multiples, un aérosol-doseur ou un nébuliseur à brouillard doux.

**12.** Composition pharmaceutique selon la revendication 9, apte à être administrée par voie orale, choisie parmi des gélules, capsules, comprimés, granules, pastilles et poudres en vrac ou solutions aqueuses et non aqueuses, émulsions, suspensions, sirops, ou formulations d'élixirs.

**13.** Composé ou composition pharmaceutique selon l'une quelconque des revendications 1 à 8 ou 9, pour utilisation comme médicament.

**14.** Composé ou composition pharmaceutique pour utilisation selon la revendication 13, dans la prévention et/ou le

traitement de troubles du système immunitaire, y compris la maladie du greffon contre l'hôte (GVHD), et des maladies pulmonaires choisies dans le groupe constitué d'asthme, maladie pulmonaire obstructive chronique MPOC, fibrose pulmonaire idiopathique (FPI), hypertension pulmonaire (HP) et plus particulièrement hypertension artérielle pulmonaire (HTAP).

**15.** Composé ou composition pharmaceutique pour utilisation selon la revendication 14, pour utilisation par voie d'administration orale, en particulier dans la prévention et/ou le traitement d'asthme, maladie pulmonaire obstructive chronique MPOC, fibrose pulmonaire idiopathique (FPI), hypertension pulmonaire (PH) et plus particulièrement hypertension artérielle pulmonaire (HTAP).

**16.** Combinaison d'un composé selon l'une quelconque des revendications 1 à 8 avec un ou plusieurs ingrédients actifs choisis parmi les classes constituées de nitrates organiques et donneurs de NO ; NO inhalé ; stimulateur de la guanylate cyclase soluble (sGC) ; analogue de la prostacycline PGI2 et agoniste des récepteurs de la prostacycline ; composés qui inhibent la dégradation de la guanosine monophosphate cyclique (GMPc) et/ou de l'adénosine monophosphate cyclique (AMPc) ; inhibiteurs de l'élastase des neutrophiles humains ; composés inhibant la cascade de transduction du signal ; substances actives permettant d'abaisser la pression artérielle ; inhibiteur de l'endopeptidase neutre ; agents osmotiques ; bloqueurs ENaC ; anti-inflammatoires, y compris corticostéroïdes et antagonistes des récepteurs de la chimiokine ; médicaments antihistaminiques ; médicaments antitussifs ; antibiotiques et substance médicamenteuse DNase et agents de clivage sélectif ; agents qui inhibent la phosphorylation ALK5 et/ou ALK4 de Smad2 et Smad3 ; inhibiteurs de la tryptophane hydroxylase 1 (TPH1) et inhibiteurs multikinases, bêta2-agonistes, corticostéroïdes, agents anticholinergiques ou antimuscariniques, inhibiteurs de protéines kinases activées par mitogène (P38 MAP kinase), inhibiteurs du facteur nucléaire kappa-B kinase sous-unité bêta (IKK2), modulateurs des leucotriènes, agents anti-inflammatoires non stéroïdiens (AINS), régulateurs du mucus, mucolytiques, modulateurs expectorants/mucocinétiques, mucolytiques peptidiques, inhibiteurs de JAK, inhibiteurs de SYK, inhibiteurs de PI3K delta ou PI3K gamma et combinaisons de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004039796 A
- WO 2006009889 A
- WO 2010032875 A
- WO 2009079008 A
- WO 2014118133 A
- WO 2018115383 A
- WO 2018138293 A
- WO 2019048479 A
- WO 2019121223 A
- WO 2019121233 A
- WO 2019121406 A
- WO 2019238628 A
- WO 2020016129 A
- WO 2012007539 A


### Non-patent literature cited in the description


- **RIENTO, K.** ; **RIDLEY, A. J.** Rocks: multifunctional kinases in cell behaviour. *Nat. Rev. Mol. Cell Biol.*, 2003, vol. 4, 446-456
- **DUONG-QUY S** ; **BEI Y** ; **LIU Z** ; **DINH-XUAN AT**. Role of Rho-kinase and its inhibitors in pulmonary hypertension. *Pharmacol Ther*, 2013, vol. 137 (3), 352-64
- **FERNANDES LB** ; **HENRY PJ** ; **GOLDIE RG**. Rho kinase as a therapeutic target in the treatment of asthma and chronic obstructive pulmonary disease. *Ther Adv Respir Dis.*, October 2007, vol. 1 (1), 25-33
- **GOSENS, R.** ; **SCHAAFSMA, D.** ; **NELEMANS, S. A.** ; **HALAYKO, A. J**. Rhokinase as a drug target for the treatment of airway hyperresponsiveness in asthma. *Mini-Rev. Med. Chem.*, 2006, vol. 6, 339-348
- **JIANG, C.** ; **HUANG, H.** ; **LIU, J.** ; **WANG, Y.** ; **LU, Z.** ; **XU, Z**. Fasudil, a rho-kinase inhibitor, attenuates bleomycin-induced pulmonary fibrosis in mice. *Int. J. Mol. Sci.*, 2012, vol. 13, 8293-8307
- **BRINGMANN G et al.** *Angew. Chemie Int. Ed.*, 2005, vol. 44 (34), 5384-5427
- **OKI M**. *Topics in Stereochemistry*, 1983, vol. 14, 1-82
- Strategic application of named reactions in organic synthesis. 2005
- Remington's Pharmaceutical Sciences Handbook. Mack Pub.